(19) Europäisches Patentamt
European Patent Office
Office européen des brevets

(11) Veröffentlichungsnummer: **0 192 175**
**A2**

(12) # EUROPÄISCHE PATENTANMELDUNG

(21) Anmeldenummer: 86101746.5

(22) Anmeldetag: 12.02.86

(51) Int. Cl.[4]: C 12 N 15/00
A 61 K 39/21, C 12 N 1/20
C 07 K 13/00, C 12 P 21/02

(30) Priorität: 15.02.85 DE 3505148

(43) Veröffentlichungstag der Anmeldung:
27.08.86 Patentblatt 86/35

(84) Benannte Vertragsstaaten:
AT BE CH DE FR GB IT LI LU NL SE

(71) Anmelder: Boehringer Ingelheim International G.m.b.H
D-6507 Ingelheim am Rhein(DE)

(72) Erfinder: Küchler, Ernst, Prof.Dr.
Laudongasse 25/18
A-1080 Wien(AT)

(72) Erfinder: Skern, Timothy, Dr.
15, Rue de St. Diê
F-67100 Strasbourg-Neudorf(AT)

(72) Erfinder: Sommergruber, Wolfgang, Dr.
Stösslgasse 6/5
A-1130 Wien(AT)

(72) Erfinder: Blaas, Dieter, Dr.Dipl.-Ing.
Liechtensteinstrasse 119/13
A-1090 Wien(AT)

(72) Erfinder: Gründler, Peter
Klostermanngasse 13
A-1230 Wien(AT)

(72) Erfinder: Fraundorfer, Franz
Steingasse 2A
A-1030 Wien(AT)

(72) Erfinder: Düchler, Markus
Rubensgasse 1/8
A-1040 Wien(AT)

(54) **Polypeptide des Rhinovirusstammes HRV2 sowie die hierfür codierenden DNA-Moleküle.**

(57) Die vorliegende Erfindung betrifft Peptide, die ganz oder teilweise denen der viralen Proteine des humanen Rhinovirus Stamm 2 (HRV2) entsprechen, die für diese Peptide codierenden Deoxyribonukleinsäuremoleküle, sowie die Herstellung und Verwendung dieser Substanzen.

EP 0 192 175 A2

Croydon Printing Company Ltd.

Die vorliegende Erfindung betrifft Peptide, die ganz oder teilweise denen der viralen Proteine des humanen Rhinovirus Stamm 2 (HRV2) entsprechen, die für diese Peptide codierenden Deoxyribonukleinsäuremoleküle, sowie die Herstellung und Verwendung dieser Substanzen.

Rhinoviren sind RNA-Viren und stellen nach der herkömmlichen Einteilung der Viren eine Gattung innerhalb der Familie der Picornaviren dar (Cooper, P.D., Agol, H.L., Bachrach, H.L., Brown, F., Ghendon, Y., Gibbs, A.J., Gillespie, J.H., Lonberg-Holm, K., Mandel, B., Melnick, J.L., Mohanty, S.B., Povey, R.C., Rueckert, R.R., Schaffer, F.L. und Tyrrell, D.A.J., 1978, Intervirology, 10, 165-180; MacNaughton, 1982, Current Top. Microbiol. Immunol. 97, 1 - 26).

Sie sind weit verbreitet, befallen den oberen respiratorischen Trakt des Menschen und verursachen akute Infektionen, die zu Schnupfen, Husten, Heiserkeit etc. führen und allgemein als Erkältungen bezeichnet werden (Stott, E.J. und Killington, R.A., 1972, Ann.Rev.Microbiol. 26, 503 - 524). Infektionen durch Rhinoviren zählen zu den häufigsten Erkrankungen des Menschen. Obwohl der Verlauf dieser Erkrankungen meist harmloser Natur ist, führen Erkältungen doch zu einer vorübergehenden Schwächung des Organismus. Dadurch kann es zu Sekundärinfektionen durch andere Viren oder Bakterien kommen, die dann unter Umständen schwere Erkrankungen zur Folge haben. Außerdem ist der durch Rhinoviren verursachte volkswirtschaftliche Schaden beträchtlich. Es ist berechnet worden, daß in den U.S.A. durch Rhinovirusinfektionen jährlich mehr als 200 Millionen Arbeitstage bzw. Schultage verloren gehen (Davis, B.D., Dulbecco, R., Eisen, H.N. und Ginsberg, H.S., 1980, Microbiology, Third Edition, Harper & Row, Publ., New York, S. 1114). In den letzten Jahren ist zusätzlich eine beträchtliche Zunahme an Rhinovirusinfektionen in Ballungszentren festgestellt worden. Während es bei den meisten anderen Infektionskrankheiten zu einer langdauernden oder bleibenden Immunität gegen den betreffenden Krankheitserreger kommt, können Infektionen durch Rhinoviren immer wieder auftreten. Der Grund für das Fehlen einer bleibenden Immunität ist die große Vielfalt an Rhinovirusstämmen. Bis jetzt wurden über 100 Rhinovirusstämme isoliert, die untereinander keine oder nur wenig immunologische Kreuzreaktion zeigen (Fox, J.P., 1976, American J. Epidemiol. 103, 345 - 354; Melnick, J.L., 1980, Proc. Med. Virol. 26, 214 - 232). Nach erfolgter Infektion lassen sich zwar Antikörper gegen den betreffenden Virusstamm nachweisen, doch gewähren diese keinen Schutz gegen andere Rhinovirusstämme. Auf Grund der großen Anzahl von Stämmen, die in der Bevölkerung zirkulieren, sind wiederholte Infektionen durch Rhinoviren möglich.

Aufgabe der vorliegenden Erfindung war es daher, Mittel bereitzustellen, die einen Schutz gegen Infektionen durch Rhinoviren ermöglichen.

Unter Verwendung von Hyperimmunseren ist es möglich gewesen, 50 von insgesamt 90 Rhinovirus Serotypen in 16 Gruppen einzuteilen (Conney, M.K., Fox, J.P. und Kenny, G.E., 1982, Infect. Immun. 37, 642 - 647). Eine andere Form der Einteilung ergibt sich auf Grund der Bindung an zelluläre Rezeptoren. Trotz der ausgeprägten Heterogenität in den immunologischen Eigenschaften verhalten sich nämlich die Rhinoviren in Bezug auf die Bindung an Zelloberflächen-Rezeptoren untereinander ähnlich.

Mit Hilfe von Kompetitionsexperimenten konnte festgestellt werden, daß es für die 24 untersuchten Rhinovirusstämme auf HeLa-Zellen (Klon R-19) nur zwei verschiedene Rezeptoren gibt (Abraham, G. und Colonno, R.J., 1984, J. Virol. 51, 340 - 344). Da es sich bei dieser Gruppe um willkürlich ausgewählte Stämme gehandelt hat, wird angenommen, daß diese Befunde auch auf andere Rhinovirusstämme ausgedehnt werden können. Einschränkend muß dazu allerdings gesagt werden, daß diese Ergebnisse mit HeLa-Zellen erhalten worden sind und nicht notwendigerweise auch für die Rezeptoren an den natürlichen Wirtszellen im oberen respiratorischen Trakt des Menschen Gültigkeit haben.

Eine weitere Aufgabe der Erfindung bestand darin, Oligopeptide bereitzustellen, die ganz oder teilweise den viralen Proteinen entsprechen, die entweder zur Anregung einer gegen intakte Viren gerichteten Immunantwort verwendet oder zur Bindung und Blockierung von zellulären Rezeptoren eingesetzt werden können.

Als typische Picornaviren enthalten Rhinoviren eine einzelsträngige RNA, die von einem Kapsid umgeben ist, das aus 4 Polypeptiden besteht, die als VP1 (P1D), VP2 (P1B), VP3 (P1C) und VP4 (P1A) bezeichnet werden (Medappa, K.C., McLean, C. und Rueckert, R.R., 1971, Virology 44, 259-270; die Ausdrücke in Klammer sind die neuen Bezeichnungen entsprechend dem Nomenklaturvorschlag von Rueckert, R.R. und Wimmer, E., 1984, J. Virol. 50, 957 - 959). Ein einzelnes Viruspartikel enthält 60 Kopien von jedem dieser Polypeptide. Die relativen Molekülmassen betragen bei verschiedenen Rhinoviren für VP1 34 - 36000, für VP2 27 - 30000, für VP3 24 - 28000 und für VP4 7 - 8000 MacNaughton, M.R., 1982, loc.cit.). Darüberhinaus ist für Rhinoviren charakteristisch, daß sie bei pH-Werten unter 5 rasch inaktiviert werden und empfindlich gegen Salzlösungen hoher Konzentration sind. Weiters zeigen die meisten Rhinoviren ein optimales Wachstum bei 33 - 34°C (Luria, S.E., Darnell, Jr.,J.E., Baltimore, D. und Campbell, A., 1978, General Virology, Third Edition, John Wiley & Sons, New York, 308 ff.).

Die einzelsträngige RNA mit einer Länge von ca. 7100 Nukleotiden stellt das Genom des Virus dar und kann gleichzeitig auch als Matrize für die Synthese der viralen Proteine dienen. Dabei wird ein Großteil der Nukleotidsequenz zunächst in ein langes Polyprotein übersetzt, aus dem durch proteolytische Spaltung die fertigen viralen Proteine entstehen (Butterworth, B.E., 1973, Virology 56, 439 - 453; McLean, C. und Rueckert, R.R., 1973, J.Virol. 11, 341 - 344; McLean, C., Matthews, T.J. und Rueckert, R.R., 1976, J. Virol. 19, 903 - 914). Als Produkte dieser Prozessierung werden neben den viralen Hüllenproteinen VP1, VP2, VP3 und VP4 noch eine Reihe von Proteinen gebildet, wie P2C, P3B, P3C und P3D. P3B (meist als VPg bezeichnet) ist kovalent an das 5'-terminale Nukleotid der HRV2-RNA gebunden. In Analogie zu Poliovirus kann angenommen werden, daß P3C eine Protease ist, die teilweise für die Prozessierung des viralen Polyproteins verantwortlich ist (MacNaughton, M.R., 1982, loc.cit.). Dementsprechend ist P3D die Polymerase für die Replikation der viralen RNA. Über die Funktion des Proteins P2C ist bisher nur wenig bekannt.

Beim Prozessieren des viralen Polyproteins werden Teile der Aminosäuresequenz abgespalten und anschließend abgebaut (McLean, C., Matthews, T.J. und Rueckert, R.R., 1976, loc.cit.). Zur Zeit kann keine Aussage darüber gemacht werden, ob diesen Peptiden nicht doch eine bis jetzt noch unentdeckte Funktion zukommt.

Über die Sequenz des Rhinovirusstammes HRV2 ist bis jetzt nur aus Veröffentlichungen unserer Arbeitsgruppe (Skern, T., Sommergruber, W., Blaas, D., Pieler, Ch. und Kuechler, E., 1984, Virology 136, 125 - 132; Skern, T., Sommergruber, W., Blaas, D., Pieler, Ch. und Kuechler, E., 1984, Sixth Int. Congress Virology, Sendai, Abstract, P8-20) die Sequenz der 3'-untranslatierten Region, der RNA-Polymerase (P3D) und des VPg (P3B) bekannt. Der Vergleich der Nukleotidsequenz mit Poliovirus (Typ 1) und Maul- und Klauenseuchevirus (Subtyp A12) zeigt in der 3'-untranslatierten Region keine signifikanten Homologien. Auffällig ist, daß die 3'-untranslatierte Region bei HRV2 mit 42 Nukleotiden sehr viel kürzer ist als bei anderen Picornaviren. In der Region der RNA-Polymerase hingegen zeigt sich eine auffällige Homologie in der Aminosäuresequenz zwischen HRV2 und Poliovirus (56 %), während die Homologie zur RNA-Polymerase von Maul- und Klauenseuchevirus nur 27 % beträgt (Skern, T., Sommergruber W., Blaas, D., Pieler, Ch. und Kuechler, E., 1984, Virology, loc. cit.). Diese Daten weisen auf eine große Ähnlichkeit zwischen Rhinoviren und Enteroviren hin. So ist im VPg (P3B) von HRV2 auch das Tyrosin, über das im Poliovirus die Bindung an das 5'-terminale Nukleotid der RNA erfolgt, konserviert (Skern, T., Sommergruber, W., Blaas, D., Pieler, Ch. und Kuechler, E.,1984, 6th Int. Congress Virology, Sendai, loc.cit.). Die Verwandtschaft zu den Enteroviren wird auch aus dem Sequenzvergleich zwischen HRV2, dem humanen Rhinovirusstamm HRV14 (Stanway, G., Hughes, P.J., Mountford, R.C., Minor, P.D.und Almond, J.W., 1984, Nucleic Acids Res. 12, 7859-7877) und Polioviren deutlich.

Zur Gewinnung des viralen Ausgangsmaterials wurden HeLa-Zellen in einem geeigneten Infektionsmedium mit HRV2 infiziert und mehrere Stunden unter optimalen Wachstumsbedingungen inkubiert.

Virus konnte sowohl aus den Zellen als auch aus dem Medium gewonnen werden.

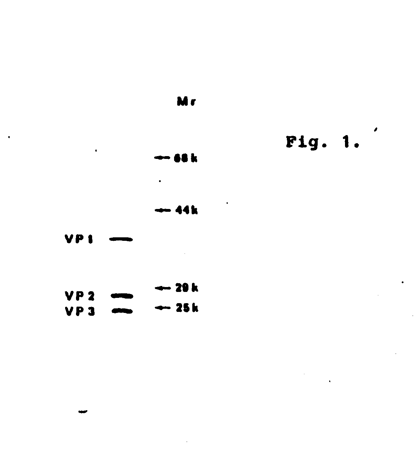

Verschiedene Reinigungsschritte erbrachten eine Viruspräparation, deren Proteinmuster sich typischerweise wie in Fig. 1 gezeigt darstellt.

Die virale RNA wurde aus der Viruspräparation beispielsweise durch Phenolextraktion gewonnen und gereinigt; sie wurde anschließend mit Hilfe reverser Transkriptase und Oligo dT als Primer cDNA transkribiert. Die erhaltenen RNA/cDNA-Hybride wurden homopolymer verlängert und in ein geeignetes Plasmid, beispielsweise das pBR322 eingebaut.

Die Verwendung von Methoden zur reversen Transkription von RNA, zur Integration von RNA-cDNA Hybriden in Plasmide und zur Transformation von Bakterien sind in der Literatur ausreichend beschrieben worden (Kitamura, N. und Wimmer, E., 1980, Proc. Natl. Acad. Sci. USA 77, 3196 - 3200; Nelson, T. und Brutlag, D., 1979, Methods in Enzymology 68, 41 - 50; Zain, S., Sambrook, J., Roberts, J.J., Keller, W., Fried, M. und Dunn, A., 1979, Cell 16, 851 - 861; Roychoudhury, R. und Wu, R., 1980, Methods in Enzymology 65, 42 - 62; Kitamura, N., Semler, B.L. Rothberg, P.G., Larsen, G.R., Adler, C.J., Dorner, A.J., Emini, E.A., Hanecak, R., Lee, J.L., van der Werf, S., Anderson, C.W. und Wimmer, E., 1981, Nature (London) 291, 547 - 553;

van der Werf, S., Bregegere, F., Kopecka, H., Kitamura, N., Rothberg, P.G. Kourilsky, P., Wimmer, E. und Girard, M., 1981, Proc. Natl. Acad. Sci. USA 78, 5983 - 5987; Namoto, A., Omata, T., Toyoda, H., Kuge, S., Hosie, H., Kataoka,Y., Genba, A., Nakano, Y. und Imura, N., 1982, Proc. Natl. Acad. Sci. USA 79, 5793 - 5797; Stanway, G., Cann, A.J., Hauptmann, R., Hughes, P., Clarke, L.D., Mountford, R.C., Minor, P.D., Schild, G.C. und Almond, J.W., 1983, Nucleic Acids Res. 11, 5629 - 5643).

Nach erfolgter Transformation eines geeigneten Wirtsorganismus, beispielsweise E. coli HB 101 wurde die Plasmid-DNA nach der "Mini-Plasmid-Präparationstechnik" isoliert und die Größe der rekombinanten DNA ermittelt. Hierzu wurden die rekombinanten Plasmide mit Hilfe der Restriktionsendonuclease PstI geschnitten, die Bruchstücke elektrophoretisch aufgetrennt und mit bekannter Lambda-HindIII Marker DNA verglichen.

Zur Subklonierung der DNA wurden die durch PstI Verdau der rekombinanten pBR322 Klone erhaltenen und gereinigten DNA-Fragmente in einem geeigneten Vektor, beispielsweise in das Plasmid pUC9 eingebaut und die rekombinanten Vektoren in einen geeigneten Wirt, beispielsweise E. coli JM101 transformiert. Die Subklone, die mit rekombinanten Vektoren erfolgreich transformiert worden waren, wurden hochgezüchtet und deren Plasmid-DNA isoliert und gereinigt.

Aus zwei Subklonen wurden zwei verschieden lange Primer-Fragmente /59 Nukleotide (Fragment A) und 68 Nukleotide (Fragment B)/ isoliert und gereinigt. Mit Hilfe dieser komplementären Einzelstrang DNA wurde ein mit $^{32}P$ markiertes reverses Transkript von HRV2-RNA hergestellt.

Zum Nachweis von HRV2-Sequenzen in rekombinanter DNA wurde die Plasmid DNA mit PstI verdaut, elektrophoretisch analysiert und die DNA Fragmente vom Gel auf Nitrozellulosefilter transferiert und fixiert. Diese DNA-tragenden Filter wurden mit der radioaktiv markierten HRV2-cDNA hybridisiert, die Filter anschließend exponiert. Von den so ermittelten, zur HRV2-RNA komplementären, rekombinanten DNA-Fragmenten wurden die Restriktionsstellen kartiert und sequenziert. Man erhielt Klone, die das Genom von HRV2 repräsentieren.

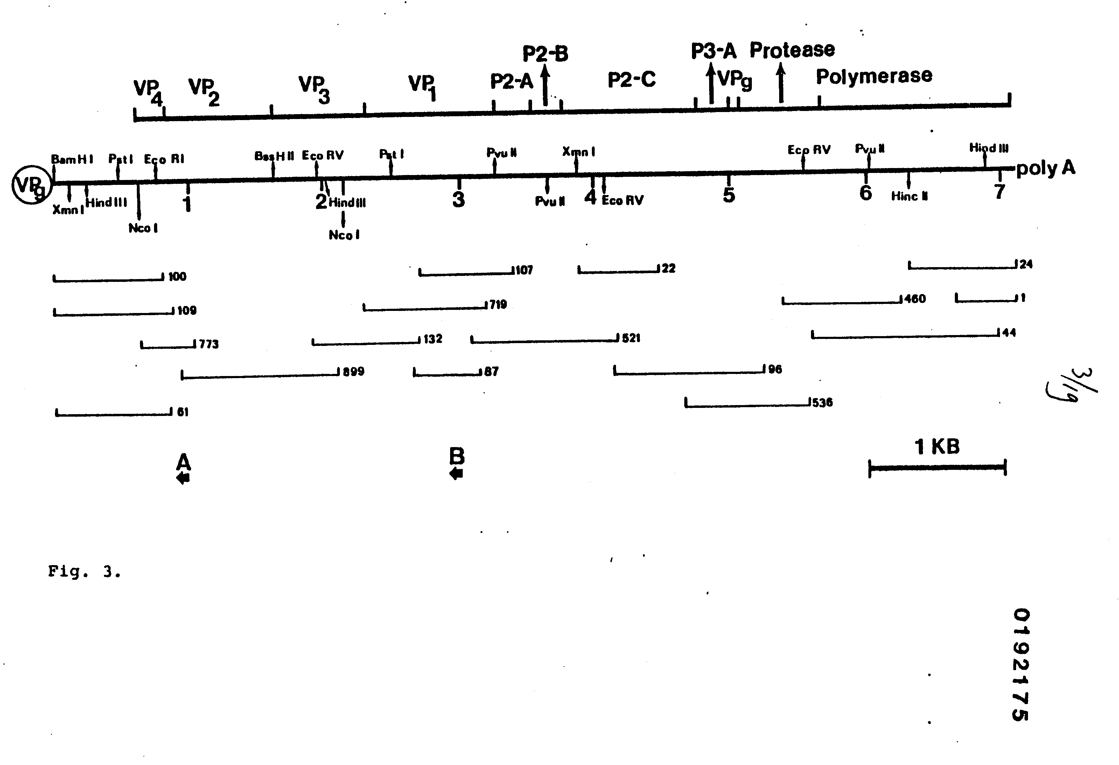

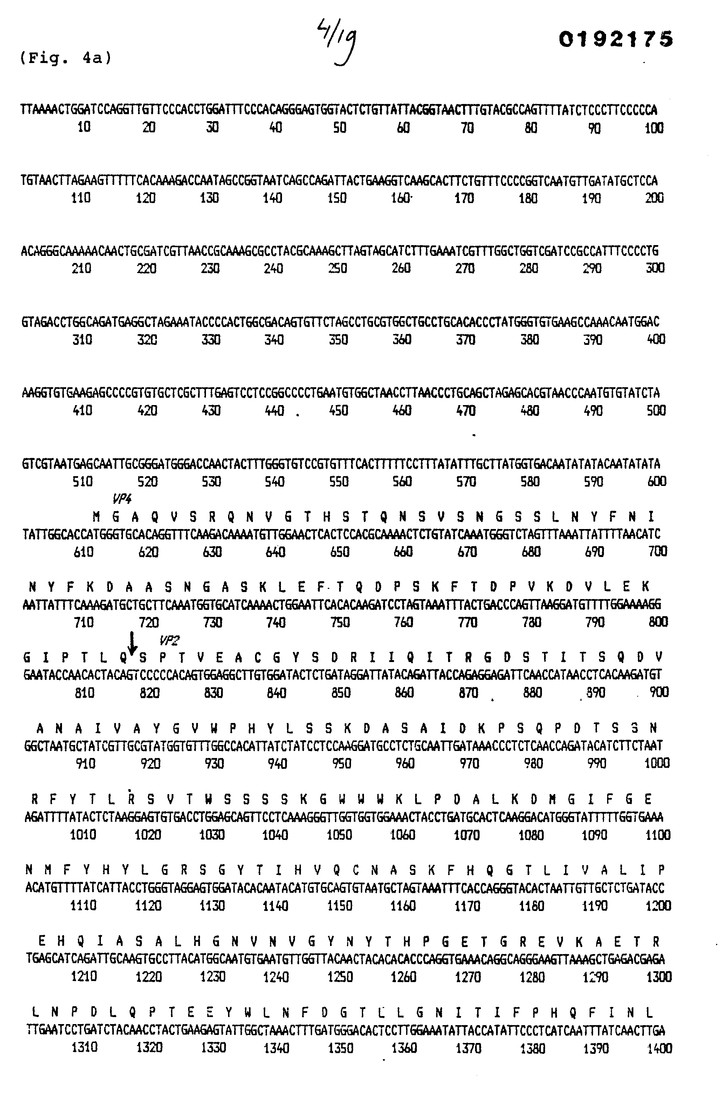

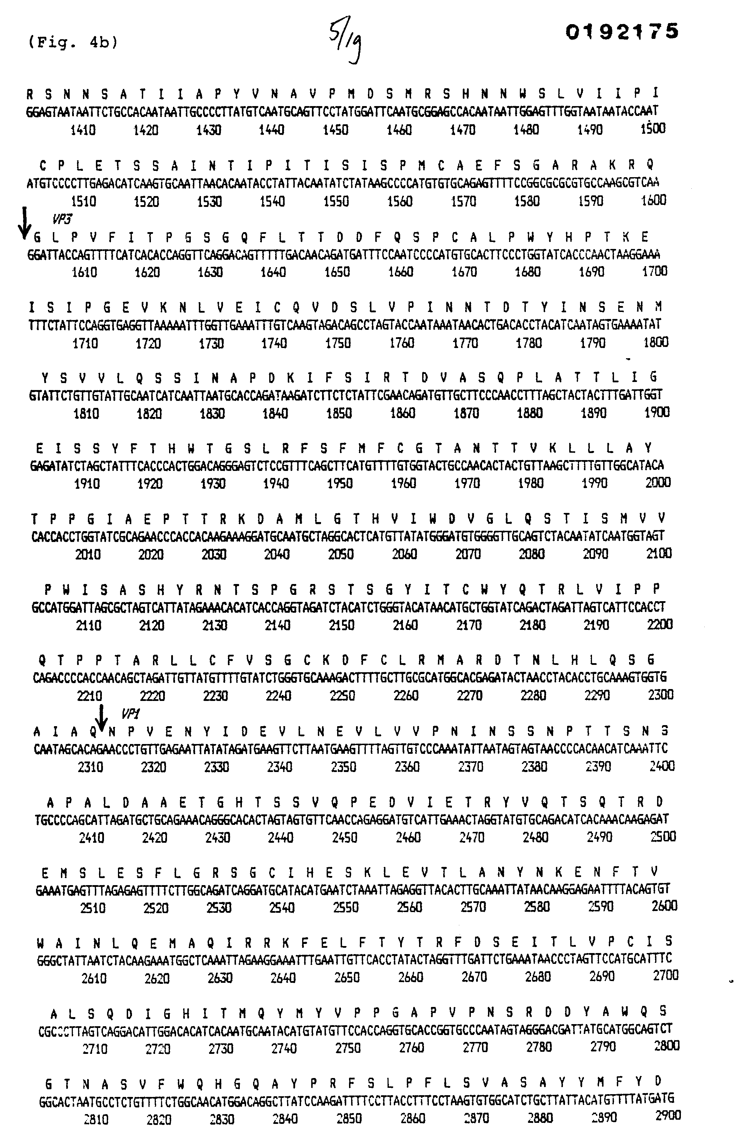

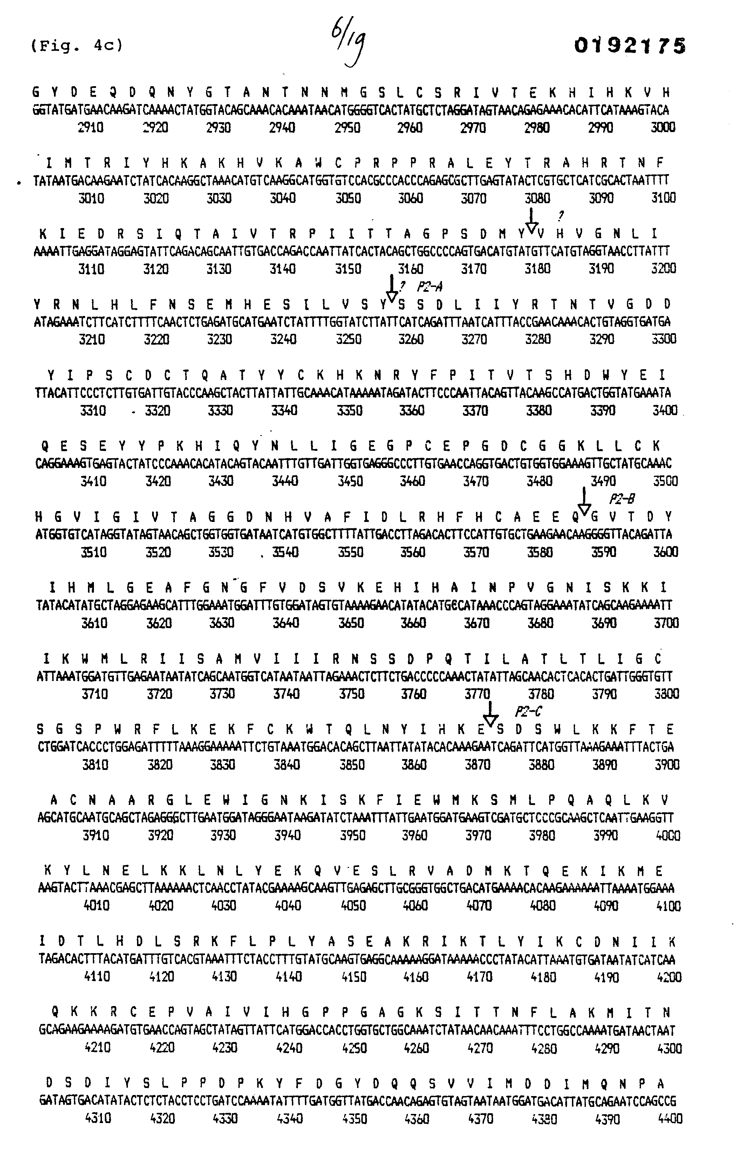

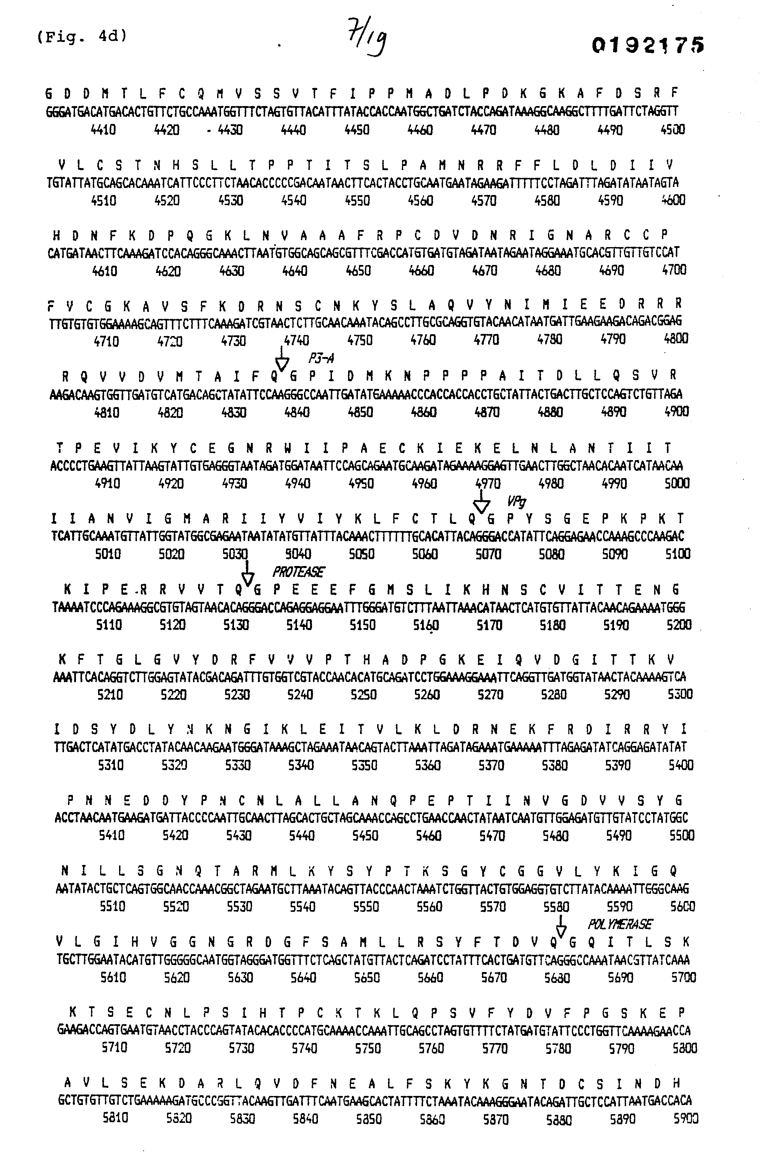

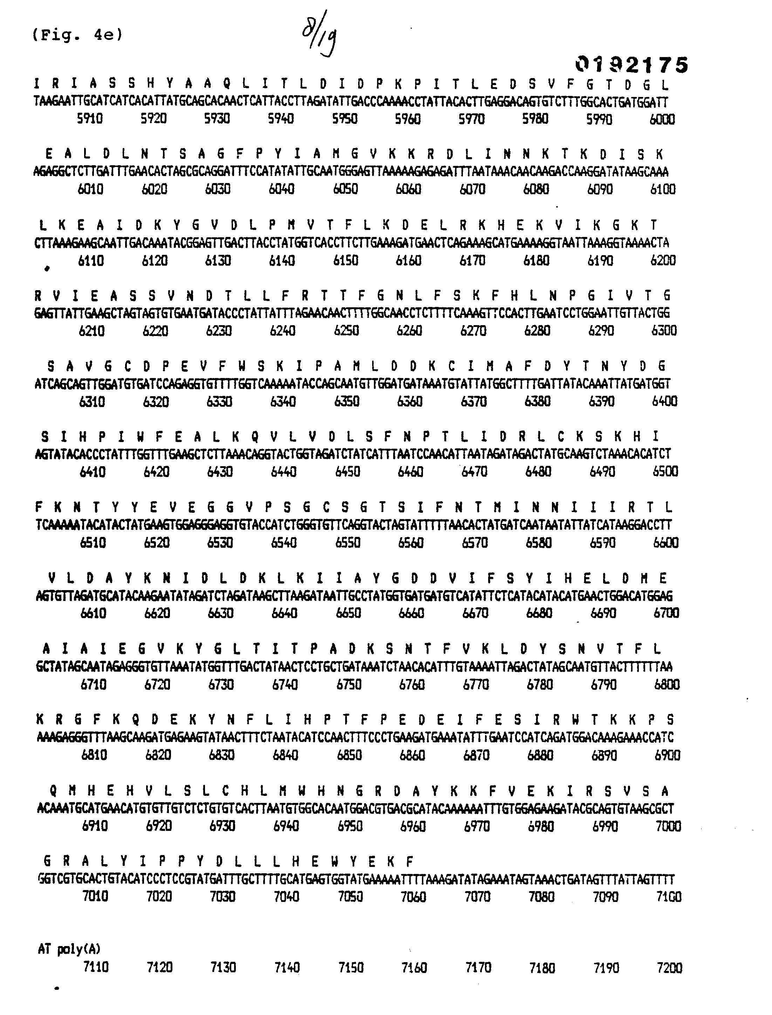

Die Sequenz des HRV2 Genoms ist in Fig. 4 gezeigt. Sie ist in Form der DNA dargestellt, wie sie aus der Sequenzierung der Rekombinanten-DNA erhalten worden ist. Zur Bestimmung der Sequenz wurden 17 Klone, die in Fig. 3 gezeigt sind, und zusätzlich 25 weitere Klone verwendet. Die Sequenz umfaßt 7102 Nukleotide, wobei das 3'-terminale Poly-A nicht mitgezählt ist. Sie enthält einen offenen Leserahmen, der für ein Polyprotein einer Länge von 2150 Aminosäuren kodiert. Der Leserahmen beginnt mit einem AUG in Position 611 und endet mit einem UAA Stop Codon 42 Nukleotide vor dem Beginn des Poly-A.

Die 5'-terminale Sequenz wurde aus den Klonen Nr. 61, 100 und 109 erhalten, wobei Fragment A als Primer verwendet wurde (siehe Fig. 3). Spaltung mit PstI ergab ein Fragment konstanter Länge. Durch Sequenzierung wurde festgestellt, daß alle drei Klone die Sequenz TTAAAAC unmittelbar anschließend an Oligo-G enthielten, das von der Integrationsstelle im Plasmid stammt. Daraus wurde der Schluß gezogen, daß diese Klone den 5'-Terminus des HRV2-Genoms darstellen. Diese Sequenz entspricht den ersten 7 Nukleotiden von den drei Poliovirus-Typen, Coxsackie-Virus B1 (Hewlett, M.J. und Florkiewicz, R.Z., 1980, Proc. Natl. Acad. Sci, USA 77, 303 - 307; Stanway, G., Cann, A.J., Hauptmann, R.,

Hughes P., Clarke, L.D., Mountford, R.C., Minor, P.D., Schild, G.C. und Almond, J.W., 1983, loc. cit.) und HRV-14 (Stanway, G., Hughes, P.J., Mountford, R.C., Minor, P.D. und Almond, J.W., 1984 loc. cit.). Analyse der 610 Nukleotide zwischen dem 5'-terminalen und dem Beginn des langen offenen Leserahmens zeigt das Vorhandensein mehrerer kurzer Leserahmen. Ein Vergleich der Nukleotidsequenz der 5'-terminalen Region des HRV2 mit dem des HRV14 und Poliovirus Typ 1 zeigt einen hohen Grad an Homologie. Unter Berücksichtigung von Insertionen wurden in dieser Region Homologien von 65 % zwischen HRV2 und HRV14 und von 55 % zwischen HRV2 und Poliovirus Typ 1 festgestellt. Dabei ist die Homologie in einigen Bereichen besonders groß. Insgesamt sind 5 verschiedene Blöcke von 16 oder mehr hintereinander liegenden identischen Nukleotiden im 5'-terminalen Bereich des HRV2- und HRV14-Genoms vorhanden. Ein Vergleich von HRV2 und Poliovirus Typ 1 zeigt ebenfalls 5 Blöcke identischer Sequenz, von denen allerdings nur 2 in HRV14 gefunden werden. Dies sind eine Sequenz von 16 Nukleotiden beginnend mit Base 436 und eine Sequenz von 23 Nukleotiden beginnend mit Base 531 in HRV2.

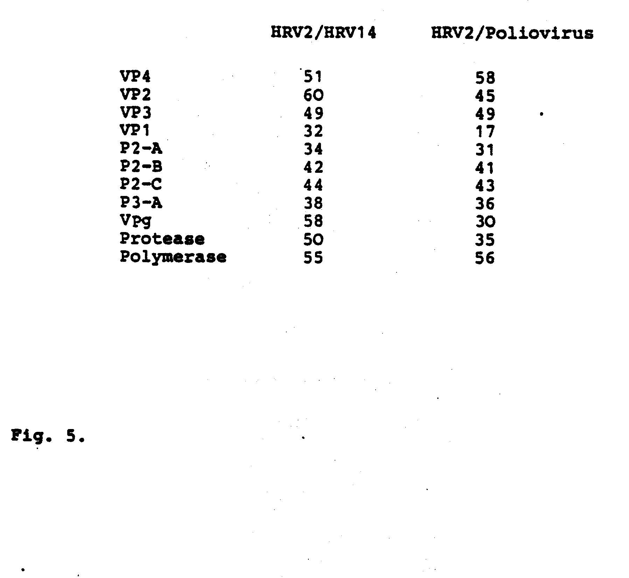

Der Vergleich der Aminosäure-Sequenzen zeigte, daß sich die Bereiche der Homologie auch in der für das Polyprotein kodierenden Region fortsetzen (Fig.5). Besonders auffällig ist, daß die Homologie zwischen HRV2 und HRV14 vielfach nicht oder nur wenig größer ist, als die zwischen HRV2 und Poliovirus Typ 1. Überraschend war die Entdeckung, daß die Homologie zwischen HRV2 und Poliovirus im Bereich des VP4 und in geringfügigem Maß auch in der Polymerase sogar größer ist als die zwischen HRV2 und HRV14. Dies ist umso bemerkenswerter, als nach der klassischen Taxonomie die Rhinoviren als separate Gattung innerhalb der Familie der Picornaviren betrachtet werden. Auch zwischen HRV14 und Poliovirus wurden Homologien gefunden, so daß kürzlich vorgeschlagen worden ist, Rhinoviren und Enteroviren gemeinsam als eine Gattung innerhalb der Familie der Picornaviren zusammenzufassen (Stanway, G., Hughes, P.J., Mountford, R.D., Minor, P.D. und Almond, J.W., 1984, loc.cit.). Daneben gibt es aber auch einige Gene wie VP1, VP2, VPg und die Protease, in denen die Ähnlichkeit zwischen HRV2 und HRV14 wesentlich größer ist als die zwischen HRV2 und Poliovirus. Bemerkenswert ist weiters, daß die geringste Homologie jeweils im Bereich des VP1 zu finden ist.

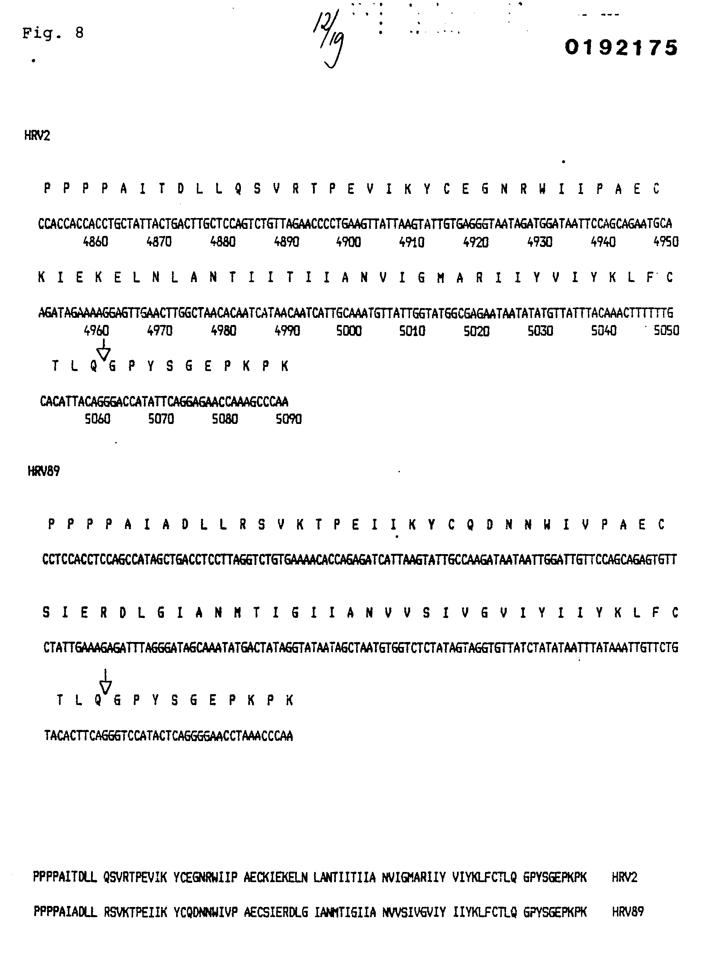

Zur weiteren Unterstützung eines Teiles der auf Grund der beschriebenen Beispiele ermittelten Sequenz wurde ein Vergleich mit einem Genfragment aus dem Rhinovirus HRV89 durchgeführt. Der HRV89 wurde in analoger Weise wie HRV2 gezüchtet. HRV89 wurde im Neutralisationstest durch ein spezifisches Antiserum (ATCC VR-1199 AS/GP) neutralisiert, während ein Antiserum gegen HRV2 (ATCC VR-1112 AS/GP) als Kontrollserum keinen Effekt zeigte. Die Isolierung der viralen RNA, die Charakterisierung, Klonierung, Isolierung der Klone und Sequenzierung wurde in gleicher Weise wie für HRV2 beschrieben, durchgeführt. Fig. 8 zeigt einen Sequenzvergleich mit einer Teilsequenz aus dem HRV89 Klon 34/1, die offensichtlich aus dem Bereich der Gene für P3A und P3B (VPg) stammt. Die weitgehende Homologie in der Aminosäuresequenz ist leicht erkennbar. Selbst an den Stellen, an denen vereinzelt andere Aminosäuren vorkommen, findet man meist eine Substitution durch chemisch sehr nahe verwandte Aminosäuren, wobei Arginin (R) gegen Lysin (K), Valin (V) gegen Isoleucin (I), Leucin (L) gegen Isoleucin (I) etc. ausgetauscht sind. Auch die Spaltstelle zwischen P3A und P3B (VPg) ist vollkommen konserviert, wie auf Grund der Homologie leicht erkennbar ist. Auffällig ist hingegen eine Abweichung in einem kleinen Bereich (entsprechend der Sequenz zwischen den Nukleotiden 5018 und 5029 in HRV2), die zu einer Divergenz in der Aminosäuresequenz in der entsprechenden Region im P3A Anlaß gibt. Die Signifikanz dieses Befundes ist zur Zeit noch nicht geklärt. Über die Funktion des P3A ist bis jetzt nichts bekannt. Es kann daher nicht ausgeschlossen werden, daß Subtypen von HRV2 exisitieren, die in diesem Bereich eine größere Homologie zu HRV89 aufweisen, als in diesem Beispiel dargestellt ist. Daher sind selbstverständlich auch jene Subtypen von HRV2 bzw. solche Rhinoviren, deren Nukleinsäuren wie in den beschriebenen Hybridisierungsbedingungen definiert - mit HRV2 cDNA hybridisieren, und die in diesem Bereich ein größeres Maß an Homologie zu HRV89 aufweisen, Gegenstand der vorliegenden Erfindung.

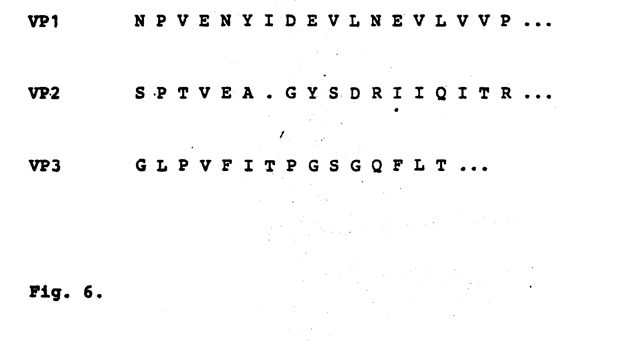

Die viralen Proteine werden aus dem Polyprotein durch proteolytische Spaltung erhalten. Zur Ermittlung der Spaltstellen wurden die viralen Hüllenproteine isoliert und die N-terminale Aminosäuresequenz bestimmt (s.Fig. 6). Hierdurch wurden nicht nur die Spaltstellen in den viralen Hüllenproteinen eindeutig identifiziert, sondern auch das Leseraster,das aus der Nukleotidsequenz abgeleitet worden war, wurde bestätigt. Aus dem Vergleich mit der aus der Nukleotidsequenz abgeleiteten Aminosäuresequenz ergibt sich, daß die VP4/VP2 Spaltung zwischen Glutamin und Serin, die VP2/VP3-Spaltung zwischen Glutamin und Glycin und die VP3/VP1-Spaltung zwischen Glutamin und Asparagin erfolgt.

Die vorliegende Erfindung ermöglicht es, eine DNA zu erzeugen, die die Information für die virale RNA des HRV2 enthält.

Gegenstand der Erfindung sind jedoch nicht nur die Gen-Sequenzen, die spezifisch für die viralen Proteine codieren, sondern auch die Modifikationen, die beispielsweise durch Mutation, Abbau, Transposition oder Addition erhalten werden können. Jede Sequenz, die im Vergleich mit den gezeigten degeneriert ist, ist eingeschlossen. Auch die Sequenzen, die unter stringenten Bedingungen, beispielsweise unter Bedingungen, die für mehr als 85 %, bevorzugt mehr als 90 % Homologie selektieren, mit den gezeigten Sequenzen oder Teilen davon hybridisieren und für die Proteine mit dem viralen Aktivitätsspektrum codieren, sind eingeschlossen. Die Hybridisierungen werden in 6 x SSC/5 x Denhardt's-Lösung/0,1 % SDS bei 65°C durchgeführt. Der Grad der Stringenz wird im Waschschritt festgelegt. So sind die Bedingungen, die für eine Selektionierung auf DNA-Sequenz mit ca. 85 % oder mehr Homologie geeignet sind, wie folgt: 0,2 x SSC/0,01 % SDS/65°C, für die Selektonierung auf DNA-Sequenzen mit ca. 90 % oder mehr Homologie: 0,1 x SSC/0,01 % SDS/65°C.

Diese DNA kann, wie gezeigt, sowohl vollständig als auch in Fragmenten in geeignete Plasmidvektoren eingebaut werden um nach der Transformation von geeigneten Wirtsorganismen entweder die DNA zu vervielfältigen oder die Expression der Proteine selbst zu erreichen. Geeignete Wirte, Vektoren und die Bedingungen für diese Operationen sind dem Fachmann bestens bekannt. Ebenso sind viele Arbeiten publiziert, in denen die Synthese fremder Proteine in Bakterien mit Hilfe gentechnologischer Methoden beschrieben ist (Übersichtsartikel, siehe Harris, T.J.R. in "Genetic Engineering", Williamson, R., Hrsg., 1983, Vol.4. Academic Press, London, 127 ff.). Zu diesem Zweck wird die fremde DNA in die Nähe geeigneter bakterieller Kontrollregionen (Promotoren, Ribosomenbindungsstellen) von Plasmiden eingeführt, die es möglich machen, diese Information - meist in Form von Fusionsproteinen - in hohen Ausbeuten zu exprimieren und so die entsprechenden Proteine zu gewinnen. Auch auf dem Gebiet der Picornaviren gibt es bereits eine Reihe von Publikationen, in denen die Expression viraler Gene in Bakterien beschrieben ist (Küpper, H., Keller, W., Kurz, C., Forss , S., Schaller, H., Franzel, R., Strohmaier, K., Marquardt, O., Zaslavsky, V.G. und Hofschneider, P.H., 1981, Nature (London) 289, 555 - 559; Kleid, D.G., Yansura, D., Small, B., Darbenko, D., Moore, D.M.,

Grubman, M.J., McKercher, P.D., Morgan, D.O., Robertson, B.H. und Bachrach, H.L., 1981, Science 214, 1125 - 1129; Wychowski, C., van der Werf, S., Siffert, O., Crainic, R., Bruneau, P. und Girard, M., 1983, EMBO J. 11, 2019 - 2024; Klump, W., Marquardt, O. und Hofschneider, P.H., 1984, Proc.Nat.Acad. Sci. USA 81, 3351 - 3355; Hanecak, R., Semler, B.L., Ariga, H., Anderson, C.W. und Wimmer, E., 1984, Cell 37, 1063 - 1073).

Für die Expression bevorzugt sind Prokaryoten, beispielsweise E. coli K 12, Stamm 294 (ATCC No. 31 446). Andere Stämme, die geeignet sind, beinhalten E.coli X 1776 (ATCC Nr. 31 537). Ebenso wie die vorerwähnten Stämme können auch E. coli W 3110 $F^-$, $Lambda^-$, Prototroph, ATCC Nr. 27325), Bazillen wie Bacillus subtilis, und andere Enterobacteriaceae, wie Salmonella typhimurium oder Serratia marcescens und verschiedene Pseudomonaden verwendet werden.

Im allgemeinen können Plasmid-Vektoren, die Replikon und Kontrollsequenzen, die aus Spezies stammen, die kompatibel mit den Wirtszellen sind, enthalten, in Verbindung mit diesen Wirten verwendet werden. Der Vektor trägt üblicherweise neben einer Replikationsstelle Erkennungssequenzen, die es ermöglichen, in transformierten Zellen phenotypisch zu selektionieren. Zum Beispiel wird E. coli üblicherweise mit pBR322 transformiert, ein Plasmid, das aus E. coli Spezies stammt (Bolivar, et al., Gene 2, 95 (1977)).

pBR 322 enthält Gene für Ampicillin- und Tetracyclin-Resistenz und liefert damit einfache Mittel, transformierte Zellen zu identifizieren. Das pBR322-Plasmid oder auch andere Plasmide müssen außerdem von sich aus Promotoren enthalten oder müssen dahingehend modifiziert sein, daß sie Promotoren enthalten, die vom mikrobiellen Organismus zur Expression ihrer eigenen Proteine verwendet werden können. Die Promotoren, die am häufigsten bei der Herstellung rekombinanter DNA verwendet werden, beinhalten die Beta-Lactamase (Penicillinase) und Lactose-Promotor-Systeme (Chang et al., Nature 275, 615 (1978); Itakura et al., Science 198, 1056 (1977); Goeddel et al., Nature 281, 544 (1979)) und Tryptophan (trp) Promotor-Systeme (Goeddel et al., Nucleic Acids Res. 8, 4057 (1980); Europa-Anmeldung, Offenlegungs-Nr. 00 36 776). Während die erwähnten die gebräuchlichsten Promotoren sind, sind darüber hinaus auch andere mikrobielle Promotoren entwickelt und benutzt worden. Die erfindungsgemäße Gen-Sequenz kann beispielsweise unter der Kontrolle des Leftward-Promotors des Bakteriophagen Lambda ($P_L$) eingesetzt werden. Dieser Promotor ist einer der als besonders stark bekannten Promotoren, der steuerbar ist. Die Steuerung wird möglich durch den Lambda-Repressor, von dem benachbarte Restriktionsschnittstellen bekannt sind.

Ein temperaturempfindliches Allel dieses Gens kann in einem Vektor, der eine virale DNA-Sequenz enthält, eingefügt werden. Wird die Temperatur auf 42°C erhöht, wird der Repressor inaktiviert und der Promotor bis zu seiner maximalen Konzentration exprimiert. Die Summe der mRNA, die unter diesen Bedingungen produziert wird, sollte ausreichend sein, um eine Zelle zu erhalten, die unter ihren neuen synthetischen Ribonukleinsäuren ungefähr 10 % enthält, die von dem $P_L$-Promotor stammt. Auf diese Weise ist es möglich, eine Clon-Bank zu etablieren, in der eine funktionelle virale DNA-Sequenz

in Nachbarschaft zu einer Ribosom-Bindungsstelle plaziert wird in variierenden Abständen zu dem Lambda-$P_L$-Promotor. Diese Klone können dann überprüft und der mit der höchsten Ausbeute selektiert werden.

Die Expression und Translation einer viralen DNA-Sequenz kann auch unter Kontrolle anderer Regulationssysteme, die als "homolog" zu dem Organismus in seiner untransformierten Form gelten können, ablaufen. So enthält z.B. chromosomale DNA von einem Lactose-abhängigen E. coli ein Lactose oder Lac-Operon, der durch Ausschüttung des Enzyms Beta-Galactosidase den Lactose-Abbau ermöglicht.

Die Lac-Kontrollelemente können aus dem Bacteriophagen Lambda-plac5, der infektiös für E. coli ist, erhalten werden. Das Lac-Operon des Phagen kann durch Transduktion aus derselben Bakterien-Spezies stammen. Regulationssyteme, die bei dem erfindungsgemäßen Verfahren Verwendung finden können, können aus plasmidischer DNA stammen, die dem Organismus eigen ist. Das Lac-Promotor-Operator-System kann durch IPTG induziert werden.

Andere Promotor-Operator-Systeme oder Teile hiervon können genausogut verwendet werden: beispielsweise Arabinose-Operator, Colicine $E_1$-Operator, Galactose-Operator, alkalischer Phosphatase-Operator, trp-Operator, Xylose A operator, tac-Promotor u.ä..

Die Gene können vorzugsweise in dem Expressions-Plasmid pER103 (E. Rastl-Dworkin et al., Gene 21, 237-248 (1983); vergleiche auch europäische Patentanmeldung No. 83112812.9, Hinterlegung DSM 2773, 20. Dezember 1983) exprimiert werden. Diese Vektoren enthalten alle Regulationselemente die zu einer hohen Expressionsrate der klonierten Gene führen.

Zusätzlich zu Prokaryoten können auch eukaryotische Mikroorganismen, wie Hefekulturen verwendet werden. Saccharomyces cerevisiae ist der am meisten verwendete unter den eukaryotischen Mikroorganismen, obwohl eine Anzahl anderer Spezies allgemein erhältlich ist. Zur Expression in Saccharomyces wird beispielsweise das Plasmid YRp7 (Stinchcomb, et al. Nature 282, 39 (1979); Kingsman et al., Gene 7, 141 (1979); Tschumper, et al., Gene 10, 157 (1980)) und das Plasmid YEp 13 (Bwach et al., Gene 8, 121-133 (1979)) üblicherweise verwendet. Das Plasmid YRp7 enthält das TRP1-Gen, das eine Selektionierungsmarkierung für eine Hefemutante, die unfähig ist, in tryptophanfreiem Medium zu wachsen, bereitstellt; beispielsweise ATCC Nr. 44076.

Das Vorhandensein des TRP1 Schadens als Charakteristikum des Hefe-Wirts Genoms stellt dann ein wirksames Hilfsmittel dar, um die Transformation nachzuweisen, indem ohne Tryptophan kultiviert wird. Ganz ähnlich verhält es sich bei dem Plasmid YEp13, das das Hefe-Gen LEU 2, das zur Ergänzung einer LEU-2-Mutante verwendet werden kann, enthält. Geeignete Promotor-Sequenzen für Hefe Vektoren beinhalten die 5'-flankierende Region des ADH I (Ammerer, G., Methods of Enzymology 101, 192-201 (1983)), 3-Phosphoglycerat-Kinase (Hitzeman, et al., J. Biol. Chem. 255, 2073 (1980) oder andere glykolytische Enzyme (Kawasaki und Fraenkel, BBRC* 108, 1107-1112 (1982)), wie Enolase, Glycerinaldehyd-3-phosphat-Dehydrogenase, Hexokinase, Pyruvat-Decarboxylase, Phosphofructokinase, Glucose-6-Phosphat-Isomerase, und Glucokinase. Bei der Konstruktion geeigneter Expressions-Plasmide können die mit diesen Genen assoziierten Terminationssequenzen ebenfalls in den Expressions-Vektor am 3'-Ende der zu exprimierenden Sequenz eingesetzt werden, um Polyadenylierung und Termination der mRNA vorzusehen.

*Biochem.Biophys. Res. Comm.

Andere Promotoren, die zudem noch den Vorteil der durch Wachstumsbedingungen kontrollierten Transkription besitzen, sind die Promotor-Regionen der Alkohol-Dehydrogenase-2, Isocytochrom C, Saure Phosphatase, abbauende Enzyme, die mit dem Stickstoff-Metabolismus gekoppelt sind, die obenerwähnte Glyceraldehyd-3-Phosphat Dehydrogenase und Enzyme, die für die Verarbeitung von Maltose und Galaktose verantwortlich sind. Promotoren, die durch den Hefe Mating Typ Locus reguliert werden, beispielsweise Promotoren der Gene BARI, MFα1, STE2, STE3, STE5 können bei temperaturregulierten Systemen durch die Verwendung von temperaturabhängigen sir Mutationen eingesetzt werden. (Rhine, Ph.D. Thesis, University of Oregon, Eugene, Oregon (1979), Herskowitz and Oshima, The Molecular Biology of the Yeast Saccharomyces, part I, 181-209 (1981), Cold Spring Harbor Laboratory). Diese Mutationen beeinflussen die Expression der ruhenden Mating Typ Kassetten von Hefen und dadurch indirekt die Mating Typ abhängigen Promotoren. Generell ist jedoch jeder Plasmid-Vektor, der einen Hefekompatiblen Promotor, originäre Replikations- und Terminationssequenzen enthält, geeignet.

Zusätzlich zu Mikroorganismen sind Kulturen multizellulärer Organismen ebenfalls geeignete Wirtsorganismen. Im Prinzip ist jede dieser Kulturen einsetzbar, ob von Wirbeltier- oder wirbellosen Tierkulturen. Größtes Interesse besteht jedoch an Wirbeltier-Zellen, so daß die Vermehrung von Wirbeltierzellen in Kultur (Gewebe-Kultur) in den letzten Jahren zu einer routinemäßigen Methode wurde.

(Tissue Culture, Academic Press, Kruse and Patterson, Editors (1973)). Beispiele solcher nützlicher Wirtszellinien sind VERO- und HeLa-Zellen, Hamster-Eierstock (CHO)-Zellen und WI38, BHK, COS-7 und MDCK-Zellinien. Expressionsvektoren für diese Zellen enthalten üblicherweise (wenn nötig) eine Replikationsstelle, einen Promotor der vor dem zu exprimierenden Gen lokalisiert ist, gemeinsam mit jeder notwendigen Ribosomenbindungsstelle, RNA-Spleißstelle, Polyadenylierungsstelle und transkriptionelle Terminations-Sequenzen.

Bei der Verwendung in Säugetierzellen werden die Kontrollfunktionen auf den Expressions-Vektoren oftmals aus viralem Material vorgesehen. Beispielsweise stammen die üblicherweise verwendeten Promotoren aus Polyoma,Adenovirus 2, und besonders häufig aus Simian Virus 40 (SV 40). Die Anfangs- und Endpromotoren des SV 40 sind besonders nützlich, da beide leicht aus dem Virus als Fragment zu erhalten sind, das auch noch die virale Replikationsstelle des SV 40 enthält (Fiers et al., Nature 273, 113 (1978)). Auch können kleinere oder größere Fragmente des SV 40 verwendet werden, vorausgesetzt, sie enthalten die annähernd 250 bp lange Sequenz die von der Hind III Schnittstelle bis zur Bgl 1 Schnittstelle in der viralen Replikationsstelle reicht. Außerdem ist es ebenfalls möglich und oft empfehlenswert, Promotor- oder Kontroll-Sequenzen zu verwenden, die normalerweise mit den gewünschten Gensequenzen verknüpft sind, vorausgesetzt, diese Kontroll-Sequenzen sind kompatibel zu den Wirtszellsystemen.

Eine Replikationsstelle kann entweder durch entsprechende Vektorkonstruktion vorgesehen werden, um eine exogene Stelle einzubauen, beispielsweise aus SV 40 oder anderen viralen Quellen (z.B. Polyoma, Adeno, VSV, etc.)

....

oder kann durch die chromosomalen Replikationsmechanismen der Wirtszelle vorgesehen werden. Wird der Vektor in das Wirtszellenchromosom integriert, reicht die zuletztgenannte Maßnahme meistens aus.

Transformation der Zellen mit den Vehikeln kann durch eine Vielzahl an Verfahren erreicht werden. Beispielsweise kann sie durch Kalzium erfolgen, wobei entweder die Zellen in Magnesium gewaschen werden und die DNA den in Kalzium suspendierten Zellen zugegeben wird oder die Zellen einem Kopräzipitat von DNA und Kalziumphosphat ausgesetzt werden. Bei nachfolgender Genexpression werden die Zellen auf Medien übertragen, die für transformierte Zellen selektieren.

Nach erfolgter Transformation des Wirtes, Expression des Gens und Fermentation oder Zellkultivierung unter Bedingungen, bei denen das Protein exprimiert wird, kann das Produkt üblicherweise durch bekannte chromatographische Trennmethoden extrahiert werden, um so ein Material zu erhalten, das das virale Protein mit oder ohne Leader und Tailing-Sequenzen enthält. Das Protein kann mit einer Leader-Sequenz am N-Terminus exprimiert werden (Pre-Protein), die von einigen Wirtszellen entfernt werden kann. Wenn nicht, so ist eine Abspaltung des Leader-Polypeptids (wenn vorhanden) erforderlich, um reifes Protein zu erhalten. Alternativ kann das reife Protein im Mikroorganismus direkt produziert werden. Dazu kann die Precursor Sequenz des Hefe-Mating-Pheromons MF-alpha-1 verwendet werden, um eine korrekte "Reifung" des fusionierten Proteins und die Ausscheidung der Produkte in das Wachstumsmedium oder den periplasmischen Raum zu gewährleisten. Die DNA-Sequenz für funktionelles oder reifes Protein kann mit MF-alpha-1 an der vermuteten Schnittstelle verbunden sein.

Neben der Verwendung der erfindungsgemäßen DNA zur Herstellung der betreffenden Proteine in Bakterien oder eukaryontischen Zellen kann sie auch dazu dienen, die aus der Nukleotidsequenz abgeleiteten Aminosäuresequenzen synthetisch, ganz oder in Teilen davon, herzustellen.

Diese Oligopeptide können dann, ebenso wie die gentechnologisch hergestellten Proteine entweder zur Anregung einer gegen intakte Viren gerichteten Immunantwort verwendet oder zur Bindung und Blockierung von zellulären Rezeptoren eingesetzt werden. Studien über die Verwendung von Oligopeptiden zur Anregung einer gegen Polioviren gerichteten Immunantwort sind publiziert worden (Emini, E.A., Jameson, B.A. und Wimmer, E., 1983, Nature (London) 30, 699-703; ähnliche Untersuchungen sind auch bei Maul- und Klauenseucheviren durchgeführt worden (Bittle, J.L., Houghton, R.A., Alexander, H., Shinnick, T.M., Sutcliffe, J.G., Lerner, R.A., Rowlands, D.J. und Brown, F., 1982 Nature (London), 298, 30 - 33; Pfaff, E., Mussgay, M., Böhm, H.O., Schulz, G.E. und Schaller, H., 1982, EMBO J. 1, 869 - 874). Diese Erfindung erstreckt sich auch auf die Oligo- und Polypeptidanteile von HRV2-Proteinen, die als Folge der Synthese oder zum Zwecke der Applikation, z.B. als Vakzine, mit anderen Oligo- oder Polypeptiden verbunden sind.

Gegenstand der vorliegenden Erfindung sind im Einzelnen:

DNA-Moleküle, die für mindestens ein virales Protein des Rhinovirusstammes HRV2 codieren,

- die der gesamten viralen RNA oder Teilen der viralen RNA der Rhinovirusstämme HRV2 und HRV89 entsprechen

- die für ein virales Protein codieren ,das aus den viralen Proteinen VP1, VP2, VP3, VP4, P2A, P2B, P2C, P3A und P3C zusammengesetzt ist,

- die für ein virales Protein codieren, das aus mindestens zwei der genannten, in beliebiger Kombination miteinander verknüpften Proteine besteht

- oder DNA-Moleküle, die für die einzelnen viralen Proteine selbst codieren.

Bevorzugt sind DNA-Moleküle, die vollständig oder in Teilen der Sequenz gemäß Fig.4 entsprechen.

Ebenfalls Gegenstand der Erfindung sind DNA-Moleküle, die für mindestens ein virales Protein des Rhinovirusstammes HRV2 codieren, die unter stringenten Bedingungen, die es erlauben, eine Homologie zu erkennen, die größer als 85% ist, mit einem der angegebenen DNA-Moleküle oder mit degenerierten Variationen dieser Moleküle hybridisieren. Die erfindungsgemäßen DNA-Moleküle können in ein geeignetes Expressionsvehikel, beispielsweise in ein Plasmid, das replizierbar in Mikroorganismen, vorzugsweise in Prokaryoten, Eukaryoten oder in Säugetierzellen ist, eingebaut werden. Auch sind DNA-Moleküle, die für Proteine codieren, die die biologische Aktivität zumindest von einem der erfindungsgemäßen Proteine aufweisen, Gegenstand der vorliegenden Erfindung.

Weiterhin sind Gegenstand der Erfindung transformierte Wirtsorganismen, die die für die viralen Proteine gemäß der Erfindung codierenden genetischen Informationen enthalten, vorzugsweise Prokaryoten, Eukaryoten oder Säugetierzellinien, insbesondere E.coli.

Die genetische Information ist dabei vorzugsweise in Vehikeln enthalten, die in dem betreffenden Wirtsorganismus replizierbar sind.

Gegenstand der Erfindung sind ferner Polypeptide, die die biologische Aktivität mindestens eines der viralen Proteine des Rhinovirusstammes HRV2 aufweisen und/oder von einem der beschriebenen DNA-Moleküle codiert werden, insbesondere Polypeptide mit der Aminosäuresequenz für VP1, VP2, VP3, VP4, P2A, P2B, P2C, P3A oder P3C vorzugsweise mit der Aminsosäuresequenz gemäß Figur 4 oder Teilen hiervon. Polypeptide, die in Teilen oder insgesamt mindestens einem der viralen Proteine des Rhinovirusstammes HRV2 entsprechen, Polypeptide, die mindestens zwei der viralen Proteine in beliebiger Kombination und Reihenfolge verknüpft enthalten und Polypeptide, die aus der Aminosäuresequenz gemäß. Fig.4 oder aus Teilen hiervon abgeleitet sind und/oder an die zellulären Rezeptoren für die Rhinoviren Stamm HRV2 binden und/oder diese blockieren sind ebenfalls Gegenstand der vorliegenden Erfindung.

Die DNA-Moleküle gemäß der Erfindung werden hergestellt, indem die virale RNA des Rhinovirusstammes HRV2 isoliert, die hierzu komplementäre DNA hergestellt, das virale cDNA/RNA Hybrid in einen geeigneten replizierbaren Vektor eingebaut und ein geeigneter Wirtsorganismus mit diesem Vektor transformiert wird.

Die erfindungsgemäßen Polypeptide werden erhalten, indem ein geeigneter Wirtsorganismus, vorzugsweise ein Prokaryot, Eukaryot oder eine Säugetierzelle insbesondere E.coli mit den erfindungsmäßen genetischen Informationen, die für ein virales Polypeptid gemäß der Erfindung codieren, transformiert, die Information exprimiert und ein virales Polypeptid gemäß der Erfindung isoliert wird.

Verwendet werden können die erfindungsgemäßen Polypeptide zur therapeutischen Behandlung, beispielsweise zur Stimulierung des Immunsystems und zur Bindung und/oder Blockierung der zellulären Rezeptoren für die Rhinoviren Stamm HRV2 .

Eingesetzt werden können die erfindungsgemäßen Polypeptide in Form von Arzneimitteln, die neben pharmazeutisch inerten Hilfs- oder Trägerstoffen eine wirksame Menge mindestens eines der erfindungsgemäßen Polypeptide enthalten.

Weitere Eigenschaften und Merkmale der vorliegenden Erfindung sind in den folgenden Beispielen beschrieben, die Ausführungsbeispiele der vorliegenden Erfindung betreffen und diese nicht einschränken.

Wird im Rahmen dieser Anmeldung von biologischer Aktivität im Zusammenhang mit Proteinen (Polypeptiden) gesprochen, so bedeutet dies, daß das betreffende Protein (Polypeptid)im biologischen Test eine Immunantwort stimulieren und/oder irgendeine Reaktion mit den zellulären Rezeptoren für die Rhinoviren eingeht.

## Legende zu den Abbildungen

Fig. 1 Elektrophoretische Trennung der Hüllenproteine von HRV2 auf einem 12,5 %igen Polyacrylamid-Gel in Gegenwart von Natriumdodecylsulfat. VP4 ist nicht sichtbar, da es mit der Front aus dem Gel herausgewandert ist.

Fig. 2 Elektrophorese von HRV2-RNA auf einem 2%igen Agarose-Gel in Gegenwart von Natriumdodecylsulfat. Rechts sind die Positionen der ribosomalen RNA Marker gezeigt.

Fig. 3 Restriktionskarte des HRV2 Genoms. 17 überlappende Klone, die zur Sequenzierung herangezogen wurden,sind gezeigt. Charakteristische Schnittstellen einiger Restriktionsenzyme sind angeführt. Die Pfeile (A,B) repräsentieren Restriktionsfragmente, die als Primer für die reverse Transkription verwendet wurden.

Fig. 4 Sequenz des klonierten HRV2 Genoms und die davon abgeleitete Aminosäuresequenz. Spaltstellen im Polyprotein sind durch Pfeile dargestellt. Ausgefüllte Pfeile ( ↓ ) zeigen experimentell ermittelte Spaltstellen an, offene Pfeile ( ⇩ ) zeigen auf Grund der Homologie mit anderen Picornaviren vorhergesagte Spaltstellen im Polyprotein an.

Fig. 5 Vergleich der Homologie in der Aminosäuresequenz (in %) einzelner Gene zwischen HRV2, HRV14 und Poliovirus Typ 1.

Fig. 6 N-terminale Aminosäuresequenzen der Hüllenproteine von HRV2.

Fig. 7 Identifikation des viralen Kapsidproteins VP1 als stärkstes Antigen von HRV2 in Kaninchen.

Fig. 8 Vergleich der Aminosäuren- und Nukleotidsequenzen aus dem Bereich der Gene für P3A und P3B (VPg) von HRV2 und HRV89. Die auf Grund der Homologie mit anderen Picornaviren vorhergesagte Spaltstelle ist mit einem offenen Pfeil markiert.

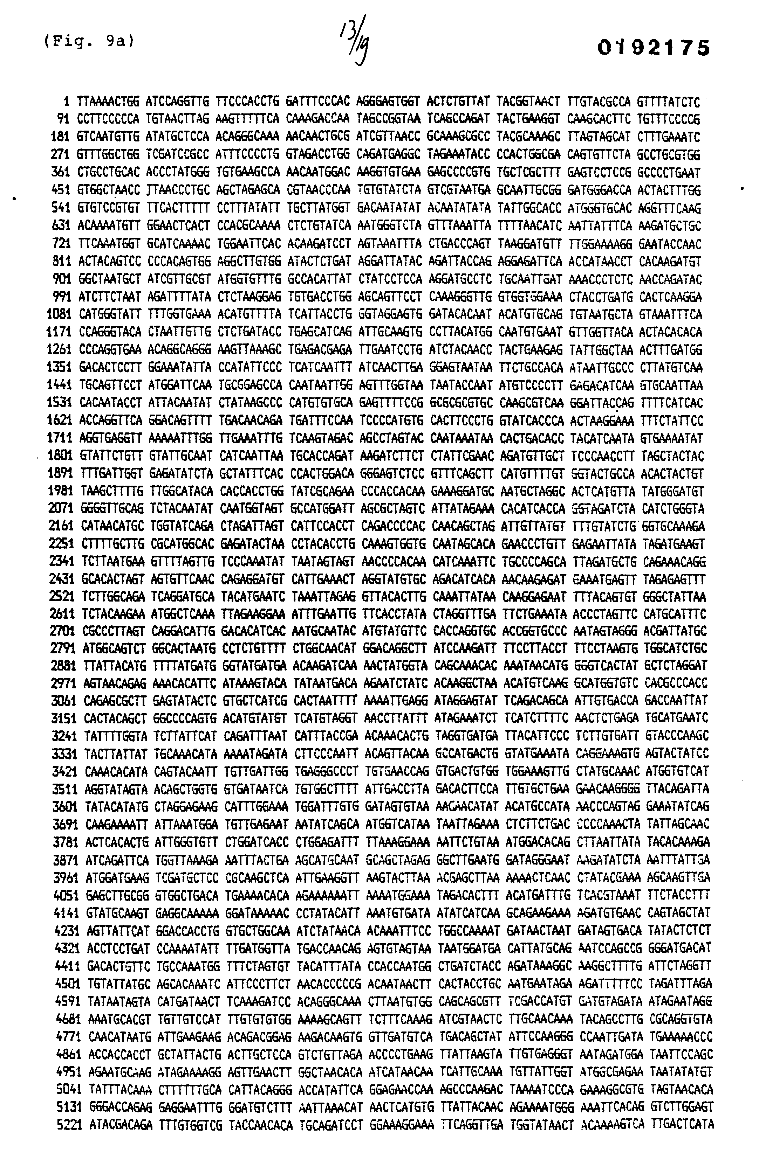

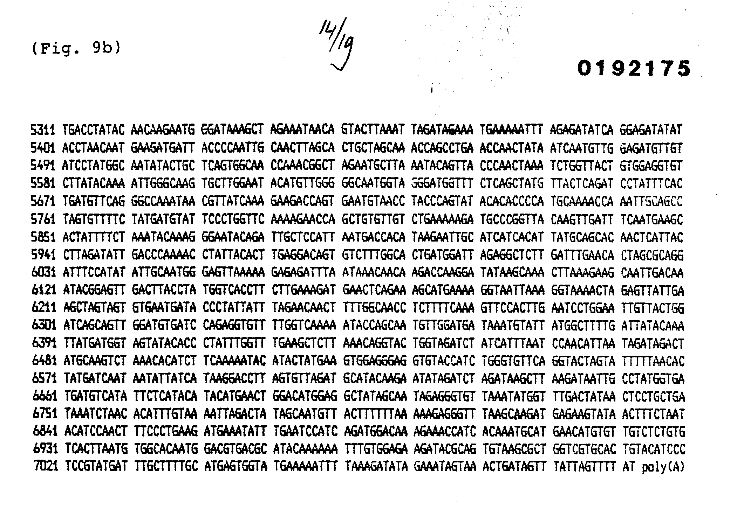

Fig. 9 komplette Nukleotidsequenz des HRV2

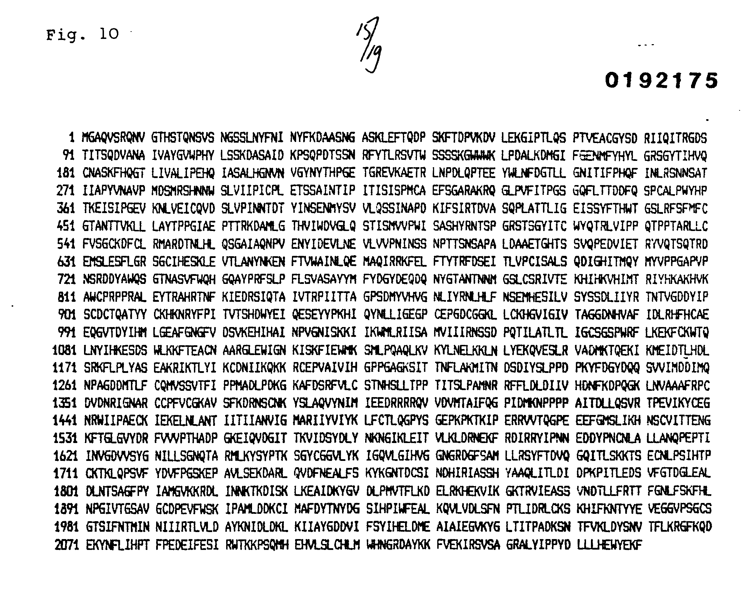

Fig.10 Aminosäuresequenz des Polypeptides HRV2

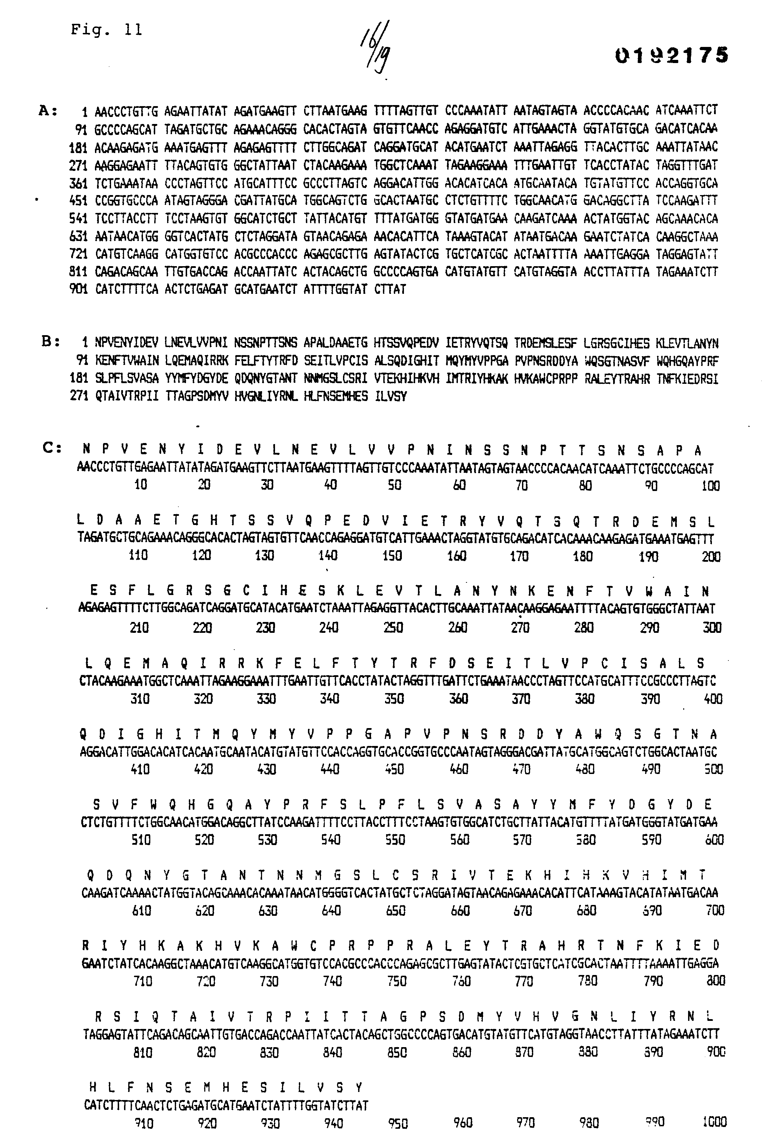

Fig.11 A: Nukleotidsequenz des Polypeptides VP1
B: Aminosäuresequenz des Polypeptides VP1
C: Übersetzte Nukleotidsequenz des Polypeptides VP1

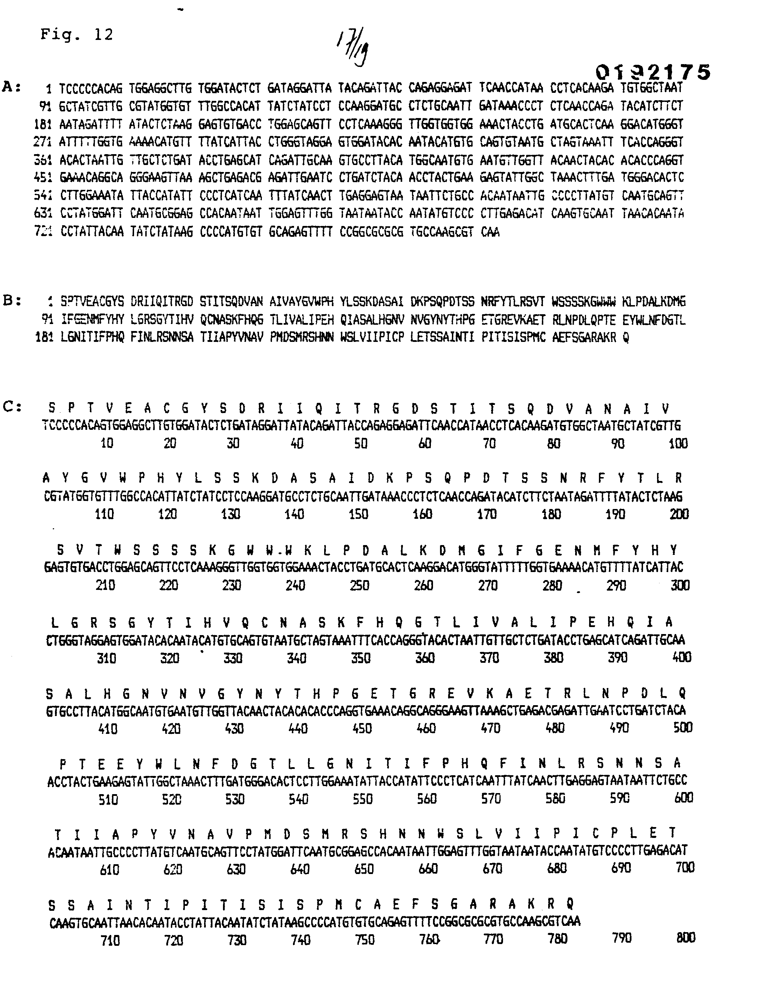

Fig.12 A: Nukleotidsequenz des Polypeptides VP2
B: Aminosäuresequenz des Polypeptides VP2
C: Übersetzte Nukleotidsequenz des Polypeptides VP2

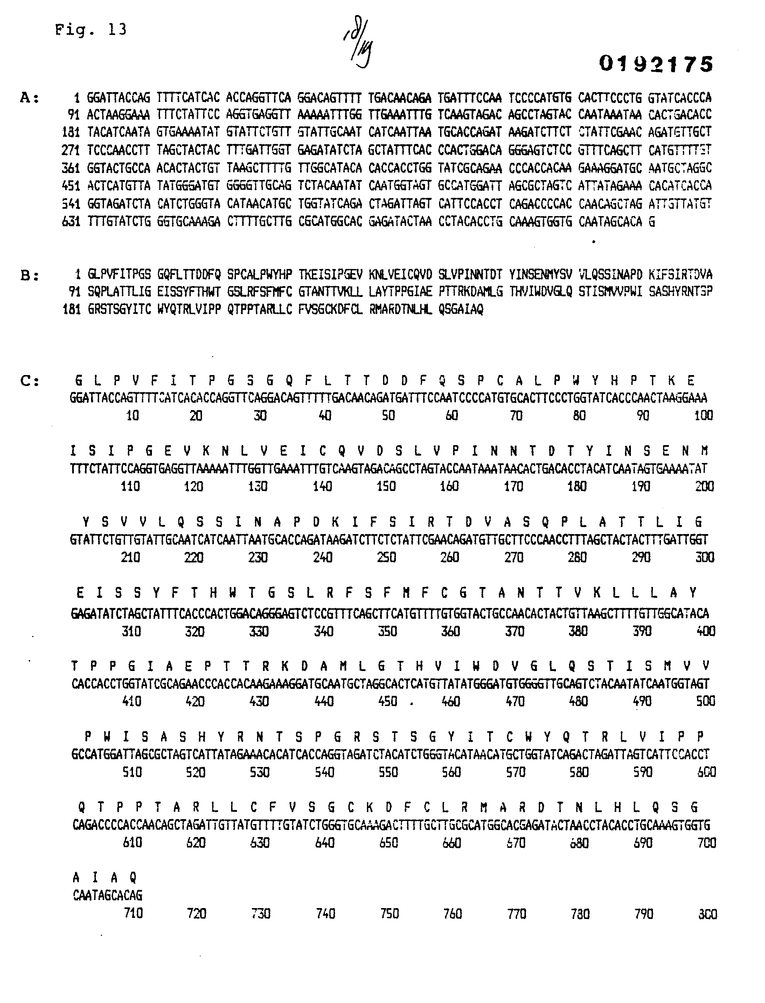

Fig.13 A: Nukleotidsequenz des Polypeptides VP3
B: Aminosäuresequenz des Polypeptides VP3
C: Übersetzte Nukleotidsequenz des Polypeptides VP3

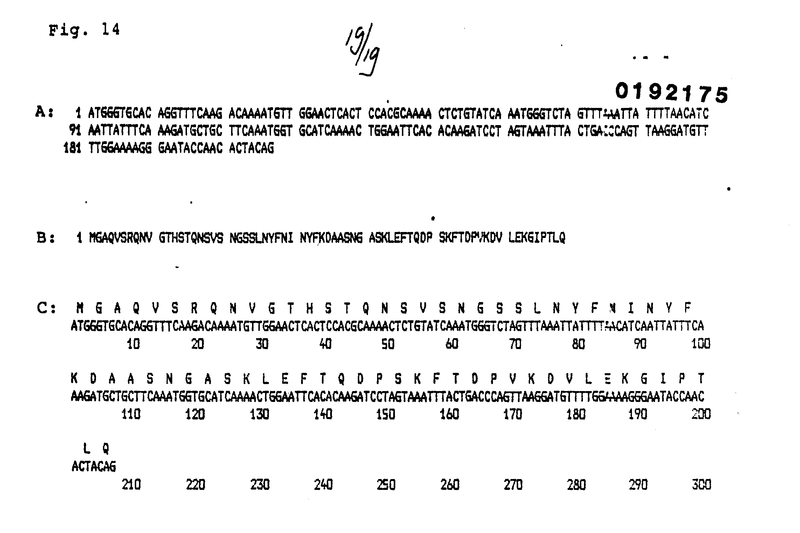

Fig.14 A: Nukleotidsequenz des Polypeptides VP4
B: Aminosäuresequenz des Polypeptides VP4
C: Übersetzte Nukleotidsequenz des Polypeptides VP4

Beispiel 1

Präparation von HRV2

HeLa-Zellen (Stamm HeLa-Ohio, 03-147, Flow Laboratories, England) wurden in Suspension bei 37°C gezüchtet. Das Suspensionsmedium (Thomas, D.C., Conant, R.M. und Hamparian, V.U., 1970, Proc. Soc. Exp. Biol. Med. 133, 62 - 65; Stott, E.J. und Heath, G.F., 1970, J. gen. Virol, 6, 15 - 24) bestand aus einer Joklik-Modifikation von MEM für Suspension (Gibco 072-1300) und 7 % Pferdeserum (Seromed 0135). Die Inokulationsdichte betrug 5 - 10 x $10^4$ Zellen/ml, das Volumen 500 ml. Die Suspension wurde bei einer Zelldichte von 1 x $10^6$ Zellen/ml unter sterilen Bedingungen bei 300 g 10 min zentrifugiert. Der Überstand wurde abgesaugt und die Zellen in 100 ml Infektionsmedium (Joklik-Modifikation von MEM für Suspensionskultur mit 2 % Pferdeserum und 2 mM $MgCl_2$) resuspendiert. Durch mehrmaliges vorsichtiges Aufsaugen in einer 20 ml Pipette wurden die Zellen homogen im Infektionsmedium verteilt. Danach wurde auf 500 ml aufgefüllt. Anschließend wurde die Zellsuspension auf 34°C gebracht und mit HRV2 (zweimal Plaque - gereinigt) bei einer Multiplizität von 0,1 Viren/Zelle infiziert. Der HRV2 Stamm wurde von Dr. D. Tyrrell (Common Cold Center, Salisbury, England) erhalten und kann auch von der American Type Culture Collection (ATCC VR-482 und ATCC VR-1112) bezogen werden. Der verwendete Stamm wurde durch Antiserum gegen HRV2 (American Type Culture Collection, Cat.No. ATCC VR-1112 AS/GP) neutralisiert. Als Kontrollserum diente ein Antiserum gegen HRV7 (Cat.No. ATCC VR-1117 AS/GP), das keine Neutralisation zeigte. Nach 40 Stunden bei 34°C wurde das Virus geerntet.

Virus wurde sowohl aus den Zellen bzw. Zellfragmenten als auch aus dem Medium gewonnen. Zu diesem Zweck wurde das Medium von infizierten Zellen und Zellfragmenten durch eine 10 min Zentrifugation bei 1500 g abgetrennt und abgesaugt. Der Niederschlag wurde bei -70° C eingefroren.

Die Zellniederschläge von 12 l Suspensionskultur wurden vereinigt, in 40 ml TM-Puffer (20 mM Tris/HCl, pH 7,5, 2 mM $MgCl_2$) resuspendiert, 15 min auf Eis gestellt, anschließend im Dounce-Homogenisator aufgebrochen und das Gemisch 30 min bei 6000 g zentrifugiert. Der Niederschlag wurde anschließend noch einmal in 10 ml TM-Puffer gewaschen. Die beiden Überstände wurden vereinigt und 3 Stunden bei 5000 g zentrifugiert, um das Virus zu pelletieren. Das Viruspellet wurde dann in 10 ml KTMP-Puffer (50 mM KCl, 50 mM Tris/HCl, pH 7,5, 5 mM $MgCl_2$, 2 mM Mercaptoethanol, 1 mM Puromycin, 0,5 mM GTP) aufgenommen und nach Zugabe von 150 µg DNase I (Sigma, Ribonukleasefrei) 1 Stunde auf Eis inkubiert.

Aus dem Infektionsmedium wurde das Virus unter Rühren bei 4°C mit Polyethylenglykol 6000 (PEG 6000; Merck) bei einer Konzentration von 7 % und 450 mM NaCl gefällt (Korant, B.D., Lonberg-Holm, K., Noble, J. und Stasny, J.T., 1972, Virology <u>48</u>, 71 - 86). Nach 4 Stunden in der Kälte wurde das Virus 30 min bei 1500 g abzentrifugiert, der Niederschlag in 10 ml KTMP-Puffer, der 75 µg DNase I enthielt, resuspendiert, das Gemisch 1 Stunde auf Eis inkubiert und anschließend bei -70°C eingefroren.

Die Virussuspensionen, die aus den Zellen und aus dem Medium gewonnen worden waren, wurden vereinigt, 5 min bei 37°C inkubiert, durch Zugabe von 60 ml kaltem TE-Puffer (10 mM Tris/HCl, pH 7,4, 1 mM EDTA) abgekühlt und anschließend 5 min lang im Eisbad sonikiert.

Danach wurde 30 min bei 6000 g zentrifugiert. Zum Überstand wurden 920 ml TE-Puffer, der 7 % PEG 6000 und 450 mM NaCl enthielt, hinzugefügt, 4 Stunden vorsichtig bei 4°C gerührt und das entstandene Präzipitat 30 min bei 6000 g pelletiert. Der Niederschlag wurde abermals in 100 ml TM-Puffer aufgenommen, das Virus wie oben durch Zugabe von PEG 6000 und NaCl gefällt und pelletiert. Der Niederschlag wurde in 40 ml TM-Puffer resuspendiert, die Suspension 30 min bei 6000 g zentrifugiert, und das Virus 3 Stunden bei 85000 g pelletiert. Das Präzipitat wurde in 1 ml TM-Puffer gelöst, nach Zugabe von 50 µg DNase I 1 Stunde bei 4°C inkubiert und anschließend 1 ml TE-Puffer hinzugefügt. Zur weiteren Reinigung wurde die Virussuspension auf Saccharosegradienten (10 - 30 % w/w in TE-Puffer) 4 Stunden bei 4°C bei 85000 g zentrifugiert. Aus der Extinktion bei 260 nm wurden die das Virus enthaltenden Fraktionen ermittelt und mit TM-Puffer verdünnt, so daß die Endkonzentration an Saccharose 10 % betrug. Danach wurde 8 Stunden bei 85000 g zentrifugiert. Das Viruspellet wurde in 1 ml TM-Puffer aufgenommen und bei -70°C aufbewahrt. Zur Überprüfung der Reinheit der Viruspräparation wurde eine Elektrophorese auf einem 12,5 %igen Polyacrylamid-Gel in Gegenwart von 0,1 % Natriumdodecylsulfat durchgeführt (Laemmli, U.K., 1970, Nature (London) 277, 680-685) und die Proteinbanden mit Coomassie-Brillant-Blau angefärbt. Ein typisches Bild des Proteinmusters einer Präparation von HRV2 ist in Fig. 1 gezeigt.

## Beispiel 2

### Klonierung des cDNA-RNA-Hybrids

### RNA-Extraktion aus der HRV2-Präparation

Das Virus wurde in 1 ml NTES-Puffer (100 mM NaCl, 10 mM Tris/HCl, pH 9, 1 mM EDTA, 0,1 % Natriumdodecylsulfat), suspendiert und mit Phenol extrahiert. Zur besseren Phasentrennung wurde Chloroform zugesetzt. Zur wässrigen Phase wurden 20 µl 5 M NaCl und 20 µl 3 M Natriumacetat (pH 5,6) zugegeben und die virale RNA mit dem zweifachen Volumen Ethanol gefällt.

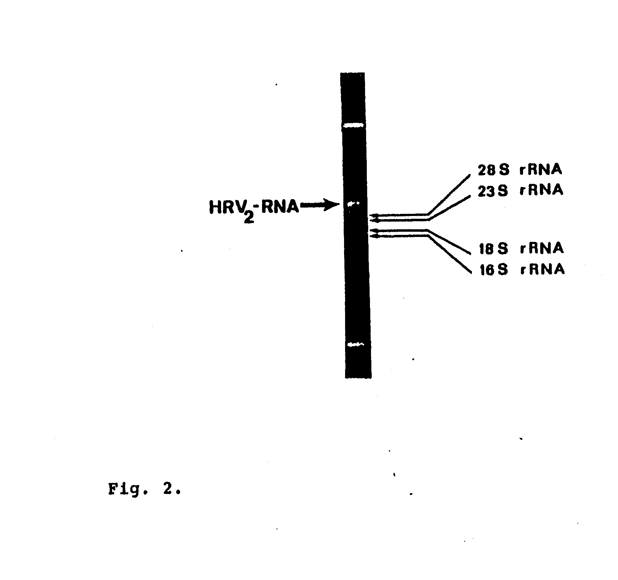

Um das kovalent an das 5'-Ende der viralen RNA gebundene VPg zu entfernen, wurde die RNA anschließend in NTES-Puffer mit 1 mg/ml Proteinase K (Merck) 15 min bei 37°C verdaut. Um Kontaminationen von Ribonuklease zu entfernen, war die Proteinase K Stamm-Lösung (50 mg/ml) vor Gebrauch 15 min bei 37°C vorinkubiert worden. Nach Beendigung des Proteinase K-Verdaus wurde die Lösung, wie oben beschrieben, mit Phenol/Chloroform extrahiert und die RNA durch Zugabe von Ethanol gefällt. Ein kleiner Teil der RNA wurde anschließend auf einem 2 %igen Agarose-Gel in TAE-Puffer (10 mM NaAc, 40 mM Tris/Acetat, pH 8,2, 2 mM EDTA) mit 0,1 % Natriumdodecylsulfat elektrophoretisch aufgetrennt. Nach Anfärben mit Ethidiumbromid war die Bande der intakten HRV2-RNA sichtbar (Fig. 2). Eine darunter liegende, schwache, diffuse Bande zeigte einen geringfügigen Abbau der HRV2-RNA an.

Reverse Transkription der HRV2-RNA mit Oligo-dT als Primer

4 µg HRV2-RNA wurden in 10 µl $H_2O$ gelöst, 5 µl 10 x RT-Puffer (1 x RT-Puffer = 100 mM KCl, 10 mM $MgCl_2$, 50 mM Tris/HCl, pH 8,3), 5 µg Oligo-dT (12 - 18) (Pharmacia P-L Biochemicals), 10 µCi [α-$^{32}$P]-dCTP (3000 Ci/mmol Amersham International, England), 100 U Reverse Transkriptase (Anglian Biotechnology Co, Cambridge) und je 20 nmol von dATP, dGTP, dTTP, dCTP hinzugefügt, und in einem Gesamtvolumen von 50 µl 2 Stunden bei 42°C inkubiert. Nach Zugabe von 2 µl 250 mM EDTA (pH 8) wurde mit Phenol/Chloroform extrahiert und die wässrige Phase auf eine Biogel P30 (oder Sephadex G-25)-Säule in einer Pasteur-Pipette aufgetragen. Zur Elution diente ein TE-Puffer. Das gebildete cDNA-RNA-Hybrid wurde so von überschüssigem [α-$^{32}$P]dCTP abgetrennt und nach Zugabe von 1/10 Volumsanteilen 3 M Natriumacetat (pH 5,6) und 2 Volumsanteilen Ethanol präzipitiert.

Homopolymeres Verlängern der HRV2-RNA-cDNA-Hybride ("Tailing")

Das Verlängern der HRV2-RNA-cDNA-Hybride wurde nach der Methode von Roychoudhury und Wu (1980, loc.cit.) durchgeführt. Dazu wurde das HRV2-RNA-cDNA-Hybrid in 50 µl TT-Puffer (200 mM Kaliumkakodylat, 25 mM Tris/HCl, pH 6,9, 0,5 mM $CoCl_2$, 2 mM Dithiothreit, in Gegenwart von 2 nmol [α-$^{32}$P]dCTP (5 Ci/mmol)mit 25 U terminaler Transferase (Pharmacia P-L Biochemicals) 5 min bei 37°C inkubiert. Nach Zugabe von 2 µl 0,25 M EDTA (pH 8) wurde mit Phenol/Chloroform extrahiert, das Reaktionsgemisch anschließend an einer Biogel P30-Säule, wie oben beschrieben, chromatographiert und das Oligo-dC tragende-RNA-cDNA-Hybrid mit Ethanol präzipitiert.

Einbau des Oligo-dC-tragenden HRV2-RNA-cDNA-Hybrid in das Plasmid pBR322 ("annealing") und Transformation von Escherichia coli HB 101.

Das Oligo-dC tragende RNA-cDNA-Hybrid wurde in 100 µl NTE-Puffer (100 mM NaCl, 10 mM Tris/HCl, pH 7,6, 1 mM EDTA) mit 0,3 pmol pBR322-Plasmid (das mit PstI geschnitten war und an das Oligo-dG-Reste anpolymerisiert waren; Bethesda Research Laboratories) versetzt, erst 5 min bei 65°C, dann 2 Stunden bei 42°C erhitzt, über Nacht langsam auf Raumtemperatur abgekühlt und bei 4°C aufbewahrt.

Für die Zelltransformation wurde der Stamm HB 101* in 50 ml LB-Medium (10 g Trypton, 5 g Hefeextrakt, 10 g NaCl in 1 l) gezüchtet (Mandel, M. und Higa, A., 1970, J.Mol.Biol. 53, 159 - 162). Um für die Transformation geeignete Zellen ("kompetente Zellen") zu erhalten, wurden die Bakterien pelletiert und in 25 ml TR-Puffer (150 mM KCl, 50 mM $CaCl_2$, 1 mM Tris/HCl, pH 7, 3 mM $MgCl_2$) aufgenommen, 30 min auf Eis gestellt, abermals zentrifugiert, erneut in 2 ml TR-Puffer resuspendiert und 1 Stunde auf Eis gestellt.
Zu 200 µl der Zellsuspension wurden 100 µl der Mischung, die pBR322 mit dem eingefügten HRV2-RNA-cDNA-Hybrid enthielt, und 5 µl 1 M $CaCl_2$ zugegeben, 1 Stunde bei 0°C und danach 90 sek bei 42°C inkubiert. Dann wurden 2 ml LB-Medium zugesetzt und 1 Stunde bei 37°C inkubiert. Die Zellsuspension wurde auf LB-Agar-Platten (1,5 % Agar in LB-Medium), die 10 µg/ml Tetracyclin (Sigma) enthielten, aufgebracht und über Nacht inkubiert. Tetracyclin-resistente Klone wurden anschließend auf Ampicillin-Agar-Platten (100 µg Ampicillin/ml; Sigma) auf Ampicillin-Sensitivität geprüft.

---

* (DSM 1607)

Charakterisierung und Isolierung der rekombinanten DNA-Moleküle

Klone von Tetracyclin-resistenten, Ampicillin-sensitiven Bakterien wurden in 6 ml LB-Medium (10 µg Tetracyclin/ml) über Nacht hochgezüchtet, Plasmid DNA nach der "Mini-Plasmid-Präparationstechnik" isoliert (Birnboim, H.C. und Doly, J., 1979, Nucleic Acids Res. 7, 1195 - 1204) und die Größe der rekombinanten DNA durch Verdau mit dem Restriktionsenzym PstI ermittelt. Die Plasmid-DNA wurde in 25 µl RE-Puffer (6 mM $MgCl_2$, 10 mM Tris/HCl, pH 7,5, 6 mM Mercaptoethanol) mit 50 mM NaCl in Gegenwart von 2 U des Restriktionsenzyms PstI(Bethesda Research Laboratories) und 5 µg Ribonuklease A 2 Stunden bei 37°C inkubiert. Anschließend wurden die Proben auf einem 1,4%igen Agarose-Gel elektrophoretisch aufgetrennt. Durch Anfärben mit Ethidiumbromid und Vergleich mit Lambda-Hind III-Marker DNA konnten die Größen der Inserte bestimmt werden. Diese lagen zwischen 300 und 2000 Basenpaaren.

Um größere Mengen der DNA-Inserte zu isolieren, wurden Plasmide wie oben beschrieben aus 200 ml Kulturen von Tetracyclin-resistenten, Ampicillin-sensitiven Bakterienklonen gewonnen und mit PstI verdaut. Die rekombinanten-DNA-Fragmente wurden wie oben beschrieben über ein präparatives Agarose-Gel aufgetrennt, die Banden herausgeschnitten, die DNA in 0,05 x TBE-Puffer (1 x TBE-Puffer = 100 mM Tris/Borat, pH 8,3, 2 mM EDTA) elektroeluiert und mit Ethanol ausgefällt.

Subklonierung in Escherichia coli Stamm JM 101-Zellen mit Hilfe des pUC9-Vektors

Das Plasmid pUC9 (Vieisa, J. and Messing, J.G., 1982, Gene 19, 259 - 268) enthält ein Gen für die Ampicillinresistenz, eine Region für den Start der Replikation, die aus dem Plasmid pBR322 stammen, und einen Teil des Lac-Z-Gens von E.coli. Ein kleiner DNA-Abschnitt, der eine Reihe von Restriktionsstellen enthält, befindet sich in dieser Lac-Z-Region, so daß die Klonierung von DNA in eine dieser Schnittstellen die Lac-Z-Gen-Region unterbricht. Kolonien, die DNA-Inserte enthalten, erscheinen daher auf X-Gal (X-Gal = 5-Bromo-4 chloro-3-indolyl-ß-D-galactosid, Bethesda Research Laboratories)-Indikatorplatten weiß, diejenigen ohne Inserte erscheinenblau (Rüther, U., 1980, Mol. Gen. Genet. 178, 475 - 478). Um DNA-Inserte in pUC9 zu subklonieren, wurden rekombinante pBR322-Klone (ca. 7 µg) mit PstI verdaut und nach Auftrennung am Agarose-Gel (1,2 % - 1,4 %) die DNA-Inserte von der Vektor-DNA abgetrennt. Die DNA wurde aus dem Gel wie oben beschrieben durch Elektroelution und Ethanolfällung wiedergewonnen. Das isolierte DNA-Insert wurde in 20 µl RE-Puffer mit 0,4 µg pUC9-Vektor (mit PstI geschnitten und mit bakterieller alkalischer Phosphatase vorbehandelt), in Gegenwart von 1 mM ATP und 3 U $T_4$-Ligase (Bethesda Research Laboratories) 1 Stunde bei 15°C inkubiert und bei 4°C aufbewahrt (Vieisa, J. und Messing, J.G., 1982, loc.cit). Gleichzeitig wurden E.coli Stamm JM101-Zellen (New England Biolabs), die für die Transformation kompetent waren, in der oben beschriebenen Weise hergestellt. 200 µl der kompetenten Zellsuspension wurden mit 20 µl Gemisch der pUC9-Ligase-Reaktion vermischt und 1 Stunde bei 0°C inkubiert.

Nach einem Hitzeschock (90 sek., 42°C) wurden die Zellen mit 10 µl 200 mM Isopropylthiogalactosid (Sigma), 50 µl einer Lösung von 20 mg X-Gal in 1 ml Dimethylformamid und 1 ml LB-Medium versetzt und 1 Stunde bei 37°C inkubiert. Je 200 µl dieser Zellsuspension wurden anschließend auf je eine Ampicillin-LB-Agarplatte (100 µg/ml) transferiert und über Nacht bei 37°C im Brutschrank inkubiert. Positive Transformanten wurden als weiße Kolonien identifiziert, die mit Hilfe der "Mini-Plasmid-Präparationstechnik" auf DNA-Inserte hin untersucht wurden.

Präparation von pUC9-Plasmiden mit Inserten von rekombinanter DNA

Die gewonnenen Subklone von E. coli JM 101, die mit pUC9 transformiert waren und DNA-Inserte enthielten, wurden in 200 ml LB-Medium (mit 100 µg Ampicillin/ml) hochgezüchtet und die Plasmid-DNA isoliert (Birnboim, H.C. und Doly, J., 1979 loc. cit.) Die Plasmid-DNA wurde anschließend in 100 µl TE-Puffer gelöst und die Lösung über eine Sephacryl-1000-Säule (1 x 20 cm) mit TE-Puffer chromatographiert. Die Fraktionen, die das gereinigte Plasmid enthielten, wurden an Hand der Extinktion lokalisiert, vereinigt und lyophilisiert. Das Plasmid wurde in 500 µl TE-Puffer aufgenommen, 5 min bei 65°C inkubiert und nochmals mit Phenol/Chloroform extrahiert und mit Ethanol gefällt.

Gewinnung von Primer-Fragmenten aus den Plasmiden pHRV2-773 und pHRV2-87

Klone 773 und 87, die entsprechende DNA-Inserte enthielten, wurden in pUC9 subkloniert, in 200 ml LB-Medium (mit 100 µg Ampicillin/ml) hochgezüchtet und die Plasmide wie oben beschrieben isoliert. Das Primer-Fragment (59 Nukleotide) aus Klon 773 wurde durch Verdau mit den Restriktionsenzymen AhaIII und EcoRI erhalten (das Primer-Fragment ist in Fig. 3 als Fragment A bezeichnet).

Zu diesem Zweck wurden 200 µg gereinigtes Plasmid aus Klon 773 in 200 µl RE-Puffer in Gegenwart von 50 mM NaCl mit 30 U EcoRI (Bethesda Research Laboratories) 15 Stunden bei 37°C inkubiert. Nach Beendigung der Reaktion wurde mit Ethanol gefällt und das geschnittene Plasmid in 100 µl RE-Puffer in Gegenwart von 50 mM NaCl mit 40 U AhaIII (New England Biolabs) 15 Stunden bei 37°C inkubiert. Der Verlauf des Verdaues mit Restriktionsenzymen wurde durch Elektrophorese auf 1,4 %igen Agarose-Gelen überprüft. Das EcoRI/AhaIII-Fragment wurde in 100 µl OG-Lösung (1 % Ficoll, 1 mM EDTA, 0,01 % Orange G) aufgenommen und die DNA auf einem präparativen 15 %igem Polyacrylamid-Gel (Acrylamid/ Bisacrylamid = 19 : 1, Geldicke 1,2 mm) in 1 x TBE-Puffer aufgetrennt. Die Bande, die dem 59 Basen-EcoRI/AhaIII-Bruchstück entsprach, wurde nach Ethidiumbromid-Anfärbung herausgeschnitten, das Fragment in 0,05 x TBE -Puffer elektroeluiert und die DNA mit Ethanol präzipitiert. Das DNA-Fragment wurde anschließend in 50 µl 100 mM Tris/HCl, pH 8, mit 200 U bakterieller alkalischer Phosphatase (Bethesda Research Laboratories) 1 Stunde bei 65°C inkubiert. Nach der Reaktion wurden 2,5 µl 0,5 M EDTA, pH 8, zugesetzt, die wässrige Phase 2 x mit Phenol/Chloroform extrahiert und die DNA durch Ethanolfällung präzipitiert. Die DNA wurde in Wasser gelöst und in 50 µl K-Puffer (10 mM $MgCl_2$, 50 mM Tris/HCl, pH 8, 5 mM Dithioerythrit) mit 20 µCi γ-[$^{32}$P]-ATP (spez. Aktivität 5000 Ci/mmol; Amersham International) und 5 U Polynukleotidkinase (Pharmacia-PL Laboratories) bei 37°C 30 min inkubiert. Das mit $^{32}$P-markierte Fragment wurde anschließend ausgefällt und in 30 µl 30 %igem Dimethylsulfoxid, das 1 mM EDTA und 0,01 % Xylencyanol-Bromphenolblau enthielt, aufgenommen. Diese Lösung wurde 2 min bei 90°C inkubiert, in Eis abgekühlt, auf ein 15 %iges Polyacrylamid-Gel (Acrylamid/Bisacrylamid = 59 : 1;Geldicke 1,2 mm) in TBE-Puffer aufgetragen und die beiden DNA-Stränge elektrophoretisch voneinander getrennt (15 Stunden bei 200 Volt). An Hand des Autoradiogramms wurden die beiden Stränge lokalisiert, herausgeschnitten und elektroeluiert. Der mit HRV2-RNA-hybridisierende Strang wurde durch ein "dot-blot"-Experiment ermittelt. Dazu wurden zwei 2 x 2 cm große Nitrozellulosestreifen (Schleicher & Schüll, BA 85, 0,45 μm) mit $H_2O$ befeuchtet, einmal mit 20 x SSC (1 x SSC = 150 mM NaCl, 15 mM Natriumcitrat, pH 7,4) gewaschen und an der Luft getrocknet. Auf jedem Streifen wurde punktförmig ca. 1 μg HRV2-RNA aufgetragen, getrocknet und bei 80°C 2 Stunden inkubiert. Anschließend wurden die Streifen mit 2 x SSC befeuchtet und in einer Plastikfolie gemeinsam in 1 ml H-Puffer (400 mM NaCl, 40 mM PIPES, pH 6,4, 1 mM EDTA, 80 % Formamid) mit 4 μg denaturierter Lachsspermien-DNA (Sigma; 2 min bei 100°C inkubiert und auf 0°C abgekühlt) 1 Stunde bei 42°C inkubiert. Danach wurden die beiden Nitrozellulosestreifen mit je 0,5 ml H-Puffer, 4 μg denaturierter Lachsspermien-DNA und je einem Aliquot der isolierten Stränge (20 000 cpm) getrennt in Plastikfolien eingeschweißt und über Nacht bei 42°C hybridisiert. Nach dieser Inkubation wurden die Filter 2 mal mit 2 x SSC 10 min lang bei 50°C und 2 mal mit 0,1 x SSC, 0,1 % Natriumdodecylsulfat 30 min bei 50°C gewaschen und die Radioaktivität bestimmt.

Das Primer-Fragment aus pHRV2-87 wurde in gleicher Weise isoliert. Das Fragment liegt zwischen zwei RsaI-Restriktionsstellen (68 Nukleotide) und wurde durch Verdau mit diesem Restriktionsenzym erhalten. Das RsaI-Fragment ist in Fig. 3 als Fragment B bezeichnet. Strang-Trennung und Hybridisierung wurden wie oben beschrieben durchgeführt.

## Reverse Transkription von HRV2-RNA mit Hilfe von Restriktionsfragmenten als Primer

5 pmol der zu HRV2-RNA komplementären Einzelstrang-DNA aus den Restriktionsfragmenten, die, wie oben beschrieben, aus den Klonen 773 (Fragment A) und 87 (Fragment B) isoliert worden waren, wurden jeweils gemeinsam mit 0,25 pmol der HRV2-RNA aus einer wässrigen Lösung mit Ethanol präzipitiert. Der Niederschlag wurde in 20 µl H-Puffer aufgenommen, in eine Kapillare eingeschweißt, 10 min bei 72°C inkubiert, auf 50°C transferiert, langsam auf 35°C abgekühlt und anschließend auf Eis gestellt. Die Lösung wurde dann in 100 µl RT-Puffer in Gegenwart von 140 U reverser Transkriptase (Anglian Biotechnology Co, Cambridge), 8 U Ribonuklease-Inhibitor (RNasin, Bethesda Research Laboratories), je 0,2 mM dATP, dCTP, dGTP und dTTP, 30 µCi [$\alpha$-$^{32}$P]dCTP und 5 mM Dithioerythrit 2 Stunden bei 42°C inkubiert. Das entstandene reverse Transkript wurde, wie oben beschrieben, aufgearbeitet.

## Nachweis von HRV2-Sequenzen in rekombinanter DNA und Restriktionskartierung.

Plasmid-DNA der Rekombinanten wurden von 3 ml Kulturen isoliert (Birnboim, H.C. und Doly, J., 1979, loc. cit). Die DNA wurde dann mit dem Restriktionsenzym PstI inkubiert und die Proben durch Elektrophorese auf 1,4 %igem Agarose-Gel analysiert. Die Gele wurden mit Ethidiumbromid angefärbt. Anschließend wurde die DNA vom Gel auf Nitrozellulosefilter transferiert (Southern, E.M., 1975, J.Mol, Biol. 98, 503- 517) und durch eine 2-stündige Inkubation bei 80°C auf der Nitrozellulose fixiert. Die Filter wurden in 50 % Formamid, 1 x Denhardts-Lösung (Denhardt, D.T., 1966, Biochem.Biophys. Res. Comm., 23,641 - 646), 900 mM NaCl, 50 mM Natriumphosphat, pH 7,4, 5 mM EDTA mit 80 µg/ml denaturierter Lachsspermien-DNA 2 Stunden bei 42°C in einer Plastikfolie präinkubiert. Radioaktive HRV2-cDNA wurde wie oben beschrieben hergestellt, nur enthielt der Reaktionsansatz 50 µCi $[\alpha-^{32}P]$dCTP. Das cDNA-HRV2-RNA-Hybrid wurde bei 100°C 90 sek denaturiert. Zur Hybridisierung wurden die Filter mit radioaktiver HRV2-cDNA wie oben beschrieben bei 42°C 18 Stunden inkubiert, anschließend 2 mal in 2 x SSC und 2 mal in 0,1 % Natriumdodecylsulfat 30 min bei 50°C gewaschen, an der Luft getrocknet und bei -70°C exponiert (Kodak XAR-5, mit Verstärkerfolie 18 - 40 Stunden). Das Auftreten einer radioaktiven Bande zeigte das Vorhandensein von rekombinanter DNA an, die zur HRV2-RNA komplementär war.

Zur Kartierung wurden die DNA-Inserte unter Verwendung von Restriktionsenzymen (New England Biolabs und Bethesda Research Laboratories) verdaut, wobei die von den Herstellern angegebenen Inkubationsbedingungen verwendet wurden. Das Ergebnis der Restriktionskartierung ist in Fig. 3 gezeigt. Die Plasmide pHRV2-1 und pHRV2-24 enthielten - unmittelbar anschließend an das Oligo-C, das auf die Kettenverlängerung durch die terminale Transferase-Reaktion zurückzuführen ist - eine längere Sequenz von A-Resten, die einen Teil des 3'-terminalen Poly-A der HRV2-RNA darstellen. Die weiteren Plasmide wurden relativ zu pHRV2-1 und pHRV2-24 unter Zuhilfenahme charakteristischer Spaltstellen einzelner Restriktionsenzyme angeordnet. DNA-Inserte, die kleiner als 500 Basenpaare waren, wurden nicht durch Restriktionsenzymkartierung eingeordnet, sondern wurden sofort in pUC9 subkloniert und sequenziert (siehe Beispiel 3).

Die Identifizierung der restlichen Klone erhält man durch Koloniehybridisierung unter Verwendung bereits kartierter DNA-Inserte als "Nick-Translations"-Proben nach der Methode von Grunstein und Hogness (Grunstein, M. und Hogness, D.S., 1975, Proc. Natl. Acad. Sci USA 72, 3961-3965). $^{32}P$-markierte DNA-Proben erhält man mit Hilfe eines "Nick-Translation-Kit" der Firma Amersham International (England; Amersham Kit No. 5000) nach Vorschrift des Herstellers mit $[\alpha-^{32}P]$dCTP (3000 Ci/mmol). Die markierte DNA wurde über eine Biogel-P-30-Säule in einer Pasteurpipette in TE-Puffer aufgetrennt. Fraktionen, die dem Exklusionsvolumen entsprachen, wurden vereinigt, 2 min bei 100°C erhitzt und schnell in Eiswasser gestellt. Die Hybridisierung wurde wie oben beschrieben durchgeführt. Von Kolonien, die ein positives Hybridisierungsignal zeigten, wurden 50 ml Kulturen (über Nacht in LB-Medium mit 10 µg Tetracyclin/ml) hergestellt und aus der Plasmid-DNA die DNA-Inserte mit PstI isoliert. Diese wurden anschließend durch Verdau mit verschiedenen Restriktionsenzymen und durch Sequenzierung charakterisiert. In dieser Weise wurden Klone erhalten, die das Genom von HRV2 repräsentieren.

Beispiel 3

DNA-Sequenzierung

Der größte Teil der cDNA-Klone von HRV2 wurde nach einer Modifikation der Methode von Maxam und Gilbert (Maxam, A. und Gilbert, W., 1980, Methods Enzymol. 65, 499-560) sequenziert. Einige der Sequenzen wurden auch mit Hilfe der M 13-Kettenabbruchmethode nach Sanger et al. (Sanger, F., Nicklen, S. und Coulsen, A.R., 1977, Proc. Natl. Acad. Sci USA 74, 5463 – 5467) ermittelt.

Zur Sequenzierung nach Maxam und Gilbert wurden die DNA-Inserte wie oben beschrieben in pUC-9 subkloniert und damit kompetente E.coli JM 101- Zellen transformiert. Positive Transformanten wurden als weiße Kolonien isoliert und im LB-Medium (mit 100 µg/ml Ampicillin) hochgezüchtet. 10 - 20 µg DNA wurden in 100 µl über Nacht unter Standardreaktionsbedingungen mit Restriktionsenzymen verdaut, die eine Spaltstelle im Plasmid z.B. in der Polylinker-Region von pUC9 besitzen (z.B. BamHI, EcoRI, AccI, HindIII). Das restringierte Fragment wurde anschließend dephosphoryliert. Zum Restriktionsverdau wurden 5 µl 2 M Tris/HCl, pH 8, und 100 U bakterieller alkalischer Phosphatase (Bethesda Research Laboratories) zugesetzt und 3 Stunden bei 65°C inkubiert. Nach Zugabe von EDTA auf 20 mM wurde zweimal mit Phenol/Chloroform extrahiert und die DNA durch Ethanol gefällt. Die DNA wurde anschließend in 50 µl 50 mM Tris/HCl, pH8, 10 mM $MgCl_2$, 5 mM Dithioerythrit, mit 25 µCi [$\gamma$-$^{32}$P]ATP (5000 Ci/mmol, Amersham International) und 4 U $T_4$-Polynukleotidkinase (Pharmacia-P.L. Biochemicals) 30 min bei 37°C inkubiert und die markierte DNA mit Ethanol präzipitiert. Unter Zuhilfenahme eines weiteren Restriktionsenzyms, das in der Polylinker-Region von pUC9 spaltet, wurde rekombinante DNA, die in einem Strang mit $^{32}$P markiert war, erhalten.

DNA-Sequenzierung nach einer Modifikation der Methode von Maxam und Gilbert

Die Sequenzierungsreaktionen wurden mit folgenden Modifikationen nach Maxam und Gilbert (Maxam, A. und Gilbert,W., 1980, Methods Enzymol. 65, 499 - 560) durchgeführt:

- Es wurde keine Träger-DNA zugesetzt.
- Die DNA-Lösung wurde in Aliquote aufgeteilt: Guanin (G)-spezifische Reaktion 7,5 µl, Guanin und Adenin (G/A)-spezifische Reaktion 10 µl, Cytosin und Thymin (C/T)-spezifische Reaktion 10 µl, Cytosin (C)-spezifische Reaktion 6 µl.

- Dem (G/A)-Reaktionsansatz wurden 25 µl 96 % Ameisensäure zugesetzt und die Mischung 4,25 min bei 19°C inkubiert. Um die Reaktion abzustoppen, wurden 200 µl Hydrazin-Stop-Lösung und 750 µl 96 % Ethanol zugesetzt. Die (G/A)-Reaktionen wurden dann gleich wie alle 3 anderen Reaktionen behandelt.

- Anstelle von Hydrazin wurde Hydraziniumhydroxid (Merck) bei den (C/T)- und C-Reaktionen verwendet. Die Reaktionszeiten betrugen 7,5 min.

- Die Piperidinreaktionen wurden 30 min bei 95°C inkubiert. Nach Lyophilisierung wurden die Fragmente in 3 - 20 µl Puffer (80 % entionisiertes Formamid, 1 x TBE, 0,05 % Bromphenolblau und 0,05 % Xylencyanol) 90 sec auf 95°C erhitzt und schnell auf 0°C gekühlt.

Zur Sequenzierung wurden 6 %ige Polyacrylamid-Gele (40 cm x 20 cm x 0,4 mm) mit 8 M Harnstoff und 1 x TBE verwendet, die vor dem Auftragen der Proben 1 Stunde bei 50 Watt einer Elektrophorese unterworfen wurden. Zwischen 1 µl und 3µl von jedem Reaktionsansatz wurden auf das Gel aufgetragen. Üblicherweise wurden zwei zeitlich verschobene Gelbeladungen durchgeführt.

Der erste Gellauf eines Reaktionsansatzes dauerte solange bis der Bromphenolblau-Marker, der zweite bis der Xylencyanol-Marker das Gelende erreichte. Die Gele wurden anschließend 20 min in 10 % Essigsäure und 10 % Methanol (ca. 2 Liter) fixiert, auf 3 MM-Filterpapier transferiert und bei 80°C auf einem Geltrockner getrocknet. Dann wurden die Gele ohne Verstärkerfolie bei -70°C unter Verwendung eines XAR-Omat Films (Kodak) exponiert (ca. 18 bis 36 Stunden).

M 13 Sequenzierung

Die rekombinante DNA wurde mit dem Restriktionsenzym Sau3AI geschnitten und in die BamHI Schnittstelle von M13 mp9 kloniert, wobei ein "Sequencing pack" (New England Biolabs, Kat. Nr. 409) verwendet wurde. Die Sequenzierung wurde nach der Kettenabbruchmethode durchgeführt (Sanger, F., Nicklen, S. und Coulson, A.S., 1977, Proc. Natl. Aca. Sci USA 74, 5463 - 5467).

Analyse der Sequenzierungsdaten

Die Sequenzierungsergebnisse wurden mit Hilfe eines Cyber 170 Computers analysiert. Dabei wurden die Programme von Staden (Staden, R., 1980, Nucleic Acids Res. 8, 3673-3694) und die modifizierten Programmformen von Isono (Isono, K., 1982, Nucleic Acids Res. 10, 85 - 89 ) verwendet.

## Beispiel 4

## Proteolytische Spaltstellen in der P1-Hüllenprotein-Region des Polyproteins

Die viralen Proteine werden durch proteolytische Spaltung aus dem Polyprotein erhalten. Um die Spaltstellen zu identifizieren, wurden die viralen Hüllenproteine isoliert und die N-terminale Aminosäuresequenz bestimmt. Zu diesem Zweck wurden die Proteine von 2 mg HRV2 elektrophoretisch auf einem 12,5 %igen Polyacrylamid-Gel aufgetrennt (Laemmli, U.K., 1970, loc.cit.). Die Gele wurden mit einer gesättigten Lösung von Coomassie-Brillant-Blau in 50 mM Tris/HCl, pH 7,4, gefärbt und die Proteinbanden herausgeschnitten. Die einzelnen Proteine wurden in einer ISCO-Elutionsapparatur bei 50 V 16 Stunden elektroeluiert und mit Trichloressigsäure präzipitiert. Die N-terminalen Aminosäuren wurden mit Hilfe eines AB-470A Protein-Sequenators (Applied Biosystems, Inc., Foster City, CA, USA) bestimmt. Zur Sequenzierung wurden je 2 nmol VP1 und VP2 und 1 nmol VP3 verwendet. Die derivatisierten Aminosäuren wurden mittels HPLC analysiert. Die N-terminalen Sequenzen der einzelnen Proteine sind in Fig. 6 angegeben.

## Beispiel 5

## Identifikation des viralen Kapsidproteins VP1 als stärkstes Antigen von HRV2 in Kaninchen

Kaninchen wurden mit 25 µg HRV2 in 500 µl komplettem Freund'schen Adjuvans subcutan injiziert. 21 bzw. 35 Tage danach wurden weitere Immunisierungen mit jeweils 25 µg HRV2 in 1,5 ml inkomplettem Freund'schem Adjuvans durchgeführt. 50 Tage nach der ersten Immunisierung wurde Serum durch Plasmaphorese entnommen und bei -20°C aufgewahrt. Zum Nachweis der Antikörperbildung wurden

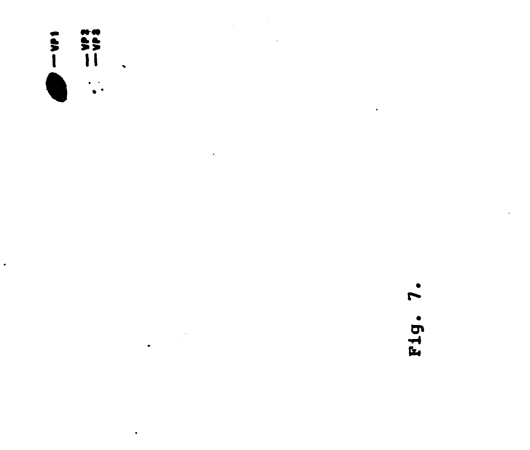

2 µg Virus auf einem 15 %igem Polyacrylamid-Gel (Laemmli, U.K., 1970, loc.cit.) aufgetragen und die Proteine elektrophoretisch getrennt. Die Proteine wurden nach der "Western Blot"-Methode durch Elektrotransfer vom Gel auf eine Nitrozellulosefiltermembran (Schleicher & Schüll, BA85, 0,45 µm) übertragen (Burnette, W.N., 1981, Analyt. Biochem. 112, 195 - 203). Das Filter mit den daran gebundenen Proteinen wurde 16 Stunden in 20 ml PBS (137 mM NaCl, 2,7 mM KCl, 6,6 mM $Na_2HPO_4$, 1,5 mM $KH_2PO_4$) mit 3 % Rinderserumalbumin (BSA) und 3 % Tween 20 gebadet. Nach 2 Stunden Inkubation mit Antiserum (1 : 200 verdünnt, in PBS/1 % BSA/1 % Tween 20) wurde das Filter dreimal 20 min in je 20 ml PBS mit 1 % BSA und 1 % Tween 20 (PBSBT) gewaschen und anschließend 2 Stunden in 20 ml PBSBT mit 10 µCi $^{125}$I-markiertem Protein aus Staphylococcus aureus (ca. 1 mCi/mg) inkubiert, dreimal 20 min mit PBSBT sowie 3 x 5 min in PBS gewaschen, zweimal kurz mit $H_2O$ gespült und über Nacht zwischen mehreren Lagen Filterpapier getrocknet. Die an das Filter gebundene Radioaktivität wurde durch Autoradiographie auf Kodak XAR5-Röntgenfilm (20 Stunden/ -70°C) ermittelt. Wie aus Fig. 7 hervorgeht, liegen im Antiserum vor allem Antikörper gegen VP1 vor.

Beispiel 6

Vergleich einer Teilsequenz aus den Genen für die Polypeptide P3A und P3B (VPg) des HRV2 mit einer entsprechenden Teilsequenz aus dem Genom des HRV89.

Zur weiteren Unterstützung eines Teiles der auf Grund der vorher beschriebenen Beispiele ermittelten Sequenz wurde ein Vergleich mit einem Genfragment aus dem Rhinovirus HRV89 durchgeführt. Der HRV89 wurde von der American Type Culture Collection (ATCC VR-1199) erhalten und in analoger Weise wie HRV2 gezüchtet. HRV89 wurde im Neutralisationstest durch ein spezifisches Antiserum (ATCC VR-1199 AS/GP) neutralisiert, während ein Antiserum gegen HRV2 (ATCC VR-1112 AS/GP) als Kontrollserum keinen Effekt zeigte. Die Isolierung der viralen RNA, die Charakterisierung, Klonierung, Isolierung der Klone und die Sequenzierung wurde in gleicher Weise wie für HRV2 beschrieben, durchgeführt. Fig.8 zeigt einen Sequenzvergleich mit einer Teilsequenz aus dem HRV89 Klon 34/1, die offensichtlich aus dem Bereich der Gene für P3A und P3B (VPg) stammt.

## PATENTANSPRÜCHE

1. DNA-Molekül, codierend für mindestens ein virales Protein des Rhinovirusstammes HRV2.

2. DNA-Molekül nach Anspruch 1, dadurch gekennzeichnet, daß es der gesamten viralen RNA oder Teilen der viralen RNA des Rhinovirusstammes HRV2 entspricht.

3. DNA-Molekül nach Anspruch 1, dadurch gekennzeichnet, daß es für ein virales Protein, zusammengesetzt aus den viralen Proteinen VP1, VP2, VP3, VP4, P2A, P2B, P2C, P3A und P3C oder daß es für mindestens zwei der genannten, in beliebiger Kombination miteinander verknüpften, viralen Proteine, codiert.

4. DNA-Molekül nach Anspruch 1, die Sequenz gemäß Fig.4, oder Teilen davon enthaltend.

5. DNA-Molekül nach Anspruch 1, dadurch gekennzeichnet, daß es für das virale Protein VP1, VP2, VP3, VP4, P2A, P2B, P2C, P3A oder P3C codiert.

6. DNA-Molekül codierend für einen Teil des viralen Proteins nach Anspruch 1, dadurch gekennzeichnet, daß die biologische Aktivität des Proteins zumindest einem der viralen Proteine entspricht.

7. DNA-Molekül nach Anspruch 1, dadurch gekennzeichnet, daß die codierende Sequenz unter stringenten Bedingungen, die es erlauben, eine Homologie zu erkennen, die größer als 85% ist, mit einem DNA-Molekül nach einem der Ansprüche 3 bis 6 oder mit degenerierten Variationen dieser

Moleküle hybridisiert.

8. DNA-Molekül nach einem der vorhergehenden Ansprüche, dadurch gekennzeichnet, daß es in einem geeigneten Expressionsvehikel, beispielsweise in einem Plasmid, replizierbar in Mikroorganismen, vorzugsweise in Prokaryoten, Eukaryoten oder in Säugetierzellen, eingebaut ist.

9. Transformierter Wirtsorganismus, vorzugsweise ein Prokaryot, Eukaryot oder eine Säugetierzellinie insbesondere E.coli, ein DNA-Molekül nach einem der Ansprüche 1 bis 7 enthaltend, wobei die genetische Information vorzugsweise in einem Vehikel enthalten ist, das in dem Wirtsorganismus replizierbar ist, insbesondere ein DNA-Molekül nach Anspruch 8.

10. Polypeptid, dadurch gekennzeichnet, daß es die biologische Aktivität mindestens eines der viralen Proteine des Rhinovirusstammes HRV2 aufweist und/oder daß es von einem DNA-Molekül nach einem der Ansprüche 1 bis 8 codiert wird und/oder daß es in Teilen oder insgesamt mindestens einem der viralen Proteine des Rhinovirusstammes HRV2 entspricht oder daß mindestens zwei der viralen Proteine oder Teile der viralen Proteine in beliebiger Kombination und Reihenfolge miteinander verknüpft sind.

11. Polypeptid nach Anspruch 10, dadurch geknnzeichnet, daß es die Aminosäuresequenz gemäß Fig. 4 oder Teile davon enthält.

12. Polypeptid nach Anspruch 27, dadurch gekennzeichnet, daß es die Aminosäuresequenz für VP1, VP2, VP3, VP4, P2A, P2B, P2C, P3A oder P3C enthält.

13. Polypeptid, abgeleitet aus der Aminosäuresequenz nach Anspruch 11 oder aus Teilen hiervon, dadurch gekennzeichnet, daß es sich an die zellulären Rezeptoren für die Rhinoviren Stamm HRV2 bindet und/oder diese blockiert.

14. Verfahren zur Herstellung eines DNA-Moleküls nach einem der Ansprüche 1 bis 8, dadurch gekennzeichnet, daß

a. die virale RNA des Rhinovirusstammes HRV2 isoliert,

b. eine zur viralen RNA komplementäre DNA hergestellt,

c. das virale cDNA/RNA-Hybrid in einen geeigneten replizierbaren Vektor eingebaut wird und

d. ein geeigneter Wirtsorganismus mit dem unter c hergestellten Vektor transformiert wird.

15. Verfahren zur Herstellung des Polypeptides nach einem der Ansprüche 10 bis 13, dadurch gekennzeichnet, daß ein geeigneter Wirtsorganismus vorzugsweise ein Wirtsorganismus gemäß Anspruch 9 mit genetischen Informationen codierend für ein virales Polypeptid nach einem der Ansprüche 10 bis 13, vorzugsweise mit genetischen Informationen, die in einem DNA-Molekül nach einem der Ansprüche 1 bis 8 enthalten sind, transformiert wird;

daß diese Information exprimiert wird, um ein virales Polypeptid nach einem der Ansprüche 10 bis 13 in dem Wirtsorganismus zu produzieren und

daß das virale Polypeptid nach einem der Ansprüche 10 bis 13 isoliert wird.

16. Verwendung eines der Polypeptide nach den Ansprüchen 10 bis 13 zur therapeutischen Behandlung.

17. Arzneimittel geeignet für die Verwendung nach Anspruch 16, dadurch gekennzeichnet, daß es neben pharmazeutisch inerten Hilfs- oder Trägerstoffen eine wirksame Menge mindestens eines der Polypeptide nach den Ansprüchen 10 bis 13 enthält.

1/19

0192175

Fig. 1.

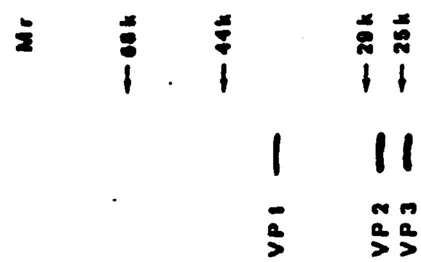

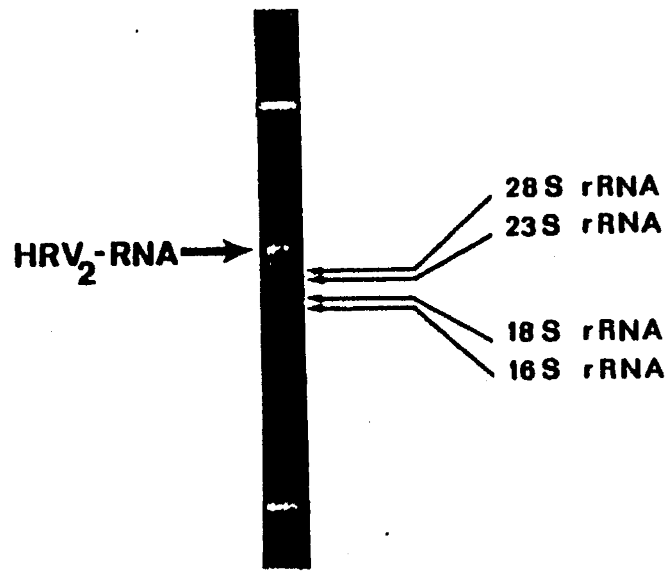


Fig. 2.

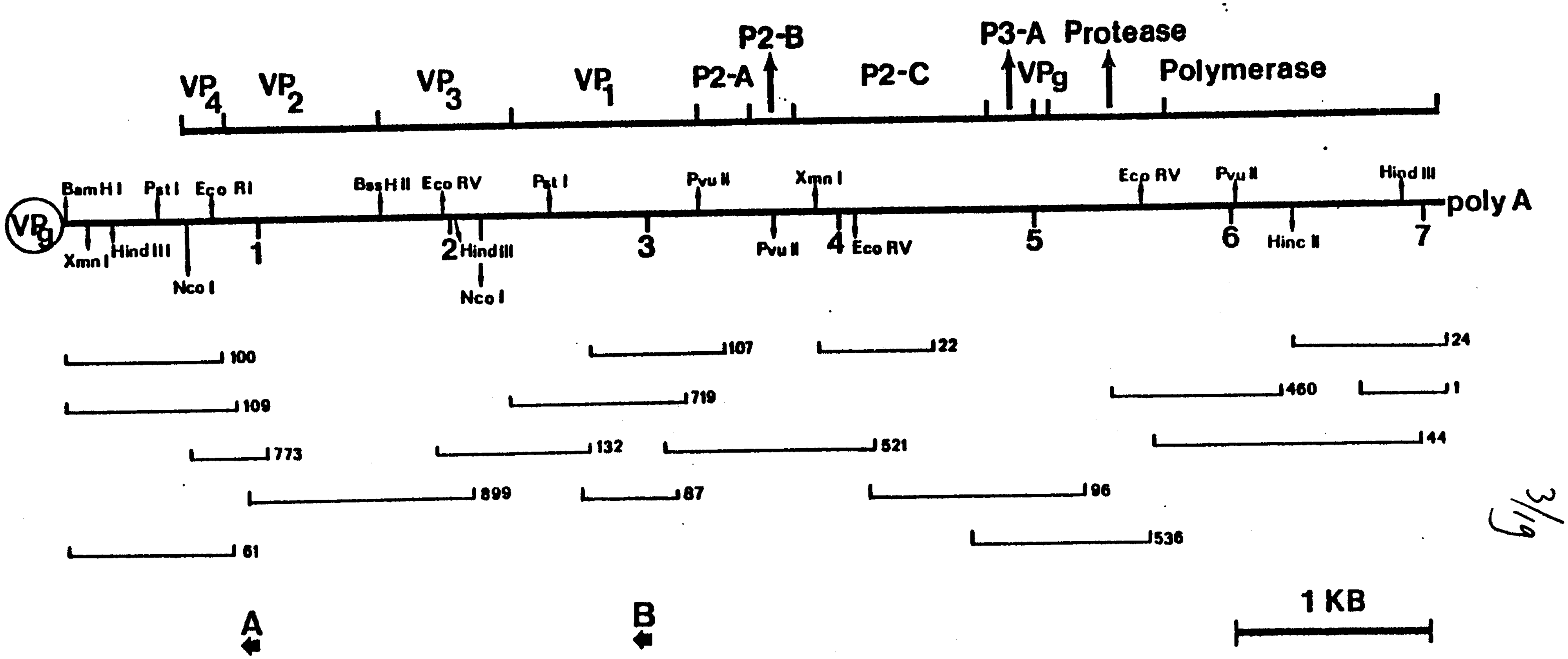
P2-B
P3-A Protease
VP4 VP2 VP3 VP1 P2-A P2-C VPg Polymerase
BamH I Pst I Eco RI BssH II Eco RV Pst I Pvu II Xmn I Eco RV Pvu II Hind III
VPg
poly A
Xmn I Hind III Nco I 1 2 Hind III Nco I 3 Pvu II 4 Eco RV 5 6 Hinc II 7
100 107 22 24
109 719 460 1
773 132 521 44
899 87 96
61 536
A
B
1 KB

Fig. 3.

(Fig. 4a)

```
TTAAAACTGGATCCAGGTTGTTCCCACCTGGATTTCCCACAGGGAGTGGTACTCTGTTATTACGGTAACTTTGTACGCCAGTTTTATCTCCCTTCCCCCA
         10        20        30        40        50        60        70        80        90       100

TGTAACTTAGAAGTTTTTCACAAAGACCAATAGCCGGTAATCAGCCAGATTACTGAAGGTCAAGCACTTCTGTTTCCCCGGTCAATGTTGATATGCTCCA
        110       120       130       140       150       160       170       180       190       200

ACAGGGCAAAAACAACTGCGATCGTTAACCGCAAAGCGCCTACGCAAAGCTTAGTAGCATCTTTGAAATCGTTTGGCTGGTCGATCCGCCATTTCCCCTG
        210       220       230       240       250       260       270       280       290       300

GTAGACCTGGCAGATGAGGCTAGAAATACCCCACTGGCGACAGTGTTCTAGCCTGCGTGGCTGCCTGCACACCCTATGGGTGTGAAGCCAAACAATGGAC
        310       320       330       340       350       360       370       380       390       400

AAGGTGTGAAGAGCCCCGTGTGCTCGCTTTGAGTCCTCCGGCCCCTGAATGTGGCTAACCTTAACCCTGCAGCTAGAGCACGTAACCCAATGTGTATCTA
        410       420       430       440       450       460       470       480       490       500

GTCGTAATGAGCAATTGCGGGATGGGACCAACTACTTTGGGTGTCCGTGTTTCACTTTTTCCTTTATATTTGCTTATGGTGACAATATATACAATATATA
        510       520       530       540       550       560       570       580       590       600
            VP4
           M  G  A  Q  V  S  R  Q  N  V  G  T  H  S  T  Q  N  S  V  S  N  G  S  S  L  N  Y  F  N  I
TATTGGCACCATGGGTGCACAGGTTTCAAGACAAAATGTTGGAACTCACTCCACGCAAAACTCTGTATCAAATGGGTCTAGTTTAAATTATTTTAACATC
        610       620       630       640       650       660       670       680       690       700

 N  Y  F  K  D  A  A  S  N  G  A  S  K  L  E  F  T  Q  D  P  S  K  F  T  D  P  V  K  D  V  L  E  K
AATTATTTCAAAGATGCTGCTTCAAATGGTGCATCAAAACTGGAATTCACACAAGATCCTAGTAAATTTACTGACCCAGTTAAGGATGTTTTGGAAAAGG
        710       720       730       740       750       760       770       780       790       800
                ↓  VP2
G  I  P  T  L  Q  S  P  T  V  E  A  C  G  Y  S  D  R  I  I  Q  I  T  R  G  D  S  T  I  T  S  Q  D  V
GAATACCAACACTACAGTCCCCCACAGTGGAGGCTTGTGGATACTCTGATAGGATTATACAGATTACCAGAGGAGATTCAACCATAACCTCACAAGATGT
        810       820       830       840       850       860       870       880       890       900

  A  N  A  I  V  A  Y  G  V  W  P  H  Y  L  S  S  K  D  A  S  A  I  D  K  P  S  Q  P  D  T  S  S  N
GGCTAATGCTATCGTTGCGTATGGTGTTTGGCCACATTATCTATCCTCCAAGGATGCCTCTGCAATTGATAAACCCTCTCAACCAGATACATCTTCTAAT
        910       920       930       940       950       960       970       980       990      1000

 R  F  Y  T  L  R  S  V  T  W  S  S  S  S  K  G  W  W  W  K  L  P  D  A  L  K  D  M  G  I  F  G  E
AGATTTTATACTCTAAGGAGTGTGACCTGGAGCAGTTCCTCAAAGGGTTGGTGGTGGAAACTACCTGATGCACTCAAGGACATGGGTATTTTTGGTGAAA
       1010      1020      1030      1040      1050      1060      1070      1080      1090      1100

N  M  F  Y  H  Y  L  G  R  S  G  Y  T  I  H  V  Q  C  N  A  S  K  F  H  Q  G  T  L  I  V  A  L  I  P
ACATGTTTTATCATTACCTGGGTAGGAGTGGATACACAATACATGTGCAGTGTAATGCTAGTAAATTTCACCAGGGTACACTAATTGTTGCTCTGATACC
       1110      1120      1130      1140      1150      1160      1170      1180      1190      1200

  E  H  Q  I  A  S  A  L  H  G  N  V  N  V  G  Y  N  Y  T  H  P  G  E  T  G  R  E  V  K  A  E  T  R
TGAGCATCAGATTGCAAGTGCCTTACATGGCAATGTGAATGTTGGTTACAACTACACACACCCAGGTGAAACAGGCAGGGAAGTTAAAGCTGAGACGAGA
       1210      1220      1230      1240      1250      1260      1270      1280      1290      1300

 L  N  P  D  L  Q  P  T  E  E  Y  W  L  N  F  D  G  T  L  L  G  N  I  T  I  F  P  H  Q  F  I  N  L
TTGAATCCTGATCTACAACCTACTGAAGAGTATTGGCTAAACTTTGATGGGACACTCCTTGGAAATATTACCATATTCCCTCATCAATTTATCAACTTGA
       1310      1320      1330      1340      1350      1360      1370      1380      1390      1400
```

(Fig. 4b)

5/19

0192175

R S N N S A T I I A P Y V N A V P M D S M R S H N N W S L V I I P I

GGAGTAATAATTCTGCCACAATAATTGCCCCTTATGTCAATGCAGTTCCTATGGATTCAATGCGGAGCCACAATAATTGGAGTTTGGTAATAATACCAAT

1410 1420 1430 1440 1450 1460 1470 1480 1490 1500

C P L E T S S A I N T I P I T I S I S P M C A E F S G A R A K R Q

ATGTCCCCTTGAGACATCAAGTGCAATTAACACAATACCTATTACAATATCTATAAGCCCCATGTGTGCAGAGTTTTCCGGCGCGCGTGCCAAGCGTCAA

1510 1520 1530 1540 1550 1560 1570 1580 1590 1600

↓ VP3

G L P V F I T P G S G Q F L T T D D F Q S P C A L P W Y H P T K E

GGATTACCAGTTTTCATCACACCAGGTTCAGGACAGTTTTTGACAACAGATGATTTCCAATCCCCATGTGCACTTCCCTGGTATCACCCAACTAAGGAAA

1610 1620 1630 1640 1650 1660 1670 1680 1690 1700

I S I P G E V K N L V E I C Q V D S L V P I N N T D T Y I N S E N M

TTTCTATTCCAGGTGAGGTTAAAAATTTGGTTGAAATTTGTCAAGTAGACAGCCTAGTACCAATAAATAACACTGACACCTACATCAATAGTGAAAATAT

1710 1720 1730 1740 1750 1760 1770 1780 1790 1800

Y S V V L Q S S I N A P D K I F S I R T D V A S Q P L A T T L I G

GTATTCTGTTGTATTGCAATCATCAATTAATGCACCAGATAAGATCTTCTCTATTCGAACAGATGTTGCTTCCCAACCTTTAGCTACTACTTTGATTGGT

1810 1820 1830 1840 1850 1860 1870 1880 1890 1900

E I S S Y F T H W T G S L R F S F M F C G T A N T T V K L L L A Y

GAGATATCTAGCTATTTCACCCACTGGACAGGGAGTCTCCGTTTCAGCTTCATGTTTTGTGGTACTGCCAACACTACTGTTAAGCTTTTGTTGGCATACA

1910 1920 1930 1940 1950 1960 1970 1980 1990 2000

T P P G I A E P T T R K D A M L G T H V I W D V G L Q S T I S M V V

CACCACCTGGTATCGCAGAACCCACCACAAGAAAGGATGCAATGCTAGGCACTCATGTTATATGGGATGTGGGGTTGCAGTCTACAATATCAATGGTAGT

2010 2020 2030 2040 2050 2060 2070 2080 2090 2100

P W I S A S H Y R N T S P G R S T S G Y I T C W Y Q T R L V I P P

GCCATGGATTAGCGCTAGTCATTATAGAAACACATCACCAGGTAGATCTACATCTGGGTACATAACATGCTGGTATCAGACTAGATTAGTCATTCCACCT

2110 2120 2130 2140 2150 2160 2170 2180 2190 2200

Q T P P T A R L L C F V S G C K D F C L R M A R D T N L H L Q S G

CAGACCCCACCAACAGCTAGATTGTTATGTTTTGTATCTGGGTGCAAAGACTTTTGCTTGCGCATGGCACGAGATACTAACCTACACCTGCAAAGTGGTG

2210 2220 2230 2240 2250 2260 2270 2280 2290 2300

↓ VP1

A I A Q N P V E N Y I D E V L N E V L V V P N I N S S N P T T S N S

CAATAGCACAGAACCCTGTTGAGAATTATATAGATGAAGTTCTTAATGAAGTTTTAGTTGTCCCAAATATTAATAGTAGTAACCCCACAACATCAAATTC

2310 2320 2330 2340 2350 2360 2370 2380 2390 2400

A P A L D A A E T G H T S S V Q P E D V I E T R Y V Q T S Q T R D

TGCCCCAGCATTAGATGCTGCAGAAACAGGGCACACTAGTAGTGTTCAACCAGAGGATGTCATTGAAACTAGGTATGTGCAGACATCACAAACAAGAGAT

2410 2420 2430 2440 2450 2460 2470 2480 2490 2500

E M S L E S F L G R S G C I H E S K L E V T L A N Y N K E N F T V

GAAATGAGTTTAGAGAGTTTTCTTGGCAGATCAGGATGCATACATGAATCTAAATTAGAGGTTACACTTGCAAATTATAACAAGGAGAATTTTACAGTGT

2510 2520 2530 2540 2550 2560 2570 2580 2590 2600

W A I N L Q E M A Q I R R K F E L F T Y T R F D S E I T L V P C I S

GGGCTATTAATCTACAAGAAATGGCTCAAATTAGAAGGAAATTTGAATTGTTCACCTATACTAGGTTTGATTCTGAAATAACCCTAGTTCCATGCATTTC

2610 2620 2630 2640 2650 2660 2670 2680 2690 2700

A L S Q D I G H I T M Q Y M Y V P P G A P V P N S R D D Y A W Q S

CGCCCTTAGTCAGGACATTGGACACATCACAATGCAATACATGTATGTTCCACCAGGTGCACCGGTGCCCAATAGTAGGGACGATTATGCATGGCAGTCT

2710 2720 2730 2740 2750 2760 2770 2780 2790 2800

G T N A S V F W Q H G Q A Y P R F S L P F L S V A S A Y Y M F Y D

GGCACTAATGCCTCTGTTTTCTGGCAACATGGACAGGCTTATCCAAGATTTTCCTTACCTTTCCTAAGTGTGGCATCTGCTTATTACATGTTTTATGATG

2810 2820 2830 2840 2850 2860 2870 2880 2890 2900

```
 G  Y  D  E  Q  D  Q  N  Y  G  T  A  N  T  N  N  M  G  S  L  C  S  R  I  V  T  E  K  H  I  H  K  V  H
GGTATGATGAACAAGATCAAAACTATGGTACAGCAAACACAAATAACATGGGGTCACTATGCTCTAGGATAGTAACAGAGAAACACATTCATAAAGTACA
        2910      2920      2930      2940      2950      2960      2970      2980      2990      3000

  I  M  T  R  I  Y  H  K  A  K  H  V  K  A  W  C  P  R  P  P  R  A  L  E  Y  T  R  A  H  R  T  N  F
TATAATGACAAGAATCTATCACAAGGCTAAACATGTCAAGGCATGGTGTCCACGCCCACCCAGAGCGCTTGAGTATACTCGTGCTCATCGCACTAATTTT
        3010      3020      3030      3040      3050      3060      3070      3080      3090      3100
                                                                                      ↓  ?
 K  I  E  D  R  S  I  Q  T  A  I  V  T  R  P  I  I  T  T  A  G  P  S  D  M  Y  V  H  V  G  N  L  I
AAAATTGAGGATAGGAGTATTCAGACAGCAATTGTGACCAGACCAATTATCACTACAGCTGGCCCCAGTGACATGTATGTTCATGTAGGTAACCTTATTT
        3110      3120      3130      3140      3150      3160      3170      3180      3190      3200
                                                           ↓ ? P2-A
 Y  R  N  L  H  L  F  N  S  E  M  H  E  S  I  L  V  S  Y  S  S  D  L  I  I  Y  R  T  N  T  V  G  D  D
ATAGAAATCTTCATCTTTTCAACTCTGAGATGCATGAATCTATTTTGGTATCTTATTCATCAGATTTAATCATTTACCGAACAAACACTGTAGGTGATGA
        3210      3220      3230      3240      3250      3260      3270      3280      3290      3300

  Y  I  P  S  C  D  C  T  Q  A  T  Y  Y  C  K  H  K  N  R  Y  F  P  I  T  V  T  S  H  D  W  Y  E  I
TTACATTCCCTCTTGTGATTGTACCCAAGCTACTTATTATTGCAAACATAAAAATAGATACTTCCCAATTACAGTTACAAGCCATGACTGGTATGAAATA
        3310      3320      3330      3340      3350      3360      3370      3380      3390      3400

  Q  E  S  E  Y  Y  P  K  H  I  Q  Y  N  L  L  I  G  E  G  P  C  E  P  G  D  C  G  G  K  L  L  C  K
CAGGAAAGTGAGTACTATCCCAAACACATACAGTACAATTTGTTGATTGGTGAGGGCCCTTGTGAACCAGGTGACTGTGGTGGAAAGTTGCTATGCAAAC
        3410      3420      3430      3440      3450      3460      3470      3480      3490      3500
                                                                                   ↓  P2-B
 H  G  V  I  G  I  V  T  A  G  G  D  N  H  V  A  F  I  D  L  R  H  F  H  C  A  E  E  Q  G  V  T  D  Y
ATGGTGTCATAGGTATAGTAACAGCTGGTGGTGATAATCATGTGGCTTTTATTGACCTTAGACACTTCCATTGTGCTGAAGAACAAGGGGTTACAGATTA
        3510      3520      3530      3540      3550      3560      3570      3580      3590      3600

  I  H  M  L  G  E  A  F  G  N  G  F  V  D  S  V  K  E  H  I  H  A  I  N  P  V  G  N  I  S  K  K  I
TATACATATGCTAGGAGAAGCATTTGGAAATGGATTTGTGGATAGTGTAAAAGAACATATACATGCCATAAACCCAGTAGGAAATATCAGCAAGAAAATT
        3610      3620      3630      3640      3650      3660      3670      3680      3690      3700

  I  K  W  M  L  R  I  I  S  A  M  V  I  I  I  R  N  S  S  D  P  Q  T  I  L  A  T  L  T  L  I  G  C
ATTAAATGGATGTTGAGAATAATATCAGCAATGGTCATAATAATTAGAAACTCTTCTGACCCCCAAACTATATTAGCAACACTCACACTGATTGGGTGTT
        3710      3720      3730      3740      3750      3760      3770      3780      3790      3800
                                                                         ↓  P2-C
 S  G  S  P  W  R  F  L  K  E  K  F  C  K  W  T  Q  L  N  Y  I  H  K  E  S  D  S  W  L  K  K  F  T  E
CTGGATCACCCTGGAGATTTTTAAAGGAAAAATTCTGTAAATGGACACAGCTTAATTATATACACAAAGAATCAGATTCATGGTTAAAGAAATTTACTGA
        3810      3820      3830      3840      3850      3860      3870      3880      3890      3900

  A  C  N  A  A  R  G  L  E  W  I  G  N  K  I  S  K  F  I  E  W  M  K  S  M  L  P  Q  A  Q  L  K  V
AGCATGCAATGCAGCTAGAGGGCTTGAATGGATAGGGAATAAGATATCTAAATTTATTGAATGGATGAAGTCGATGCTCCCGCAAGCTCAATTGAAGGTT
        3910      3920      3930      3940      3950      3960      3970      3980      3990      4000

  K  Y  L  N  E  L  K  K  L  N  L  Y  E  K  Q  V  E  S  L  R  V  A  D  M  K  T  Q  E  K  I  K  M  E
AAGTACTTAAACGAGCTTAAAAAACTCAACCTATACGAAAAGCAAGTTGAGAGCTTGCGGGTGGCTGACATGAAAACACAAGAAAAAATTAAAATGGAAA
        4010      4020      4030      4040      4050      4060      4070      4080      4090      4100

 I  D  T  L  H  D  L  S  R  K  F  L  P  L  Y  A  S  E  A  K  R  I  K  T  L  Y  I  K  C  D  N  I  I  K
TAGACACTTTACATGATTTGTCACGTAAATTTCTACCTTTGTATGCAAGTGAGGCAAAAAGGATAAAAACCCTATACATTAAATGTGATAATATCATCAA
        4110      4120      4130      4140      4150      4160      4170      4180      4190      4200

  Q  K  K  R  C  E  P  V  A  I  V  I  H  G  P  P  G  A  G  K  S  I  T  T  N  F  L  A  K  M  I  T  N
GCAGAAGAAAAGATGTGAACCAGTAGCTATAGTTATTCATGGACCACCTGGTGCTGGCAAATCTATAACAACAAATTTCCTGGCCAAAATGATAACTAAT
        4210      4220      4230      4240      4250      4260      4270      4280      4290      4300

  D  S  D  I  Y  S  L  P  P  D  P  K  Y  F  D  G  Y  D  Q  Q  S  V  V  I  M  D  D  I  M  Q  N  P  A
GATAGTGACATATACTCTCTACCTCCTGATCCAAAATATTTTGATGGTTATGACCAACAGAGTGTAGTAATAATGGATGACATTATGCAGAATCCAGCCG
        4310      4320      4330      4340      4350      4360      4370      4380      4390      4400
```

(Fig. 4d) 7/19 0192175

G D D M T L F C Q M V S S V T F I P P M A D L P D K G K A F D S R F
GGGATGACATGACACTGTTCTGCCAAATGGTTTCTAGTGTTACATTTATACCACCAATGGCTGATCTACCAGATAAAGGCAAGGCTTTTGATTCTAGGTT
4410 4420 4430 4440 4450 4460 4470 4480 4490 4500

V L C S T N H S L L T P P T I T S L P A M N R R F F L D L D I I V
TGTATTATGCAGCACAAATCATTCCCTTCTAACACCCCCGACAATAACTTCACTACCTGCAATGAATAGAAGATTTTTCCTAGATTTAGATATAATAGTA
4510 4520 4530 4540 4550 4560 4570 4580 4590 4600

H D N F K D P Q G K L N V A A A F R P C D V D N R I G N A R C C P
CATGATAACTTCAAAGATCCACAGGGCAAACTTAATGTGGCAGCAGCGTTTCGACCATGTGATGTAGATAATAGAATAGGAAATGCACGTTGTTGTCCAT
4610 4620 4630 4640 4650 4660 4670 4680 4690 4700

F V C G K A V S F K D R N S C N K Y S L A Q V Y N I M I E E D R R R
TTGTGTGTGGAAAAGCAGTTTCTTTCAAAGATCGTAACTCTTGCAACAAATACAGCCTTGCGCAGGTGTACAACATAATGATTGAAGAAGACAGACGGAG
4710 4720 4730 4740 4750 4760 4770 4780 4790 4800

P3-A
R Q V V D V M T A I F Q G P I D M K N P P P P A I T D L L Q S V R
AAGACAAGTGGTTGATGTCATGACAGCTATATTCCAAGGGCCAATTGATATGAAAAACCCACCACCACCTGCTATTACTGACTTGCTCCAGTCTGTTAGA
4810 4820 4830 4840 4850 4860 4870 4880 4890 4900

T P E V I K Y C E G N R W I I P A E C K I E K E L N L A N T I I T
ACCCCTGAAGTTATTAAGTATTGTGAGGGTAATAGATGGATAATTCCAGCAGAATGCAAGATAGAAAAGGAGTTGAACTTGGCTAACACAATCATAACAA
4910 4920 4930 4940 4950 4960 4970 4980 4990 5000

VPg
I I A N V I G M A R I I Y V I Y K L F C T L Q G P Y S G E P K P K T
TCATTGCAAATGTTATTGGTATGGCGAGAATAATATATGTTATTTACAAACTTTTTTGCACATTACAGGGACCATATTCAGGAGAACCAAAGCCCAAGAC
5010 5020 5030 5040 5050 5060 5070 5080 5090 5100

PROTEASE
K I P E R R V V T Q G P E E E F G M S L I K H N S C V I T T E N G
TAAAATCCCAGAAAGGCGTGTAGTAACACAGGGACCAGAGGAGGAATTTGGGATGTCTTTAATTAAACATAACTCATGTGTTATTACAACAGAAAATGGG
5110 5120 5130 5140 5150 5160 5170 5180 5190 5200

K F T G L G V Y D R F V V V P T H A D P G K E I Q V D G I T T K V
AAATTCACAGGTCTTGGAGTATACGACAGATTTGTGGTCGTACCAACACATGCAGATCCTGGAAAGGAAATTCAGGTTGATGGTATAACTACAAAAGTCA
5210 5220 5230 5240 5250 5260 5270 5280 5290 5300

I D S Y D L Y N K N G I K L E I T V L K L D R N E K F R D I R R Y I
TTGACTCATATGACCTATACAACAAGAATGGGATAAAGCTAGAAATAACAGTACTTAAATTAGATAGAAATGAAAAATTTAGAGATATCAGGAGATATAT
5310 5320 5330 5340 5350 5360 5370 5380 5390 5400

P N N E D D Y P N C N L A L L A N Q P E P T I I N V G D V V S Y G
ACCTAACAATGAAGATGATTACCCCAATTGCAACTTAGCACTGCTAGCAAACCAGCCTGAACCAACTATAATCAATGTTGGAGATGTTGTATCCTATGGC
5410 5420 5430 5440 5450 5460 5470 5480 5490 5500

N I L L S G N Q T A R M L K Y S Y P T K S G Y C G G V L Y K I G Q
AATATACTGCTCAGTGGCAACCAAACGGCTAGAATGCTTAAATACAGTTACCCAACTAAATCTGGTTACTGTGGAGGTGTCTTATACAAAATTGGGCAAG
5510 5520 5530 5540 5550 5560 5570 5580 5590 5600

POLYMERASE
V L G I H V G G N G R D G F S A M L L R S Y F T D V Q G Q I T L S K
TGCTTGGAATACATGTTGGGGGCAATGGTAGGGATGGTTTCTCAGCTATGTTACTCAGATCCTATTTCACTGATGTTCAGGGCCAAATAACGTTATCAAA
5610 5620 5630 5640 5650 5660 5670 5680 5690 5700

K T S E C N L P S I H T P C K T K L Q P S V F Y D V F P G S K E P
GAAGACCAGTGAATGTAACCTACCCAGTATACACACCCCATGCAAAACCAAATTGCAGCCTAGTGTTTTCTATGATGTATTCCCTGGTTCAAAAGAACCA
5710 5720 5730 5740 5750 5760 5770 5780 5790 5800

A V L S E K D A R L Q V D F N E A L F S K Y K G N T D C S I N D H
GCTGTGTTGTCTGAAAAAGATGCCCGGTTACAAGTTGATTTCAATGAAGCACTATTTTCTAAATACAAAGGGAATACAGATTGCTCCATTAATGACCACA
5810 5820 5830 5840 5850 5860 5870 5880 5890 5900

(Fig. 4e)





```
I  R  I  A  S  S  H  Y  A  A  Q  L  I  T  L  D  I  D  P  K  P  I  T  L  E  D  S  V  F  G  T  D  G  L
TAAGAATTGCATCATCACATTATGCAGCACAACTCATTACCTTAGATATTGACCCAAAACCTATTACACTTGAGGACAGTGTCTTTGGCACTGATGGATT
      5910      5920      5930      5940      5950      5960      5970      5980      5990      6000

 E  A  L  D  L  N  T  S  A  G  F  P  Y  I  A  M  G  V  K  K  R  D  L  I  N  N  K  T  K  D  I  S  K
AGAGGCTCTTGATTTGAACACTAGCGCAGGATTTCCATATATTGCAATGGGAGTTAAAAAGAGAGATTTAATAAACAACAAGACCAAGGATATAAGCAAA
      6010      6020      6030      6040      6050      6060      6070      6080      6090      6100

 L  K  E  A  I  D  K  Y  G  V  D  L  P  M  V  T  F  L  K  D  E  L  R  K  H  E  K  V  I  K  G  K  T
CTTAAAGAAGCAATTGACAAATACGGAGTTGACTTACCTATGGTCACCTTCTTGAAAGATGAACTCAGAAAGCATGAAAAGGTAATTAAAGGTAAAACTA
      6110      6120      6130      6140      6150      6160      6170      6180      6190      6200

R  V  I  E  A  S  S  V  N  D  T  L  L  F  R  T  T  F  G  N  L  F  S  K  F  H  L  N  P  G  I  V  T  G
GAGTTATTGAAGCTAGTAGTGTGAATGATACCCTATTATTTAGAACAACTTTTGGCAACCTCTTTTCAAAGTTCCACTTGAATCCTGGAATTGTTACTGG
      6210      6220      6230      6240      6250      6260      6270      6280      6290      6300

 S  A  V  G  C  D  P  E  V  F  W  S  K  I  P  A  M  L  D  D  K  C  I  M  A  F  D  Y  T  N  Y  D  G
ATCAGCAGTTGGATGTGATCCAGAGGTGTTTTGGTCAAAAATACCAGCAATGTTGGATGATAAATGTATTATGGCTTTTGATTATACAAATTATGATGGT
      6310      6320      6330      6340      6350      6360      6370      6380      6390      6400

 S  I  H  P  I  W  F  E  A  L  K  Q  V  L  V  D  L  S  F  N  P  T  L  I  D  R  L  C  K  S  K  H  I
AGTATACACCCTATTTGGTTTGAAGCTCTTAAACAGGTACTGGTAGATCTATCATTTAATCCAACATTAATAGATAGACTATGCAAGTCTAAACACATCT
      6410      6420      6430      6440      6450      6460      6470      6480      6490      6500

F  K  N  T  Y  Y  E  V  E  G  G  V  P  S  G  C  S  G  T  S  I  F  N  T  M  I  N  N  I  I  I  R  T  L
TCAAAAATACATACTATGAAGTGGAGGGAGGTGTACCATCTGGGTGTTCAGGTACTAGTATTTTTAACACTATGATCAATAATATTATCATAAGGACCTT
      6510      6520      6530      6540      6550      6560      6570      6580      6590      6600

 V  L  D  A  Y  K  N  I  D  L  D  K  L  K  I  I  A  Y  G  D  D  V  I  F  S  Y  I  H  E  L  D  M  E
AGTGTTAGATGCATACAAGAATATAGATCTAGATAAGCTTAAGATAATTGCCTATGGTGATGATGTCATATTCTCATACATACATGAACTGGACATGGAG
      6610      6620      6630      6640      6650      6660      6670      6680      6690      6700

 A  I  A  I  E  G  V  K  Y  G  L  T  I  T  P  A  D  K  S  N  T  F  V  K  L  D  Y  S  N  V  T  F  L
GCTATAGCAATAGAGGGTGTTAAATATGGTTTGACTATAACTCCTGCTGATAAATCTAACACATTTGTAAAATTAGACTATAGCAATGTTACTTTTTTAA
      6710      6720      6730      6740      6750      6760      6770      6780      6790      6800

K  R  G  F  K  Q  D  E  K  Y  N  F  L  I  H  P  T  F  P  E  D  E  I  F  E  S  I  R  W  T  K  K  P  S
AAAGAGGGTTTAAGCAAGATGAGAAGTATAACTTTCTAATACATCCAACTTTCCCTGAAGATGAAATATTTGAATCCATCAGATGGACAAAGAAACCATC
      6810      6820      6830      6840      6850      6860      6870      6880      6890      6900

 Q  M  H  E  H  V  L  S  L  C  H  L  M  W  H  N  G  R  D  A  Y  K  K  F  V  E  K  I  R  S  V  S  A
ACAAATGCATGAACATGTGTTGTCTCTGTGTCACTTAATGTGGCACAATGGACGTGACGCATACAAAAAATTTGTGGAGAAGATACGCAGTGTAAGCGCT
      6910      6920      6930      6940      6950      6960      6970      6980      6990      7000

 G  R  A  L  Y  I  P  P  Y  D  L  L  L  H  E  W  Y  E  K  F
GGTCGTGCACTGTACATCCCTCCGTATGATTTGCTTTTGCATGAGTGGTATGAAAAATTTTAAAGATATAGAAATAGTAAACTGATAGTTTATTAGTTTT
      7010      7020      7030      7040      7050      7060      7070      7080      7090      7100


AT poly(A)
      7110      7120      7130      7140      7150      7160      7170      7180      7190      7200
```

| | HRV2/HRV14 | HRV2/Poliovirus |
|---|---|---|
| VP4 | 51 | 58 |
| VP2 | 60 | 45 |
| VP3 | 49 | 49 |
| VP1 | 32 | 17 |
| P2-A | 34 | 31 |
| P2-B | 42 | 41 |
| P2-C | 44 | 43 |
| P3-A | 38 | 36 |
| VPg | 58 | 30 |
| Protease | 50 | 35 |
| Polymerase | 55 | 56 |

Fig. 5.

| | |
|---|---|
| VP1 | N P V E N Y I D E V L N E V L V V P ... |
| VP2 | S P T V E A . G Y S D R I I Q I T R ... |
| VP3 | G L P V F I T P G S G Q F L T ... |

Fig. 6.

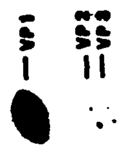


Fig. 7.

HRV2

P P P P A I T D L L Q S V R T P E V I K Y C E G N R W I I P A E C

CCACCACCACCTGCTATTACTGACTTGCTCCAGTCTGTTAGAACCCCTGAAGTTATTAAGTATTGTGAGGGTAATAGATGGATAATTCCAGCAGAATGCA

4860 4870 4880 4890 4900 4910 4920 4930 4940 4950

K I E K E L N L A N T I I T I I A N V I G M A R I I Y V I Y K L F C

AGATAGAAAAGGAGTTGAACTTGGCTAACACAATCATAACAATCATTGCAAATGTTATTGGTATGGCGAGAATAATATATGTTATTTACAAACTTTTTTG

4960 4970 4980 4990 5000 5010 5020 5030 5040 5050

T L Q G P Y S G E P K P K

CACATTACAGGGACCATATTCAGGAGAACCAAAGCCCAA

5060 5070 5080 5090

HRV89

P P P P A I A D L L R S V K T P E I I K Y C Q D N N W I V P A E C

CCTCCACCTCCAGCCATAGCTGACCTCCTTAGGTCTGTGAAAACACCAGAGATCATTAAGTATTGCCAAGATAATAATTGGATTGTTCCAGCAGAGTGTT

S I E R D L G I A N M T I G I I A N V V S I V G V I Y I I Y K L F C

CTATTGAAAGAGATTTAGGGATAGCAAATATGACTATAGGTATAATAGCTAATGTGGTCTCTATAGTAGGTGTTATCTATATAATTTATAAATTGTTCTG

T L Q G P Y S G E P K P K

TACACTTCAGGGTCCATACTCAGGGGAACCTAAACCCAA

PPPPAITDLL QSVRTPEVIK YCEGNRWIIP AECKIEKELN LANTIITIIA NVIGMARIIY VIYKLFCTLQ GPYSGEPKPK HRV2

PPPPAIADLL RSVKTPEIIK YCQDNNWIVP AECSIERDLG IANMTIGIIA NVVSIVGVIY IIYKLFCTLQ GPYSGEPKPK HRV89

(Fig. 9a) 13/19 0192175

```
   1 TTAAAACTGG ATCCAGGTTG TTCCCACCTG GATTTCCCAC AGGGAGTGGT ACTCTGTTAT TACGGTAACT TTGTACGCCA GTTTTATCTC
  91 CCTTCCCCCA TGTAACTTAG AAGTTTTTCA CAAAGACCAA TAGCCGGTAA TCAGCCAGAT TACTGAAGGT CAAGCACTTC TGTTTCCCCG
 181 GTCAATGTTG ATATGCTCCA ACAGGGCAAA AACAACTGCG ATCGTTAACC GCAAAGCGCC TACGCAAAGC TTAGTAGCAT CTTTGAAATC
 271 GTTTGGCTGG TCGATCCGCC ATTTCCCCTG GTAGACCTGG CAGATGAGGC TAGAAATACC CCACTGGCGA CAGTGTTCTA GCCTGCGTGG
 361 CTGCCTGCAC ACCCTATGGG TGTGAAGCCA AACAATGGAC AAGGTGTGAA GAGCCCCGTG TGCTCGCTTT GAGTCCTCCG GCCCCTGAAT
 451 GTGGCTAACC TTAACCCTGC AGCTAGAGCA CGTAACCCAA TGTGTATCTA GTCGTAATGA GCAATTGCGG GATGGGACCA ACTACTTTGG
 541 GTGTCCGTGT TTCACTTTTT CCTTTATATT TGCTTATGGT GACAATATAT ACAATATATA TATTGGCACC ATGGGTGCAC AGGTTTCAAG
 631 ACAAAATGTT GGAACTCACT CCACGCAAAA CTCTGTATCA AATGGGTCTA GTTTAAATTA TTTTAACATC AATTATTTCA AAGATGCTGC
 721 TTCAAATGGT GCATCAAAAC TGGAATTCAC ACAAGATCCT AGTAAATTTA CTGACCCAGT TAAGGATGTT TTGGAAAAGG GAATACCAAC
 811 ACTACAGTCC CCCACAGTGG AGGCTTGTGG ATACTCTGAT AGGATTATAC AGATTACCAG AGGAGATTCA ACCATAACCT CACAAGATGT
 901 GGCTAATGCT ATCGTTGCGT ATGGTGTTTG GCCACATTAT CTATCCTCCA AGGATGCCTC TGCAATTGAT AAACCCTCTC AACCAGATAC
 991 ATCTTCTAAT AGATTTTATA CTCTAAGGAG TGTGACCTGG AGCAGTTCCT CAAAGGGTTG GTGGTGGAAA CTACCTGATG CACTCAAGGA
1081 CATGGGTATT TTTGGTGAAA ACATGTTTTA TCATTACCTG GGTAGGAGTG GATACACAAT ACATGTGCAG TGTAATGCTA GTAAATTTCA
1171 CCAGGGTACA CTAATTGTTG CTCTGATACC TGAGCATCAG ATTGCAAGTG CCTTACATGG CAATGTGAAT GTTGGTTACA ACTACACACA
1261 CCCAGGTGAA ACAGGCAGGG AAGTTAAAGC TGAGACGAGA TTGAATCCTG ATCTACAACC TACTGAAGAG TATTGGCTAA ACTTTGATGG
1351 GACACTCCTT GGAAATATTA CCATATTCCC TCATCAATTT ATCAACTTGA GGAGTAATAA TTCTGCCACA ATAATTGCCC CTTATGTCAA
1441 TGCAGTTCCT ATGGATTCAA TGCGGAGCCA CAATAATTGG AGTTTGGTAA TAATACCAAT ATGTCCCCTT GAGACATCAA GTGCAATTAA
1531 CACAATACCT ATTACAATAT CTATAAGCCC CATGTGTGCA GAGTTTTCCG GCGCGCGTGC CAAGCGTCAA GGATTACCAG TTTTCATCAC
1621 ACCAGGTTCA GGACAGTTTT TGACAACAGA TGATTTCCAA TCCCCATGTG CACTTCCCTG GTATCACCCA ACTAAGGAAA TTTCTATTCC
1711 AGGTGAGGTT AAAAATTTGG TTGAAATTTG TCAAGTAGAC AGCCTAGTAC CAATAAATAA CACTGACACC TACATCAATA GTGAAAATAT
1801 GTATTCTGTT GTATTGCAAT CATCAATTAA TGCACCAGAT AAGATCTTCT CTATTCGAAC AGATGTTGCT TCCCAACCTT TAGCTACTAC
1891 TTTGATTGGT GAGATATCTA GCTATTTCAC CCACTGGACA GGGAGTCTCC GTTTCAGCTT CATGTTTTGT GGTACTGCCA ACACTACTGT
1981 TAAGCTTTTG TTGGCATACA CACCACCTGG TATCGCAGAA CCCACCACAA GAAAGGATGC AATGCTAGGC ACTCATGTTA TATGGGATGT
2071 GGGGTTGCAG TCTACAATAT CAATGGTAGT GCCATGGATT AGCGCTAGTC ATTATAGAAA CACATCACCA GGTAGATCTA CATCTGGGTA
2161 CATAACATGC TGGTATCAGA CTAGATTAGT CATTCCACCT CAGACCCCAC CAACAGCTAG ATTGTTATGT TTTGTATCTG GGTGCAAAGA
2251 CTTTTGCTTG CGCATGGCAC GAGATACTAA CCTACACCTG CAAAGTGGTG CAATAGCACA GAACCCTGTT GAGAATTATA TAGATGAAGT
2341 TCTTAATGAA GTTTTAGTTG TCCCAAATAT TAATAGTAGT AACCCCACAA CATCAAATTC TGCCCCAGCA TTAGATGCTG CAGAAACAGG
2431 GCACACTAGT AGTGTTCAAC CAGAGGATGT CATTGAAACT AGGTATGTGC AGACATCACA AACAAGAGAT GAAATGAGTT TAGAGAGTTT
2521 TCTTGGCAGA TCAGGATGCA TACATGAATC TAAATTAGAG GTTACACTTG CAAATTATAA CAAGGAGAAT TTTACAGTGT GGGCTATTAA
2611 TCTACAAGAA ATGGCTCAAA TTAGAAGGAA ATTTGAATTG TTCACCTATA CTAGGTTTGA TTCTGAAATA ACCCTAGTTC CATGCATTTC
2701 CGCCCTTAGT CAGGACATTG GACACATCAC AATGCAATAC ATGTATGTTC CACCAGGTGC ACCGGTGCCC AATAGTAGGG ACGATTATGC
2791 ATGGCAGTCT GGCACTAATG CCTCTGTTTT CTGGCAACAT GGACAGGCTT ATCCAAGATT TTCCTTACCT TTCCTAAGTG TGGCATCTGC
2881 TTATTACATG TTTTATGATG GGTATGATGA ACAAGATCAA AACTATGGTA CAGCAAACAC AAATAACATG GGGTCACTAT GCTCTAGGAT
2971 AGTAACAGAG AAACACATTC ATAAAGTACA TATAATGACA AGAATCTATC ACAAGGCTAA ACATGTCAAG GCATGGTGTC CACGCCCACC
3061 CAGAGCGCTT GAGTATACTC GTGCTCATCG CACTAATTTT AAAATTGAGG ATAGGAGTAT TCAGACAGCA ATTGTGACCA GACCAATTAT
3151 CACTACAGCT GGCCCCAGTG ACATGTATGT TCATGTAGGT AACCTTATTT ATAGAAATCT TCATCTTTTC AACTCTGAGA TGCATGAATC
3241 TATTTTGGTA TCTTATTCAT CAGATTTAAT CATTTACCGA ACAAACACTG TAGGTGATGA TTACATTCCC TCTTGTGATT GTACCCAAGC
3331 TACTTATTAT TGCAAACATA AAAATAGATA CTTCCCAATT ACAGTTACAA GCCATGACTG GTATGAAATA CAGGAAAGTG AGTACTATCC
3421 CAAACACATA CAGTACAATT TGTTGATTGG TGAGGGCCCT TGTGAACCAG GTGACTGTGG TGGAAAGTTG CTATGCAAAC ATGGTGTCAT
3511 AGGTATAGTA ACAGCTGGTG GTGATAATCA TGTGGCTTTT ATTGACCTTA GACACTTCCA TTGTGCTGAA GAACAAGGGG TTACAGATTA
3601 TATACATATG CTAGGAGAAG CATTTGGAAA TGGATTTGTG GATAGTGTAA AAGAACATAT ACATGCCATA AACCCAGTAG GAAATATCAG
3691 CAAGAAAATT ATTAAATGGA TGTTGAGAAT AATATCAGCA ATGGTCATAA TAATTAGAAA CTCTTCTGAC CCCCAAACTA TATTAGCAAC
3781 ACTCACACTG ATTGGGTGTT CTGGATCACC CTGGAGATTT TTAAAGGAAA AATTCTGTAA ATGGACACAG CTTAATTATA TACACAAAGA
3871 ATCAGATTCA TGGTTAAAGA AATTTACTGA AGCATGCAAT GCAGCTAGAG GGCTTGAATG GATAGGGAAT AAGATATCTA AATTTATTGA
3961 ATGGATGAAG TCGATGCTCC CGCAAGCTCA ATTGAAGGTT AAGTACTTAA ACGAGCTTAA AAAACTCAAC CTATACGAAA AGCAAGTTGA
4051 GAGCTTGCGG GTGGCTGACA TGAAAACACA AGAAAAAATT AAAATGGAAA TAGACACTTT ACATGATTTG TCACGTAAAT TTCTACCTTT
4141 GTATGCAAGT GAGGCAAAAA GGATAAAAAC CCTATACATT AAATGTGATA ATATCATCAA GCAGAAGAAA AGATGTGAAC CAGTAGCTAT
4231 AGTTATTCAT GGACCACCTG GTGCTGGCAA ATCTATAACA ACAAATTTCC TGGCCAAAAT GATAACTAAT GATAGTGACA TATACTCTCT
4321 ACCTCCTGAT CCAAAATATT TTGATGGTTA TGACCAACAG AGTGTAGTAA TAATGGATGA CATTATGCAG AATCCAGCCG GGGATGACAT
4411 GACACTGTTC TGCCAAATGG TTTCTAGTGT TACATTTATA CCACCAATGG CTGATCTACC AGATAAAGGC AAGGCTTTTG ATTCTAGGTT
4501 TGTATTATGC AGCACAAATC ATTCCCTTCT AACACCCCCG ACAATAACTT CACTACCTGC AATGAATAGA AGATTTTTCC TAGATTTAGA
4591 TATAATAGTA CATGATAACT TCAAAGATCC ACAGGGCAAA CTTAATGTGG CAGCAGCGTT TCGACCATGT GATGTAGATA ATAGAATAGG
4681 AAATGCACGT TGTTGTCCAT TTGTGTGTGG AAAAGCAGTT TCTTTCAAAG ATCGTAACTC TTGCAACAAA TACAGCCTTG CGCAGGTGTA
4771 CAACATAATG ATTGAAGAAG ACAGACGGAG AAGACAAGTG GTTGATGTCA TGACAGCTAT ATTCCAAGGG CCAATTGATA TGAAAAACCC
4861 ACCACCACCT GCTATTACTG ACTTGCTCCA GTCTGTTAGA ACCCCTGAAG TTATTAAGTA TTGTGAGGGT AATAGATGGA TAATTCCAGC
4951 AGAATGCAAG ATAGAAAAGG AGTTGAACTT GGCTAACACA ATCATAACAA TCATTGCAAA TGTTATTGGT ATGGCGAGAA TAATATATGT
5041 TATTTACAAA CTTTTTTGCA CATTACAGGG ACCATATTCA GGAGAACCAA AGCCCAAGAC TAAAATCCCA GAAAGGCGTG TAGTAACACA
5131 GGGACCAGAG GAGGAATTTG GGATGTCTTT AATTAAACAT AACTCATGTG TTATTACAAC AGAAAATGGG AAATTCACAG GTCTTGGAGT
5221 ATACGACAGA TTTGTGGTCG TACCAACACA TGCAGATCCT GGAAAGGAAA TTCAGGTTGA TGGTATAACT ACAAAAGTCA TTGACTCATA
```

(Fig. 9b)





5311 TGACCTATAC AACAAGAATG GGATAAAGCT AGAAATAACA GTACTTAAAT TAGATAGAAA TGAAAAATTT AGAGATATCA GGAGATATAT
5401 ACCTAACAAT GAAGATGATT ACCCCAATTG CAACTTAGCA CTGCTAGCAA ACCAGCCTGA ACCAACTATA ATCAATGTTG GAGATGTTGT
5491 ATCCTATGGC AATATACTGC TCAGTGGCAA CCAAACGGCT AGAATGCTTA AATACAGTTA CCCAACTAAA TCTGGTTACT GTGGAGGTGT
5581 CTTATACAAA ATTGGGCAAG TGCTTGGAAT ACATGTTGGG GGCAATGGTA GGGATGGTTT CTCAGCTATG TTACTCAGAT CCTATTTCAC
5671 TGATGTTCAG GGCCAAATAA CGTTATCAAA GAAGACCAGT GAATGTAACC TACCCAGTAT ACACACCCCA TGCAAAACCA AATTGCAGCC
5761 TAGTGTTTTC TATGATGTAT TCCCTGGTTC AAAAGAACCA GCTGTGTTGT CTGAAAAAGA TGCCCGGTTA CAAGTTGATT TCAATGAAGC
5851 ACTATTTTCT AAATACAAAG GGAATACAGA TTGCTCCATT AATGACCACA TAAGAATTGC ATCATCACAT TATGCAGCAC AACTCATTAC
5941 CTTAGATATT GACCCAAAAC CTATTACACT TGAGGACAGT GTCTTTGGCA CTGATGGATT AGAGGCTCTT GATTTGAACA CTAGCGCAGG
6031 ATTTCCATAT ATTGCAATGG GAGTTAAAAA GAGAGATTTA ATAAACAACA AGACCAAGGA TATAAGCAAA CTTAAAGAAG CAATTGACAA
6121 ATACGGAGTT GACTTACCTA TGGTCACCTT CTTGAAAGAT GAACTCAGAA AGCATGAAAA GGTAATTAAA GGTAAAACTA GAGTTATTGA
6211 AGCTAGTAGT GTGAATGATA CCCTATTATT TAGAACAACT TTTGGCAACC TCTTTTCAAA GTTCCACTTG AATCCTGGAA TTGTTACTGG
6301 ATCAGCAGTT GGATGTGATC CAGAGGTGTT TTGGTCAAAA ATACCAGCAA TGTTGGATGA TAAATGTATT ATGGCTTTTG ATTATACAAA
6391 TTATGATGGT AGTATACACC CTATTTGGTT TGAAGCTCTT AAACAGGTAC TGGTAGATCT ATCATTTAAT CCAACATTAA TAGATAGACT
6481 ATGCAAGTCT AAACACATCT TCAAAAATAC ATACTATGAA GTGGAGGGAG GTGTACCATC TGGGTGTTCA GGTACTAGTA TTTTTAACAC
6571 TATGATCAAT AATATTATCA TAAGGACCTT AGTGTTAGAT GCATACAAGA ATATAGATCT AGATAAGCTT AAGATAATTG CCTATGGTGA
6661 TGATGTCATA TTCTCATACA TACATGAACT GGACATGGAG GCTATAGCAA TAGAGGGTGT TAAATATGGT TTGACTATAA CTCCTGCTGA
6751 TAAATCTAAC ACATTTGTAA AATTAGACTA TAGCAATGTT ACTTTTTTAA AAAGAGGGTT TAAGCAAGAT GAGAAGTATA ACTTTCTAAT
6841 ACATCCAACT TTCCCTGAAG ATGAAATATT TGAATCCATC AGATGGACAA AGAAACCATC ACAAATGCAT GAACATGTGT TGTCTCTGTG
6931 TCACTTAATG TGGCACAATG GACGTGACGC ATACAAAAAA TTTGTGGAGA AGATACGCAG TGTAAGCGCT GGTCGTGCAC TGTACATCCC
7021 TCCGTATGAT TTGCTTTTGC ATGAGTGGTA TGAAAAATTT TAAAGATATA GAAATAGTAA ACTGATAGTT TATTAGTTTT AT poly(A)

Fig. 10





1 MGAQVSRQNV GTHSTQNSVS NGSSLNYFNI NYFKDAASNG ASKLEFTQDP SKFTDPVKDV LEKGIPTLQS PTVEACGYSD RIIQITRGDS
91 TITSQDVANA IVAYGVWPHY LSSKDASAID KPSQPDTSSN RFYTLRSVTW SSSSKGWWWK LPDALKDMGI FGENMFYHYL GRSGYTIHVQ
181 CNASKFHQGT LIVALIPEHQ IASALHGNVN VGYNYTHPGE TGREVKAETR LNPDLQPTEE YWLNFDGTLL GNITIFPHQF INLRSNNSAT
271 IIAPYVNAVP MDSMRSHNNW SLVIIPICPL ETSSAINTIP ITISISPMCA EFSGARAKRQ GLPVFITPGS GQFLTTDDFQ SPCALPWYHP
361 TKEISIPGEV KNLVEICQVD SLVPINNTDT YINSENMYSV VLQSSINAPD KIFSIRTDVA SQPLATTLIG EISSYFTHWT GSLRFSFMFC
451 GTANTTVKLL LAYTPPGIAE PTTRKDAMLG THVIWDVGLQ STISMVVPWI SASHYRNTSP GRSTSGYITC WYQTRLVIPP QTPPTARLLC
541 FVSGCKDFCL RMARDTNLHL QSGAIAQNPV ENYIDEVLNE VLVVPNINSS NPTTSNSAPA LDAAETGHTS SVQPEDVIET RYVQTSQTRD
631 EMSLESFLGR SGCIHESKLE VTLANYNKEN FTVWAINLQE MAQIRRKFEL FTYTRFDSEI TLVPCISALS QDIGHITMQY MYVPPGAPVP
721 NSRDDYAWQS GTNASVFWQH GQAYPRFSLP FLSVASAYYM FYDGYDEQDQ NYGTANTNNM GSLCSRIVTE KHIHKVHIMT RIYHKAKHVK
811 AWCPRPPRAL EYTRAHRTNF KIEDRSIQTA IVTRPIITTA GPSDMYVHVG NLIYRNLHLF NSEMHESILV SYSSDLIIYR TNTVGDDYIP
901 SCDCTQATYY CKHKNRYFPI TVTSHDWYEI QESEYYPKHI QYNLLIGEGP CEPGDCGGKL LCKHGVIGIV TAGGDNHVAF IDLRHFHCAE
991 EQGVTDYIHM LGEAFGNGFV DSVKEHIHAI NPVGNISKKI IKWMLRIISA MVIIIRNSSD PQTILATLTL IGCSGSPWRF LKEKFCKWTQ
1081 LNYIHKESDS WLKKFTEACN AARGLEWIGN KISKFIEWMK SMLPQAQLKV KYLNELKKLN LYEKQVESLR VADMKTQEKI KMEIDTLHDL
1171 SRKFLPLYAS EAKRIKTLYI KCDNIIKQKK RCEPVAIVIH GPPGAGKSIT TNFLAKMITN DSDIYSLPPD PKYFDGYDQQ SVVIMDDIMQ
1261 NPAGDDMTLF CQMVSSVTFI PPMADLPDKG KAFDSRFVLC STNHSLLTPP TITSLPAMNR RFFLDLDIIV HDNFKDPQGK LNVAAAFRPC
1351 DVDNRIGNAR CCPFVCGKAV SFKDRNSCNK YSLAQVYNIM IEEDRRRRQV VDVMTAIFQG PIDMKNPPPP AITDLLQSVR TPEVIKYCEG
1441 NRWIIPAECK IEKELNLANT IITIIANVIG MARIIYVIYK LFCTLQGPYS GEPKPKTKIP ERRVVTQGPE EEFGMSLIKH NSCVITTENG
1531 KFTGLGVYDR FVVVPTHADP GKEIQVDGIT TKVIDSYDLY NKNGIKLEIT VLKLDRNEKF RDIRRYIPNN EDDYPNCNLA LLANQPEPTI
1621 INVGDVVSYG NILLSGNQTA RMLKYSYPTK SGYCGGVLYK IGQVLGIHVG GNGRDGFSAM LLRSYFTDVQ GQITLSKKTS ECNLPSIHTP
1711 CKTKLQPSVF YDVFPGSKEP AVLSEKDARL QVDFNEALFS KYKGNTDCSI NDHIRIASSH YAAQLITLDI DPKPITLEDS VFGTDGLEAL
1801 DLNTSAGFPY IAMGVKKRDL INNKTKDISK LKEAIDKYGV DLPMVTFLKD ELRKHEKVIK GKTRVIEASS VNDTLLFRTT FGNLFSKFHL
1891 NPGIVTGSAV GCDPEVFWSK IPAMLDDKCI MAFDYTNYDG SIHPIWFEAL KQVLVDLSFN PTLIDRLCKS KHIFKNTYYE VEGGVPSGCS
1981 GTSIFNTMIN NIIIRTLVLD AYKNIDLDKL KIIAYGDDVI FSYIHELDME AIAIEGVKYG LTITPADKSN TFVKLDYSNV TFLKRGFKQD
2071 EKYNFLIHPT FPEDEIFESI RWTKKPSQMH EHVLSLCHLM WHNGRDAYKK FVEKIRSVSA GRALYIPPYD LLLHEWYEKF

Fig. 11





A:

1 AACCCTGTTG AGAATTATAT AGATGAAGTT CTTAATGAAG TTTTAGTTGT CCCAAATATT AATAGTAGTA ACCCCACAAC ATCAAATTCT
91 GCCCCAGCAT TAGATGCTGC AGAAACAGGG CACACTAGTA GTGTTCAACC AGAGGATGTC ATTGAAACTA GGTATGTGCA GACATCACAA
181 ACAAGAGATG AAATGAGTTT AGAGAGTTTT CTTGGCAGAT CAGGATGCAT ACATGAATCT AAATTAGAGG TTACACTTGC AAATTATAAC
271 AAGGAGAATT TTACAGTGTG GGCTATTAAT CTACAAGAAA TGGCTCAAAT TAGAAGGAAA TTTGAATTGT TCACCTATAC TAGGTTTGAT
361 TCTGAAATAA CCCTAGTTCC ATGCATTTCC GCCCTTAGTC AGGACATTGG ACACATCACA ATGCAATACA TGTATGTTCC ACCAGGTGCA
451 CCGGTGCCCA ATAGTAGGGA CGATTATGCA TGGCAGTCTG GCACTAATGC CTCTGTTTTC TGGCAACATG GACAGGCTTA TCCAAGATTT
541 TCCTTACCTT TCCTAAGTGT GGCATCTGCT TATTACATGT TTTATGATGG GTATGATGAA CAAGATCAAA ACTATGGTAC AGCAAACACA
631 AATAACATGG GGTCACTATG CTCTAGGATA GTAACAGAGA AACACATTCA TAAAGTACAT ATAATGACAA GAATCTATCA CAAGGCTAAA
721 CATGTCAAGG CATGGTGTCC ACGCCCACCC AGAGCGCTTG AGTATACTCG TGCTCATCGC ACTAATTTTA AAATTGAGGA TAGGAGTATT
811 CAGACAGCAA TTGTGACCAG ACCAATTATC ACTACAGCTG GCCCCAGTGA CATGTATGTT CATGTAGGTA ACCTTATTTA TAGAAATCTT
901 CATCTTTTCA ACTCTGAGAT GCATGAATCT ATTTTGGTAT CTTAT

B:

1 NPVENYIDEV LNEVLVVPNI NSSNPTTSNS APALDAAETG HTSSVQPEDV IETRYVQTSQ TRDEMSLESF LGRSGCIHES KLEVTLANYN
91 KENFTVWAIN LQEMAQIRRK FELFTYTRFD SEITLVPCIS ALSQDIGHIT MQYMYVPPGA PVPNSRDDYA WQSGTNASVF WQHGQAYPRF
181 SLPFLSVASA YYMFYDGYDE QDQNYGTANT NNMGSLCSRI VTEKHIHKVH IMTRIYHKAK HVKAWCPRPP RALEYTRAHR TNFKIEDRSI
271 QTAIVTRPII TTAGPSDMYV HVGNLIYRNL HLFNSEMHES ILVSY

C:

N P V E N Y I D E V L N E V L V V P N I N S S N P T T S N S A P A
AACCCTGTTGAGAATTATATAGATGAAGTTCTTAATGAAGTTTTAGTTGTCCCAAATATTAATAGTAGTAACCCCACAACATCAAATTCTGCCCCAGCAT
10 20 30 40 50 60 70 80 90 100

L D A A E T G H T S S V Q P E D V I E T R Y V Q T S Q T R D E M S L
TAGATGCTGCAGAAACAGGGCACACTAGTAGTGTTCAACCAGAGGATGTCATTGAAACTAGGTATGTGCAGACATCACAAACAAGAGATGAAATGAGTTT
110 120 130 140 150 160 170 180 190 200

E S F L G R S G C I H E S K L E V T L A N Y N K E N F T V W A I N
AGAGAGTTTTCTTGGCAGATCAGGATGCATACATGAATCTAAATTAGAGGTTACACTTGCAAATTATAACAAGGAGAATTTTACAGTGTGGGCTATTAAT
210 220 230 240 250 260 270 280 290 300

L Q E M A Q I R R K F E L F T Y T R F D S E I T L V P C I S A L S
CTACAAGAAATGGCTCAAATTAGAAGGAAATTTGAATTGTTCACCTATACTAGGTTTGATTCTGAAATAACCCTAGTTCCATGCATTTCCGCCCTTAGTC
310 320 330 340 350 360 370 380 390 400

Q D I G H I T M Q Y M Y V P P G A P V P N S R D D Y A W Q S G T N A
AGGACATTGGACACATCACAATGCAATACATGTATGTTCCACCAGGTGCACCGGTGCCCAATAGTAGGGACGATTATGCATGGCAGTCTGGCACTAATGC
410 420 430 440 450 460 470 480 490 500

S V F W Q H G Q A Y P R F S L P F L S V A S A Y Y M F Y D G Y D E
CTCTGTTTTCTGGCAACATGGACAGGCTTATCCAAGATTTTCCTTACCTTTCCTAAGTGTGGCATCTGCTTATTACATGTTTTATGATGGGTATGATGAA
510 520 530 540 550 560 570 580 590 600

Q D Q N Y G T A N T N N M G S L C S R I V T E K H I H K V H I M T
CAAGATCAAAACTATGGTACAGCAAACACAAATAACATGGGGTCACTATGCTCTAGGATAGTAACAGAGAAACACATTCATAAAGTACATATAATGACAA
610 620 630 640 650 660 670 680 690 700

R I Y H K A K H V K A W C P R P P R A L E Y T R A H R T N F K I E D
GAATCTATCACAAGGCTAAACATGTCAAGGCATGGTGTCCACGCCCACCCAGAGCGCTTGAGTATACTCGTGCTCATCGCACTAATTTTAAAATTGAGGA
710 720 730 740 750 760 770 780 790 800

R S I Q T A I V T R P I I T T A G P S D M Y V H V G N L I Y R N L
TAGGAGTATTCAGACAGCAATTGTGACCAGACCAATTATCACTACAGCTGGCCCCAGTGACATGTATGTTCATGTAGGTAACCTTATTTATAGAAATCTT
810 820 830 840 850 860 870 880 890 900

H L F N S E M H E S I L V S Y
CATCTTTTCAACTCTGAGATGCATGAATCTATTTTGGTATCTTAT
910 920 930 940 950 960 970 980 990 1000

Fig. 12



0192175

A:

```
  1 TCCCCCACAG TGGAGGCTTG TGGATACTCT GATAGGATTA TACAGATTAC CAGAGGAGAT TCAACCATAA CCTCACAAGA TGTGGCTAAT
 91 GCTATCGTTG CGTATGGTGT TTGGCCACAT TATCTATCCT CCAAGGATGC CTCTGCAATT GATAAACCCT CTCAACCAGA TACATCTTCT
181 AATAGATTTT ATACTCTAAG GAGTGTGACC TGGAGCAGTT CCTCAAAGGG TTGGTGGTGG AAACTACCTG ATGCACTCAA GGACATGGGT
271 ATTTTTGGTG AAAACATGTT TTATCATTAC CTGGGTAGGA GTGGATACAC AATACATGTG CAGTGTAATG CTAGTAAATT TCACCAGGGT
361 ACACTAATTG TTGCTCTGAT ACCTGAGCAT CAGATTGCAA GTGCCTTACA TGGCAATGTG AATGTTGGTT ACAACTACAC ACACCCAGGT
451 GAAACAGGCA GGGAAGTTAA AGCTGAGACG AGATTGAATC CTGATCTACA ACCTACTGAA GAGTATTGGC TAAACTTTGA TGGGACACTC
541 CTTGGAAATA TTACCATATT CCCTCATCAA TTTATCAACT TGAGGAGTAA TAATTCTGCC ACAATAATTG CCCCTTATGT CAATGCAGTT
631 CCTATGGATT CAATGCGGAG CCACAATAAT TGGAGTTTGG TAATAATACC AATATGTCCC CTTGAGACAT CAAGTGCAAT TAACACAATA
721 CCTATTACAA TATCTATAAG CCCCATGTGT GCAGAGTTTT CCGGCGCGCG TGCCAAGCGT CAA
```

B:

```
  1 SPTVEACGYS DRIIQITRGD STITSQDVAN AIVAYGVWPH YLSSKDASAI DKPSQPDTSS NRFYTLRSVT WSSSSKGWWW KLPDALKDMG
 91 IFGENMFYHY LGRSGYTIHV QCNASKFHQG TLIVALIPEH QIASALHGNV NVGYNYTHPG ETGREVKAET RLNPDLQPTE EYWLNFDGTL
181 LGNITIFPHQ FINLRSNNSA TIIAPYVNAV PMDSMRSHNN WSLVIIPICP LETSSAINTI PITISISPMC AEFSGARAKR Q
```

C:

```
S  P  T  V  E  A  C  G  Y  S  D  R  I  I  Q  I  T  R  G  D  S  T  I  T  S  Q  D  V  A  N  A  I  V
TCCCCCACAGTGGAGGCTTGTGGATACTCTGATAGGATTATACAGATTACCAGAGGAGATTCAACCATAACCTCACAAGATGTGGCTAATGCTATCGTTG
        10        20        30        40        50        60        70        80        90       100

A  Y  G  V  W  P  H  Y  L  S  S  K  D  A  S  A  I  D  K  P  S  Q  P  D  T  S  S  N  R  F  Y  T  L  R
CGTATGGTGTTTGGCCACATTATCTATCCTCCAAGGATGCCTCTGCAATTGATAAACCCTCTCAACCAGATACATCTTCTAATAGATTTTATACTCTAAG
       110       120       130       140       150       160       170       180       190       200

S  V  T  W  S  S  S  S  K  G  W  W  W  K  L  P  D  A  L  K  D  M  G  I  F  G  E  N  M  F  Y  H  Y
GAGTGTGACCTGGAGCAGTTCCTCAAAGGGTTGGTGGTGGAAACTACCTGATGCACTCAAGGACATGGGTATTTTTGGTGAAAACATGTTTTATCATTAC
       210       220       230       240       250       260       270       280       290       300

L  G  R  S  G  Y  T  I  H  V  Q  C  N  A  S  K  F  H  Q  G  T  L  I  V  A  L  I  P  E  H  Q  I  A
CTGGGTAGGAGTGGATACACAATACATGTGCAGTGTAATGCTAGTAAATTTCACCAGGGTACACTAATTGTTGCTCTGATACCTGAGCATCAGATTGCAA
       310       320       330       340       350       360       370       380       390       400

S  A  L  H  G  N  V  N  V  G  Y  N  Y  T  H  P  G  E  T  G  R  E  V  K  A  E  T  R  L  N  P  D  L  Q
GTGCCTTACATGGCAATGTGAATGTTGGTTACAACTACACACACCCAGGTGAAACAGGCAGGGAAGTTAAAGCTGAGACGAGATTGAATCCTGATCTACA
       410       420       430       440       450       460       470       480       490       500

P  T  E  E  Y  W  L  N  F  D  G  T  L  L  G  N  I  T  I  F  P  H  Q  F  I  N  L  R  S  N  N  S  A
ACCTACTGAAGAGTATTGGCTAAACTTTGATGGGACACTCCTTGGAAATATTACCATATTCCCTCATCAATTTATCAACTTGAGGAGTAATAATTCTGCC
       510       520       530       540       550       560       570       580       590       600

T  I  I  A  P  Y  V  N  A  V  P  M  D  S  M  R  S  H  N  N  W  S  L  V  I  I  P  I  C  P  L  E  T
ACAATAATTGCCCCTTATGTCAATGCAGTTCCTATGGATTCAATGCGGAGCCACAATAATTGGAGTTTGGTAATAATACCAATATGTCCCCTTGAGACAT
       610       620       630       640       650       660       670       680       690       700

S  S  A  I  N  T  I  P  I  T  I  S  I  S  P  M  C  A  E  F  S  G  A  R  A  K  R  Q
CAAGTGCAATTAACACAATACCTATTACAATATCTATAAGCCCCATGTGTGCAGAGTTTTCCGGCGCGCGTGCCAAGCGTCAA
       710       720       730       740       750       760       770       780       790       800
```

A:

1 GGATTACCAG TTTTCATCAC ACCAGGTTCA GGACAGTTTT TGACAACAGA TGATTTCCAA TCCCCATGTG CACTTCCCTG GTATCACCCA
91 ACTAAGGAAA TTTCTATTCC AGGTGAGGTT AAAAATTTGG TTGAAATTTG TCAAGTAGAC AGCCTAGTAC CAATAAATAA CACTGACACC
181 TACATCAATA GTGAAAATAT GTATTCTGTT GTATTGCAAT CATCAATTAA TGCACCAGAT AAGATCTTCT CTATTCGAAC AGATGTTGCT
271 TCCCAACCTT TAGCTACTAC TTTGATTGGT GAGATATCTA GCTATTTCAC CCACTGGACA GGGAGTCTCC GTTTCAGCTT CATGTTTTGT
361 GGTACTGCCA ACACTACTGT TAAGCTTTTG TTGGCATACA CACCACCTGG TATCGCAGAA CCCACCACAA GAAAGGATGC AATGCTAGGC
451 ACTCATGTTA TATGGGATGT GGGGTTGCAG TCTACAATAT CAATGGTAGT GCCATGGATT AGCGCTAGTC ATTATAGAAA CACATCACCA
541 GGTAGATCTA CATCTGGGTA CATAACATGC TGGTATCAGA CTAGATTAGT CATTCCACCT CAGACCCCAC CAACAGCTAG ATTGTTATGT
631 TTTGTATCTG GGTGCAAAGA CTTTTGCTTG CGCATGGCAC GAGATACTAA CCTACACCTG CAAAGTGGTG CAATAGCACA G

B:

1 GLPVFITPGS GQFLTTDDFQ SPCALPWYHP TKEISIPGEV KNLVEICQVD SLVPINNTDT YINSENMYSV VLQSSINAPD KIFSIRTDVA
91 SQPLATTLIG EISSYFTHWT GSLRFSFMFC GTANTTVKLL LAYTPPGIAE PTTRKDAMLG THVIWDVGLQ STISMVVPWI SASHYRNTSP
181 GRSTSGYITC WYQTRLVIPP QTPPTARLLC FVSGCKDFCL RMARDTNLHL QSGAIAQ

C:

G L P V F I T P G S G Q F L T T D D F Q S P C A L P W Y H P T K E
GGATTACCAGTTTTCATCACACCAGGTTCAGGACAGTTTTTGACAACAGATGATTTCCAATCCCCATGTGCACTTCCCTGGTATCACCCAACTAAGGAAA
10 20 30 40 50 60 70 80 90 100

I S I P G E V K N L V E I C Q V D S L V P I N N T D T Y I N S E N M
TTTCTATTCCAGGTGAGGTTAAAAATTTGGTTGAAATTTGTCAAGTAGACAGCCTAGTACCAATAAATAACACTGACACCTACATCAATAGTGAAAATAT
110 120 130 140 150 160 170 180 190 200

Y S V V L Q S S I N A P D K I F S I R T D V A S Q P L A T T L I G
GTATTCTGTTGTATTGCAATCATCAATTAATGCACCAGATAAGATCTTCTCTATTCGAACAGATGTTGCTTCCCAACCTTTAGCTACTACTTTGATTGGT
210 220 230 240 250 260 270 280 290 300

E I S S Y F T H W T G S L R F S F M F C G T A N T T V K L L L A Y
GAGATATCTAGCTATTTCACCCACTGGACAGGGAGTCTCCGTTTCAGCTTCATGTTTTGTGGTACTGCCAACACTACTGTTAAGCTTTTGTTGGCATACA
310 320 330 340 350 360 370 380 390 400

T P P G I A E P T T R K D A M L G T H V I W D V G L Q S T I S M V V
CACCACCTGGTATCGCAGAACCCACCACAAGAAAGGATGCAATGCTAGGCACTCATGTTATATGGGATGTGGGGTTGCAGTCTACAATATCAATGGTAGT
410 420 430 440 450 460 470 480 490 500

P W I S A S H Y R N T S P G R S T S G Y I T C W Y Q T R L V I P P
GCCATGGATTAGCGCTAGTCATTATAGAAACACATCACCAGGTAGATCTACATCTGGGTACATAACATGCTGGTATCAGACTAGATTAGTCATTCCACCT
510 520 530 540 550 560 570 580 590 600

Q T P P T A R L L C F V S G C K D F C L R M A R D T N L H L Q S G
CAGACCCCACCAACAGCTAGATTGTTATGTTTTGTATCTGGGTGCAAAGACTTTTGCTTGCGCATGGCACGAGATACTAACCTACACCTGCAAAGTGGTG
610 620 630 640 650 660 670 680 690 700

A I A Q
CAATAGCACAG
710 720 730 740 750 760 770 780 790 800

A: 1 ATGGGTGCAC AGGTTTCAAG ACAAAATGTT GGAACTCACT CCACGCAAAA CTCTGTATCA AATGGGTCTA GTTTAAATTA TTTTAACATC
91 AATTATTTCA AAGATGCTGC TTCAAATGGT GCATCAAAAC TGGAATTCAC ACAAGATCCT AGTAAATTTA CTGACCCAGT TAAGGATGTT
181 TTGGAAAAGG GAATACCAAC ACTACAG

B: 1 MGAQVSRQNV GTHSTQNSVS NGSSLNYFNI NYFKDAASNG ASKLEFTQDP SKFTDPVKDV LEKGIPTLQ

C: M G A Q V S R Q N V G T H S T Q N S V S N G S S L N Y F N I N Y F
ATGGGTGCACAGGTTTCAAGACAAAATGTTGGAACTCACTCCACGCAAAACTCTGTATCAAATGGGTCTAGTTTAAATTATTTTAACATCAATTATTTCA
10 20 30 40 50 60 70 80 90 100

K D A A S N G A S K L E F T Q D P S K F T D P V K D V L E K G I P T
AAGATGCTGCTTCAAATGGTGCATCAAAACTGGAATTCACACAAGATCCTAGTAAATTTACTGACCCAGTTAAGGATGTTTTGGAAAAGGGAATACCAAC
110 120 130 140 150 160 170 180 190 200

L Q
ACTACAG
210 220 230 240 250 260 270 280 290 300